# EUROPEAN PATENT APPLICATION

(11) **EP 4 806 922 A2**
(43) Date of publication of application: **16.09.2026**
(21) Application number: 26194444.1
(22) Date of filing: 25.07.2024
(51) Int. Cl.: C07K 16/30

(54) **MULTI-SPECIFIC ANTIBODIES INTEGRATING DUAL IMMUNE MODULATING MOIETIES AND USES THEREOF IN IMMUNOTHERAPY**

(30) Priority: 26.07.2023 WO PCT/CN2023/109322
(62) Document number(s) of the earlier application(s) in accordance with Art. 76 EPC: 24846490.1 / 4 750 812
(71) Applicant: Lyvgen Biopharma Holdings Limited, Grand Cayman KY11-1205 (KY)
(72) Inventor: WANG, Jieyi, Belmont, California, 94002 (US); WU, Yi, Shanghai, 201203 (CN)
(74) Representative: Cabinet Becker et Associés

(57) **Abstract**

Multi-specific antibodies comprising a first Fv fragment targeting CD3, a second Fv fragment targeting an immune receptor such as CD137 or PD-L1, and one or more binding moieties targeting tumor associated antigens. Also provided herein are therapeutic uses of the multi-specific antibodies in cancer therapy.

## Description

### BACKGROUND OF THE INVENTION

Immune cell receptor CD3 and those co-stimulatory or co-inhibitory receptors such as CD137 and PD-(L)1 play important roles in modulating immune cell functionalities, thereby controlling immune responses against pathogens or diseased cells such as cancer cells and pathogen infected cells. Antibodies targeting such immune cell receptors have been used for modulating immune responses and disease treatment. However, such therapeutic approaches may fail to achieve desired clinical efficacy and/or raise safety concerns. It is therefore of great interest to develop new immune therapies that are effective and safe.

### SUMMARY OF THE INVENTION

The present disclosure is based, at least in part, on the development of multi-specific antibodies comprising two binding moieties that modulate immune responses (dual immune modulating constructs). The dual immune modulating constructs may target CD3 and CD137 or target CD3 and PD-L1. The two binding moieties targeting CD3/CD137 or CD3/PD-L1 may be connected by an Fc fragment, which may comprise one or more mutations relative to its wild-type counterpart, for example, deletion at position 237 (237Δ) and amino acid substitution at position P329 *(e.g.,* P329G). Unless explicitly pointed out, all numbers referring to positions in an Ig molecule, including positions in the Fc fragment, follow the EU numbering system. The multi-specific antibodies provided herein may further comprise one or more antigen binding moieties specific to tumor associated antigens (TAAs).

It is reported herein that multi-specific antibodies comprising the dual immune modulating constructs showed better tumor cell killing, CD8 T cell proliferation, and *in vivo* anti-tumor efficacy as compared to multi-specific antibodies comprising one immune target moiety. Further, it is reported herein that multi-specific antibodies comprising the 237Δ/P329G Fc variant abolished Fc receptor binding activity, while Fc variants having either 237Δ or P329G mutation still maintained FcR mediated cross-linking effect at a certain level. This feature could help avoid inducing systemic immune responses mediated by Fc-FcR interaction or Fc effector function, thereby reducing the risk of side effects.

In sum, the multi-specific antibodies provided herein can target two immune receptors simultaneously, resulting in avidity-driven crosslinking of immune target antigens (e.g., CD3/CD137 or CD3/ PD-L1) so as to conditionally modulate immune responses. Such multi-specific antibodies provided herein are expected to exhibit superior anti-tumor activity, e.g., in tumor microenvironment, while avoiding stimulation of systemic immune responses, which may cause undesired side effects.

Accordingly, the present disclosure provides, in some aspects, a multi-specific antibody, comprising: (i) a first antigen binding moiety that binds CD3, (ii) a second antigen binding moiety that binds CD137 or PD-L1, (iii) a third antigen binding moiety that binds a first tumor associated antigen (TAA); and optionally (iv) a fourth antigen binding moiety that binds a second TAA.

The first antigen binding moiety (anti-CD3) is a first Fv fragment comprising a first heavy chain variable region (V_{H}) and a first light chain variable region (V_{L}). Similarly, the second antigen binding moiety (anti-CD137 or anti-PD-L1) is a second Fv fragment comprising a second V_{H} and a second V_{L}. The first antigen binding moiety and the second antigen binding moiety are connected via a Fc fragment. The third antigen binding moiety (anti-TAA) is connected to the first antigen binding moiety, for example, via a first peptide linker. The optional fourth binding moiety (anti-TAA) can be connected to the first antigen binding moiety, for example, via a second peptide linker.

The Fc fragment in the multi-specific antibody provided herein may comprise a hinge domain and a CH2 domain. In some instances, the Fc fragment comprises a deletion at position 237 (237Δ) and an amino acid substitution at position P329 (e.g., P329G). In some embodiments, the Fc fragment is an IgG1 Fc fragment, which optionally comprises the amino acid sequence of SEQ ID NO: 91. In some embodiments, any of the Fc fragments provided herein may further comprise a CH3 domain. In some examples, the CH3 domain is from a wild-type immunoglobulin molecule (e.g., an IgG molecule), for example, comprising the amino acid sequence of SEQ ID NO: 92. In other examples, the CH3 domain may comprise one or more mutations that enhance heterodimerization over homodimerization of the Fc fragments comprising such as relative to the wild-type counterpart and/or reduce protein A binding. Exemplary CH3 domain variants may comprise the amino acid sequence of SEQ ID NO: 93, 94, or 95.

In any of the multi-specific antibodies provided herein the V_{H} of the anti-CD3 moiety (first V_{H}) may comprise the same heavy chain complementarity determining regions (CDRs) as those in SEQ ID NO: 7. Alternatively or in addition, the V_{L} of the anti-CD3 moiety (first V_{L}) may comprise the same light chain CDRs as those in SEQ ID NO: 8. In some embodiments, the first V_{H} may comprise the amino acid sequence of SEQ ID NO: 7 and/or the first V_{L} may comprise the amino acid sequence of SEQ ID NO: 8.

In some embodiments, the second antigen binding moiety binds CD137. In some instances, the V_{H} of the anti-CD137 moiety (the second V_{H}) may comprise the same heavy chain complementarity determining regions (CDRs) as those in SEQ ID NO: 9. Alternatively or in addition, the V_{L} of the anti-CD137 moiety (the second V_{L}) may comprise the same light chain CDRs as those in SEQ ID NO: 10. In some examples, the second V_{H} comprises the amino acid sequence of SEQ ID NO: 9 and/or the second V_{L} comprises the amino acid sequence of SEQ ID NO: 10. Exemplary multi-specific antibodies comprising anti-CD3 and anti-CD137 moieties may comprise a first polypeptide comprising the amino acid sequence of SEQ ID NO: 3 and a second polypeptide comprising the amino acid sequence of SEQ ID NO: 4.

In other embodiments, the second antigen binding moiety binds PD-L1. In some instances, the V_{H} of the anti-PD-L1 moiety (the second V_{H}) may comprise the same heavy chain complementarity determining regions (CDRs) as those in SEQ ID NO: 11. Alternatively or in addition, the VL of the anti-PD-L1 moiety (the second V_{L}) may comprise the same light chain CDRs as those in SEQ ID NO: 12. In some examples, the second V_{H} comprises the amino acid sequence of SEQ ID NO: 11 and/or the second V_{L} comprises the amino acid sequence of SEQ ID NO: 12. Exemplary multi-specific antibodies comprising anti-CD3 and anti-PD-L1 moieties may comprise a first polypeptide comprising the amino acid sequence of SEQ ID NO: 5 and a second polypeptide comprising the amino acid sequence of SEQ ID NO: 6.

The multi-specific antibodies provided herein comprise one or more binding moieties to tumor associated antigens (TAAs). In some instances, one or more of the anti-TAA moieties can be Fab fragments.

Exemplary TAAs include, but are not limited to, B7H3, CD19, CD20, PSMA, HER2, CEA, BCMA, P53mut, DLL3, MET, and EGFR. In some examples, the multi-specific antibody comprises one or more antigen binding moieties specific to HER2, CEA, BCMA, B7H3, or CD19. In one specific example, the anti-TAA moiety in the multi-specific antibody binds HER2. In another specific example, the anti-TAA moiety binds CEA. In yet another specific example, the anti-TAA moiety binds BCMA. In some instances, the multi-specific antibodies provided herein may comprise two anti-TAA moieties, which may bind to two different TAAs. In other instances, the multi-specific antibodies provided herein may comprise two anti-TAA moieties, which may bind to the same TAA (*e.g.*, bind to different epitopes of the TAA). In yet other instances, the multi-specific antibodies provided herein may comprise two identical anti-TAA moieties.

In some examples, the multi-specific antibodies disclosed herein binds HER2, CD3, and CD137. In some examples, the multi-specific antibodies disclosed herein binds HER2, CD3, and PD-L1. In some examples, the multi-specific antibodies disclosed herein binds CEA, CD3, and CD137. In some examples, the multi-specific antibodies disclosed herein binds CEA, CD3, and PD-L1. In some examples, the multi-specific antibodies disclosed herein binds BCMA, CD3, and CD137. In some examples, the multi-specific antibodies disclosed herein binds BCMA, CD3, and PD-L1.

Exemplary multi-specific antibodies with binding moieties specific to CD3, CD137, and a TAA provided herein may comprise:
(i) a first polypeptide comprising the amino acid sequence of SEQ ID NO: 23, a second polypeptide comprising the amino acid sequence of SEQ ID NO: 24, a third polypeptide comprising the amino acid sequence of SEQ ID NO: 25, and a fourth polypeptide comprising the amino acid sequence of SEQ ID NO: 26;
(ii) a first polypeptide comprising the amino acid sequence of SEQ ID NO: 39, a second polypeptide comprising the amino acid sequence of SEQ ID NO: 40, and a third polypeptide comprising the amino acid sequence of SEQ ID NO: 41;
(iii) a first polypeptide comprising the amino acid sequence of SEQ ID NO: 55, a second polypeptide comprising the amino acid sequence of SEQ ID NO: 56, and a third polypeptide comprising the amino acid sequence of SEQ ID NO: 57;
(iv) a first polypeptide comprising the amino acid sequence of SEQ ID NO: 58, a second polypeptide comprising the amino acid sequence of SEQ ID NO: 59, and a third polypeptide comprising the amino acid sequence of SEQ ID NO: 60;
(v) a first polypeptide comprising the amino acid sequence of SEQ ID NO: 54, a second polypeptide comprising the amino acid sequence of SEQ ID NO: 63, a third polypeptide comprising the amino acid sequence of SEQ ID NO: 66, and a fourth polypeptide comprising the amino acid sequence of SEQ ID NO: 69;
(vi) a first polypeptide comprising the amino acid sequence of SEQ ID NO: 74, a second polypeptide comprising the amino acid sequence of SEQ ID NO: 77, a third polypeptide comprising the amino acid sequence of SEQ ID NO: 80, and a fourth polypeptide comprising the amino acid sequence of SEQ ID NO: 83;
(vii) a first polypeptide comprising the amino acid sequence of SEQ ID NO: 74, a second polypeptide comprising the amino acid sequence of SEQ ID NO: 63, a third polypeptide comprising the amino acid sequence of SEQ ID NO: 80, and a fourth polypeptide comprising the amino acid sequence of SEQ ID NO: 69;
(viii) a first polypeptide comprising the amino acid sequence of SEQ ID NO: 54, a second polypeptide comprising the amino acid sequence of SEQ ID NO: 102, a third polypeptide comprising the amino acid sequence of SEQ ID NO: 103, and a fourth polypeptide comprising the amino acid sequence of SEQ ID NO: 104; or
(ix) a first polypeptide comprising the amino acid sequence of SEQ ID NO: 54, a second polypeptide comprising the amino acid sequence of SEQ ID NO: 63, a third polypeptide comprising the amino acid sequence of SEQ ID NO: 103, and a fourth polypeptide comprising the amino acid sequence of SEQ ID NO: 69.

Exemplary multi-specific antibodies with binding moieties specific to CD3, PD-L1, and a TAA provided herein may comprise:
(i) a first polypeptide comprising the amino acid sequence of SEQ ID NO: 27, a second polypeptide comprising the amino acid sequence of SEQ ID NO: 28, a third polypeptide comprising the amino acid sequence of SEQ ID NO: 25, and a fourth polypeptide comprising the amino acid sequence of SEQ ID NO: 26;
(ii) a first polypeptide comprising the amino acid sequence of SEQ ID NO: 46, a second polypeptide comprising the amino acid sequence of SEQ ID NO: 47, and a third polypeptide comprising the amino acid sequence of SEQ ID NO: 41
(iii) a first polypeptide comprising the amino acid sequence of SEQ ID NO: 67, a second polypeptide comprising the amino acid sequence of SEQ ID NO: 68, and a third polypeptide comprising the amino acid sequence of SEQ ID NO: 60;
(iv) a first polypeptide comprising the amino acid sequence of SEQ ID NO: 81, a second polypeptide comprising the amino acid sequence of SEQ ID NO: 82, and a third polypeptide comprising the amino acid sequence of SEQ ID NO: 57;
(v) a first polypeptide comprising the amino acid sequence of SEQ ID NO: 105, a second polypeptide comprising the amino acid sequence of SEQ ID NO: 106, a third polypeptide comprising the amino acid sequence of SEQ ID NO: 66, and a fourth polypeptide comprising the amino acid sequence of SEQ ID NO: 69;
(vi) a first polypeptide comprising the amino acid sequence of SEQ ID NO: 107, a second polypeptide comprising the amino acid sequence of SEQ ID NO: 108, a third polypeptide comprising the amino acid sequence of SEQ ID NO: 80, and a fourth polypeptide comprising the amino acid sequence of SEQ ID NO: 83;
(vii) a first polypeptide comprising the amino acid sequence of SEQ ID NO: 107, a second polypeptide comprising the amino acid sequence of SEQ ID NO: 106, a third polypeptide comprising the amino acid sequence of SEQ ID NO: 80, and a fourth polypeptide comprising the amino acid sequence of SEQ ID NO: 69;
(viii) a first polypeptide comprising the amino acid sequence of SEQ ID NO: 105, a second polypeptide comprising the amino acid sequence of SEQ ID NO: 109, a third polypeptide comprising the amino acid sequence of SEQ ID NO: 103, and a fourth polypeptide comprising the amino acid sequence of SEQ ID NO: 104; or
(ix) a first polypeptide comprising the amino acid sequence of SEQ ID NO: 105, a second polypeptide comprising the amino acid sequence of SEQ ID NO: 106, a third polypeptide comprising the amino acid sequence of SEQ ID NO: 103, and a fourth polypeptide comprising the amino acid sequence of SEQ ID NO: 69.

In another aspect, provided herein is a nucleic acid or a nucleic acid set, which collectively encodes any of the multi-specific antibodies disclosed herein. In some embodiments, one nucleic acid comprises coding sequences for all of the polypeptides of a multi-specific antibody as disclosed herein. In other embodiments, the coding sequences of the polypeptides are on two or more nucleic acids, which taken together ("collectively") encode all of the polypeptides of a multi-specific antibody as disclosed herein. In some instances, the nucleic acid or the nucleic acid set can be an expression vector or an expression vector set.

Further, the present disclosure features a host cell, comprising the nucleic acid or the nucleic acid set provided herein, which encode or collectively encode the polypeptide chains of a multi-specific antibody as disclosed herein. In some embodiments, the host cell can be a mammalian host cell.

Also provided here is a method for producing a multi-specific antibody, comprising: (i) culturing the host cell comprising the nucleic acid(s) encoding the multi-specific antibody disclosed herein under conditions allowing for expression of the antibody; and (ii) harvesting the antibody thus produced.

In yet another aspect, the present disclosure provides a pharmaceutical composition comprising any multi-specific antibodies disclosed herein or a nucleic acid or nucleic acid set encoding such, and a pharmaceutically acceptable carrier.

Moreover, the present disclosure features a method for modulating immune responses in a subject, the method comprising administering to a subject in need thereof an effective amount of the multi-specific antibody disclosed herein, a nucleic acid(s) encoding such, or a pharmaceutical composition comprising the antibody or the encoding nucleic acid(s). In some embodiments, the subject is a human patient having or suspected of having cancer.

The details of one or more embodiments of the invention are set forth in the description below. Other features or advantages of the present invention will be apparent from the following drawings and detailed description of several embodiments, and also from the appended claims.

### BRIEF DESCRIPTION OF THE DRAWINGS

The following drawings form part of the present specification and are included to further demonstrate certain aspects of the present disclosure, which can be better understood by reference to the drawing in combination with the detailed description of specific embodiments presented herein.
**FIGs. 1A-1B** include diagrams showing schematic design of exemplary multi-specific antibodies provided herein. **FIG. 1A****:** an exemplary dual immune modulating construct containing one central CD3 binding domain and one terminal CD137 or PD-L1 binding domain. **FIG. 1B****:** an exemplary design of a multi-specific antibody having the dual immune modulating construct and additional antigen binding fragments.
**FIGs. 2A-2B** include diagrams showing cytotoxicity activity and cytokine secretion of exemplary multi-specific antibodies. **FIG. 2A****:** cytotoxicity of and secretion of cytokines (IL2, IL6, IFN-γ, and TNF-α secretion) by human PBMCs co-cultured with TA-expressing tumor cells in the presence of Ly2909. **FIG. 2B****:** cytotoxicity of and section of cytokines (IL2, IL6, IFN-γ, and TNF-α) by PBMCs co-cultured with TA-expressing tumor cells in the presence of clone Ly2323.
**FIG. 3** is a diagram showing CD25+ percentage of CD8+ T cell from a T cell activation assay by co-culturing TA-expressing tumor cells with splenocytes from the huCD3/huCD137 KI mice, in the absence (control) or presence of antibodies Ly2909, Ly2915 and Ly2323 as indicated.
**FIG 4** is a diagram showing mean fluorescence intensity of CD25 in CD8+ T cell from a T cell activation assay by co-culturing TA-expressing tumor cells with splenocytes from the huCD3/huCD137 KI mice, in the absence (control) or presence of antibodies Ly2949, Ly2951 and Ly2313.
**FIG. 5** is a diagram showing anti-tumor activities of antibodies Ly3096, Ly3098 and Ly2314 in the EL4-huBCMA bearing mouse model transplanted with bone marrow from human CD3 and CD137 knock-in mice.
**FIG. 6** is a diagram showing anti-tumor activities of antibodies Ly2949, Ly2951 and Ly2313 in LL2-huBCMA bearing mouse models transplanted with bone marrow from human CD3 and CD137 knock-in mice.
**FIG 7** is a diagram showing anti-tumor activities of antibodies Ly2323, Ly2909 and Ly2915 in human PBMC engrafted mouse model bearing LS 174T tumor cells.
**FIG. 8** is a diagram showing anti-tumor activities of antibodies Ly2323, Ly2915 and Ly3060 in human PBMC engrafted mouse model bearing LS 174T tumor cells.
**FIG. 9** is a diagram showing anti-tumor activities of antibodies Ly2313 and Ly2959 in human PBMC engrafted mouse model bearing NCI-H929 tumor cells.
**FIG. 10** is a diagram showing anti-tumor activities of antibodies Ly3098, 3106 and Ly2314 in human PBMC engrafted mouse model bearing MM.1R tumor cells.

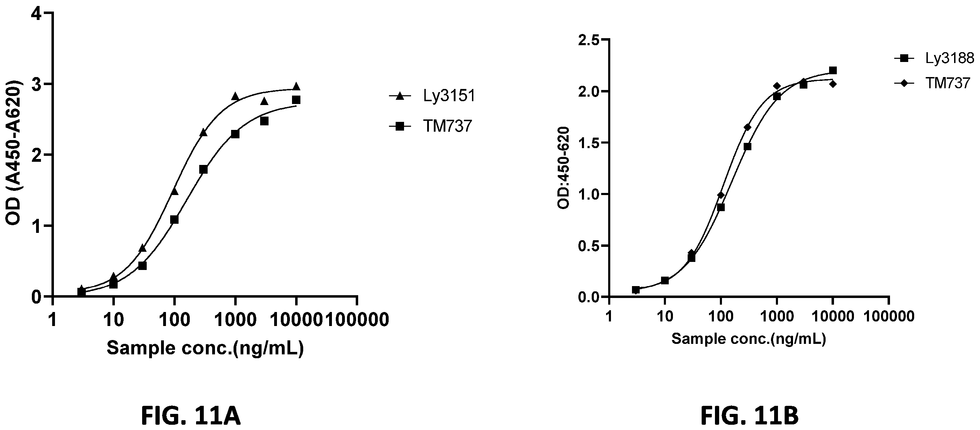

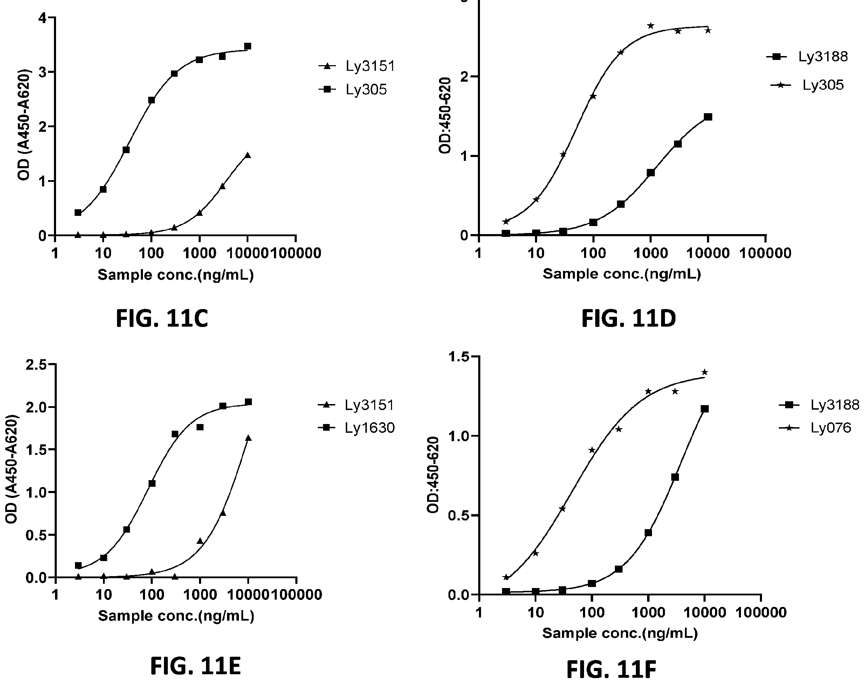

**FIGs. 11A-11F** include diagrams showing binding activities to HER2, CD3, CD137 or PD-L1 of exemplary multi-specific antibodies. **FIG. 11A**: Binding activity to human HER2 protein of antibodies Ly3151 and TM737**.** **FIG. 11B**: Binding activity to human HER2 protein of antibodies Ly3188 and TM737**.** **FIG. 11C**: Binding activity to human CD3D/E protein of antibodies Ly3151 and Ly305. **FIG. 11D**: Binding activity to human CD3D/E protein of antibodies Ly3188 and Ly305. **FIG. 11E**: Binding activity to human CD137 protein of antibodies Ly3151 and Ly1630**.** **FIG. 11F**: Binding activity to human PD-L1 protein of antibodies Ly3188 and Ly076. Parental anti-HER2 mAb TM737, anti-CD3 mAb Ly305, anti-CD137 mAb Ly1630, and anti-PD-L1 mAb Ly076 were used as controls.

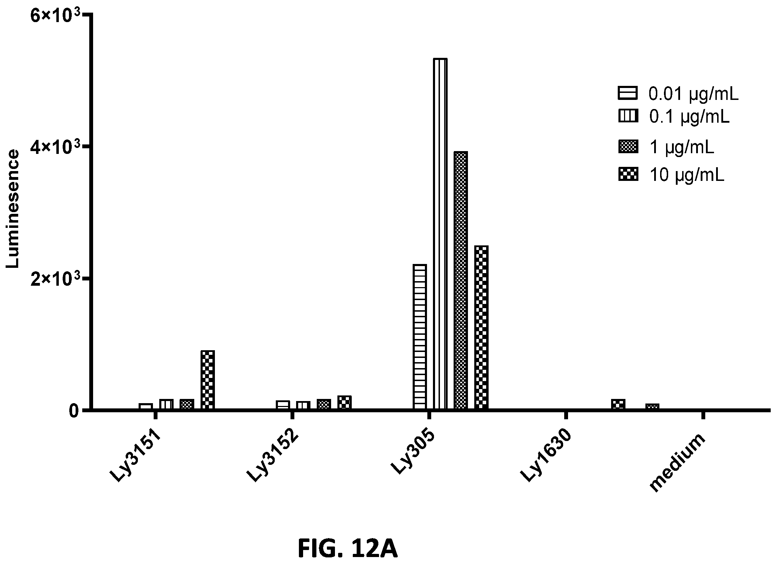

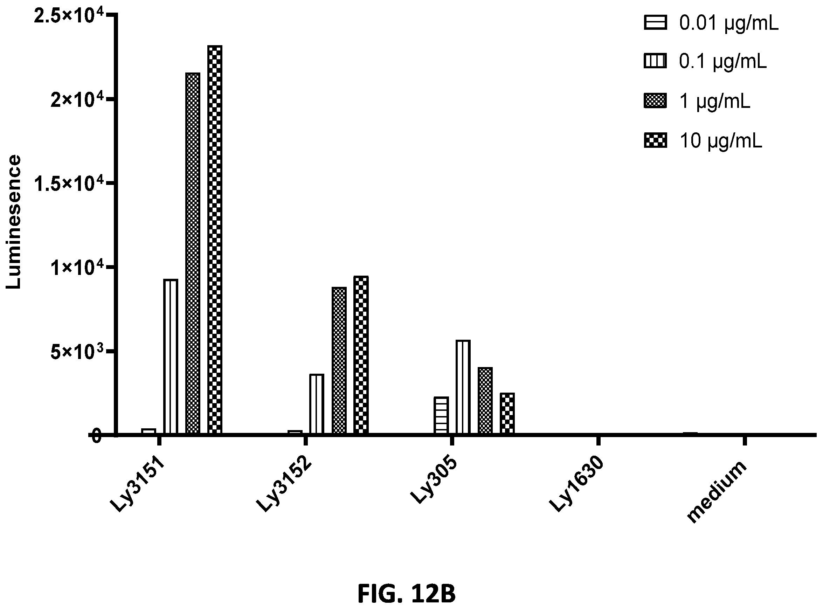

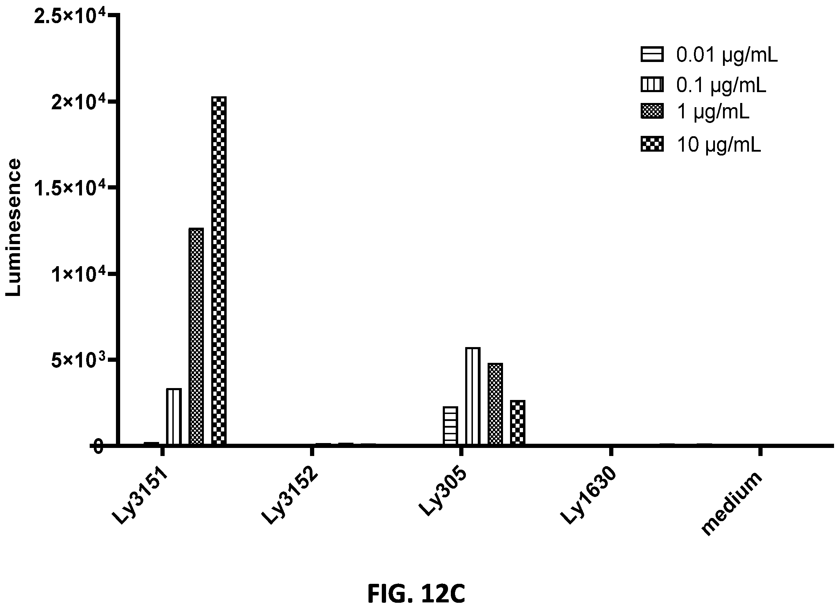

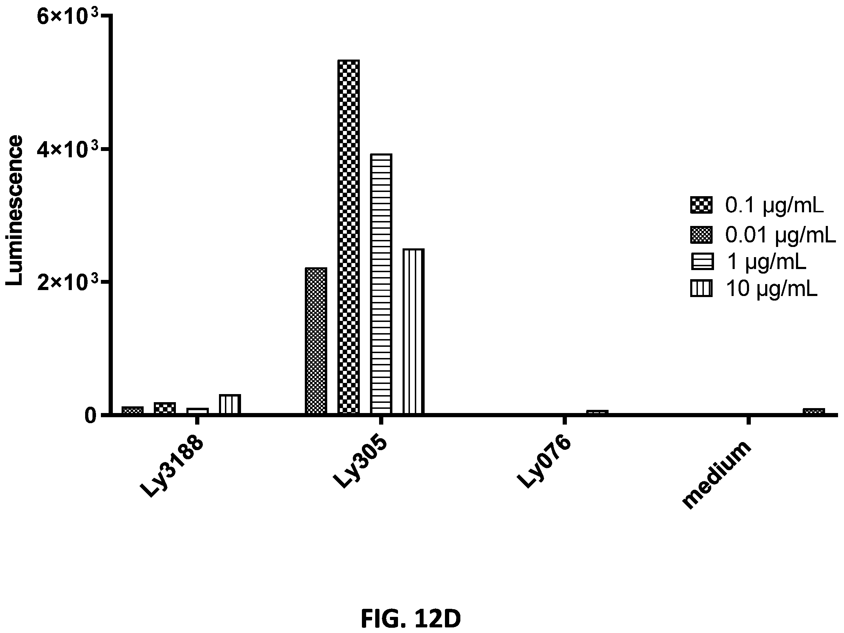

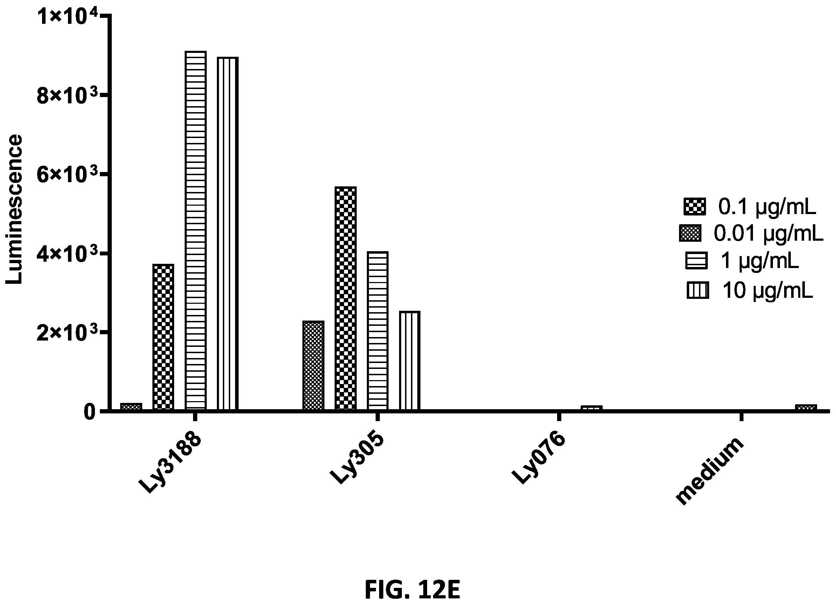

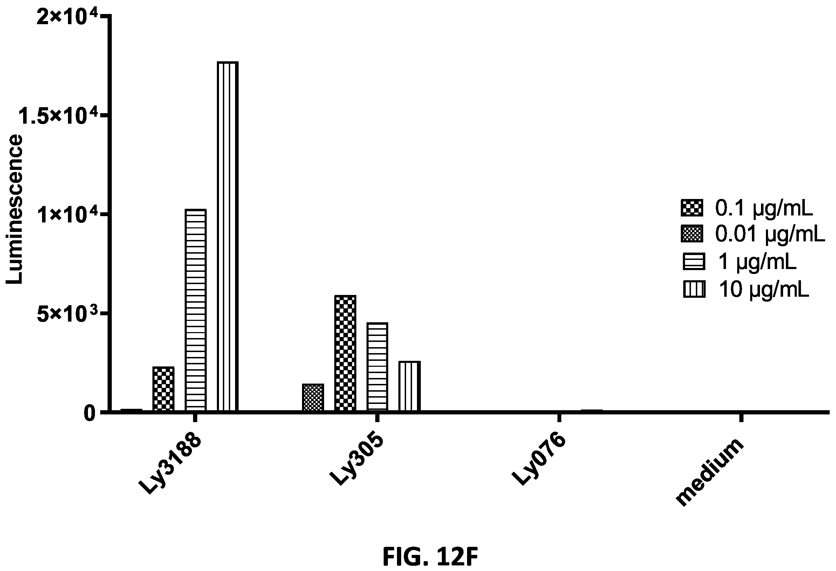

**FIGs. 12A-12F** include charts showing activation of human CD3 signaling by exemplary multi-specific antibodies as indicated. **FIGs. 12A-12C**: agonistic activity of antibodies Ly3151, Ly3152, parental anti-CD137 mAb Ly1630 and parental anti-CD3 mAb Ly305 in a Jurkat-NFAT-luminescence reporter assay alone **(A),** co-cultured with TA-expressing CHO cell (**B**) or CD137-expressing CHO cells **(C).** **FIGs. 12D-12F**: agonistic activity of antibodies Ly3188, parent anti-CD3 mAb Ly305 and parent anti-PD-L1 mAb Ly076 in a Jurkat-NFAT-luminescence reporter assay alone (**D**), co-cultured with TA-expressing CHO cell (**E**) or PD-L1-expressing CHO cells (**F**). The luminescence produced by reporter cells indicates CD3 activation.

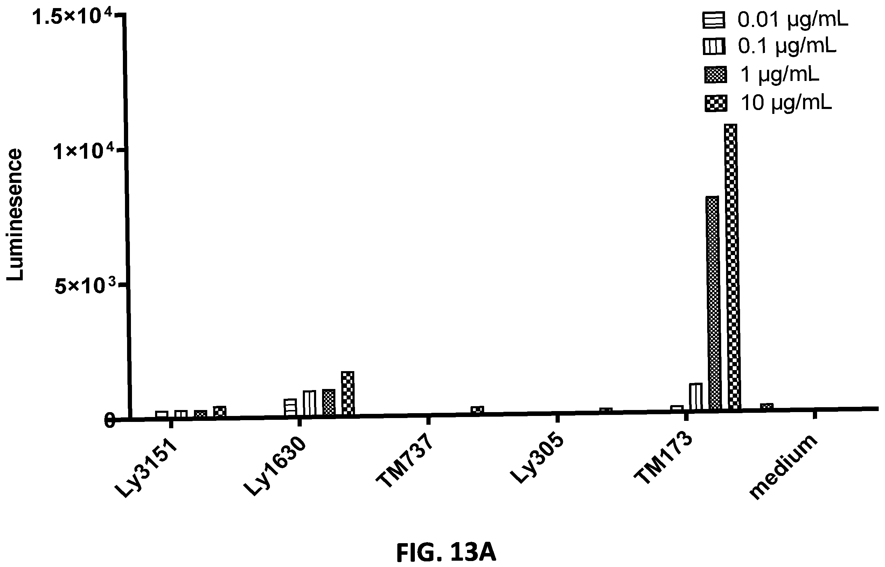

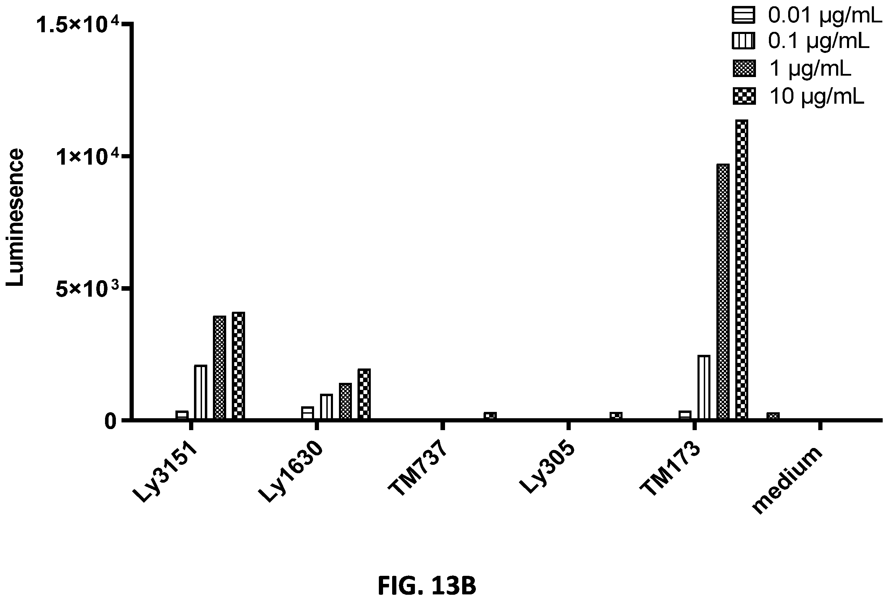

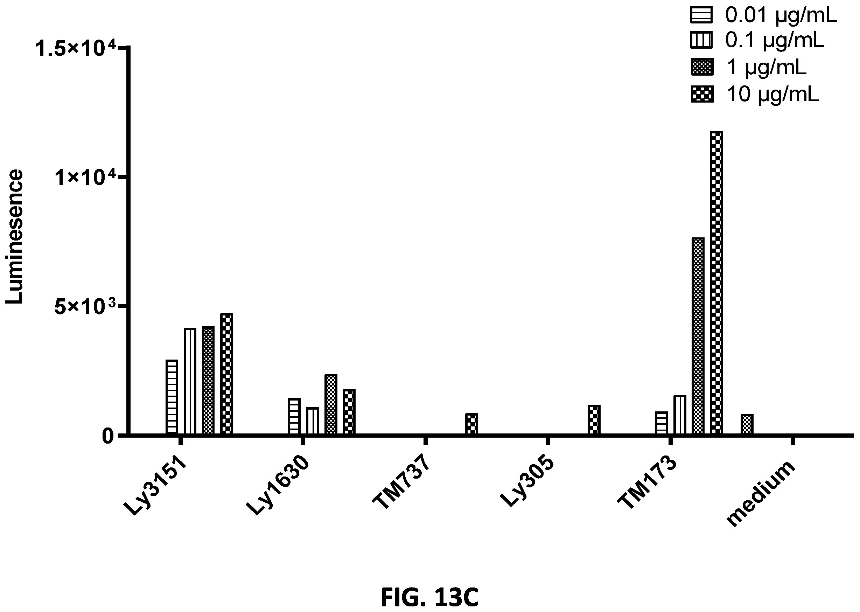

**FIGs. 13A-13C** include charts showing activation of human CD137 signaling by exemplary multi-specific antibodies as indicated. The agonistic activity of these multi-specific antibodies was evaluated either alone or co-cultured with additional target expressing cells in a CD137 reporter assay. The luminescence produced by reporter cells indicates CD137 activation**.** **FIGs. 13A-13C**: agonistic activity of antibody Ly3151, the parent anti-CD137 mAb Ly1630, anti-CD3 mAb Ly305, anti-HER2 mAb TM737 and control anti-CD137 mAb TM173 alone (**A**), co-cultured with TA-expressing CHO cells (**B**) or CD3-expressing cells (**C**).
**FIGs. 14A-14B** include diagrams showing cytotoxicity activity of exemplary multi-specific antibodies as indicated. **FIG. 14A****:** killing of HER2-expressing EBC-1-Luc cells by human PBMCs in the presence of antibodies Ly3151 and Ly2935. **FIG. 14B**: killing of EBC-1-Luc cells by human PBMCs in the presence of antibodies Ly3188 and Ly2935. Relative luminescence unit (RLU) indicates live tumor cells remaining after 48 hours incubation time.
**FIG. 15** is a diagram showing anti-tumor activities of antibodies Ly3151 and Ly2935 in the human PBMC engrafted mouse model bearing EBC-1 tumor cells.
**FIG. 16** is a diagram showing anti-tumor activities of antibodies Ly3188 and Ly2935 in a human PBMC engrafted mouse model bearing EBC-1 tumor cells.

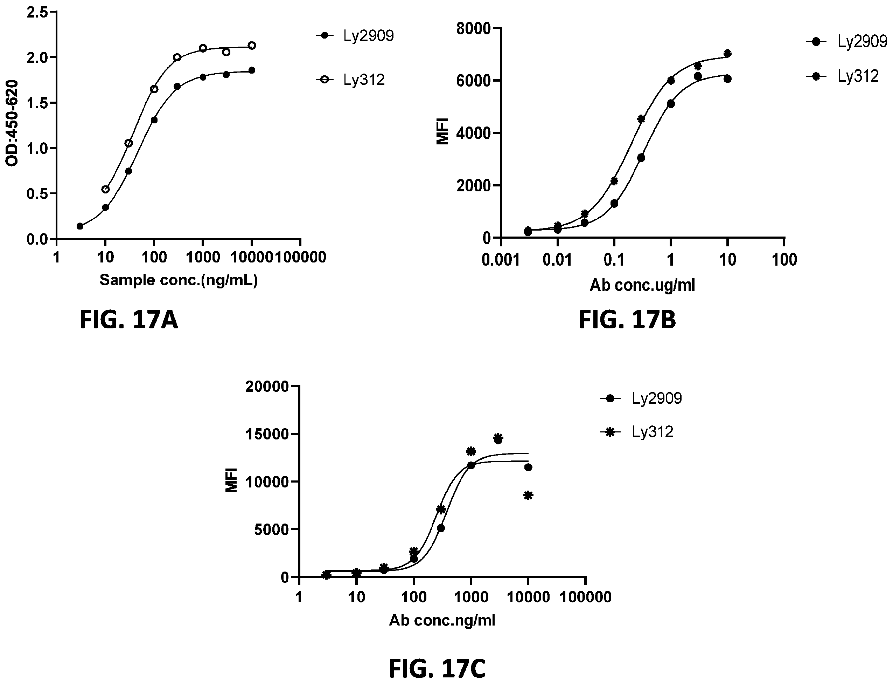

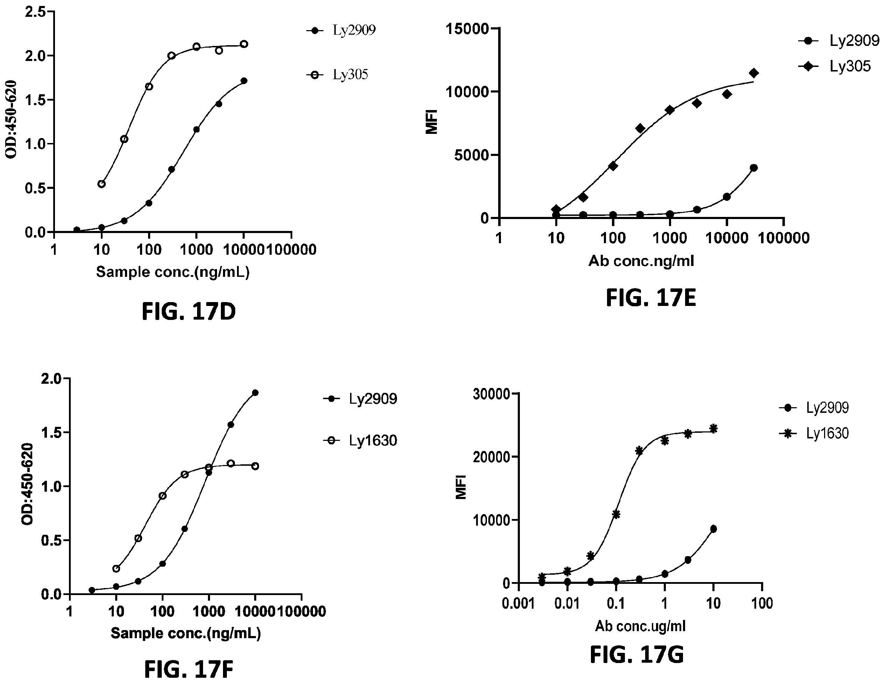

**FIGs. 17A-17G** include diagrams showing binding activities of exemplary multi-specific antibodies to CEA, CD3 or CD137 as indicated. **FIG. 17A**: Binding activity to human CEA protein of antibodies Ly2909 and Ly312**.** **FIG. 17B**: Binding activity to human CEA expressing LT 174T cells of antibodies Ly2909 and Ly312**.** **FIG. 17C**: Binding activity to human CEA expressing CHO cells of antibodies Ly2909 and Ly312. **FIG. 17D**: Binding activity to human CD3D/E protein of antibodies Ly2909 and Ly305. **FIG. 17E**: Binding activity to human CD3 expressing Jurkat cells of antibodies Ly2909 and Ly305**.** **FIG. 17F**: Binding activity to human CD137 protein of antibodies Ly2909 and Ly1630. **FIG. 17G**: Binding activity to human CD137 expressing CHO cells of antibodies Ly2909 and Ly1630. Parental anti-CEA mAb Ly312, anti-CD3 mAb Ly305, and anti-CD137 mAb Ly1630 were used as controls.
**FIGs. 18A-18D** include charts showing activation of human CD3 signaling by exemplary multi-specific antibodies as indicated in a Jurkat-NFAT-luminescence reporter assay. **FIGs. 18A-18D**: agonistic activity of antibodies Ly2909, Ly2915, Ly2918, Ly3060 and anti-CD3 mAb Ly305 alone (**A**), co-cultured with CEA overexpressing cell (**B**). co-cultured with CD137 overexpressing cell (**C**), co-cultured with PD-L1overexpressing cell (**D**). The luminescence produced by reporter cells indicates CD3 activation.
**FIGs. 19A-19D** include diagrams showing cytotoxicity activity of exemplary multi-specific antibodies. **FIG. 19A**: killing by PBMCs co-cultured with LS 174T-Luc cells in the presence of antibodies Ly2909 and Ly2323. **FIG. 19B**: killing by PBMCs co-cultured with HT29-Luc cells in the presence of antibodies Ly2909 and Ly2323**.** **FIG. 19C**: killing by PBMCs co-cultured with HT29-Luc cells in the presence of antibodies Ly3060 and Ly2323. **FIG. 19D**: killing by PBMCs co-cultured with LS 174T-Luc cells in the presence of antibodies Ly3060 and Ly2323.
**FIG. 20** is a diagram showing anti-tumor activities of antibodies Ly2909 and Ly2323 in human PBMC engrafted mouse model bearing LS 174T tumor cells.
**FIG. 21** is a diagram showing anti-tumor activities of antibodies Ly2915, Ly3060 and Ly2323 in human PBMC engrafted mouse model bearing LS 174T tumor cells.

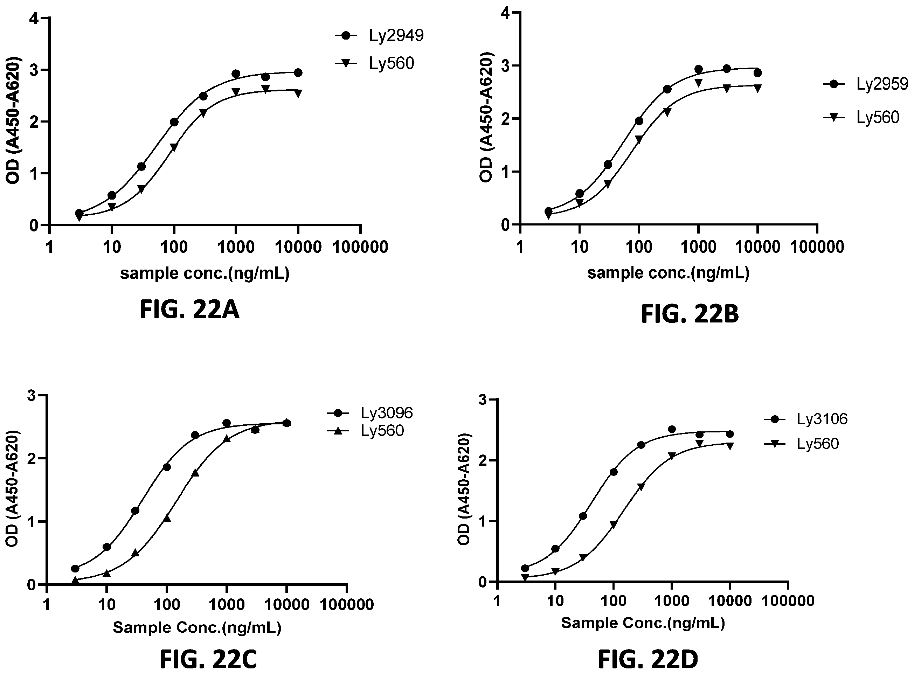

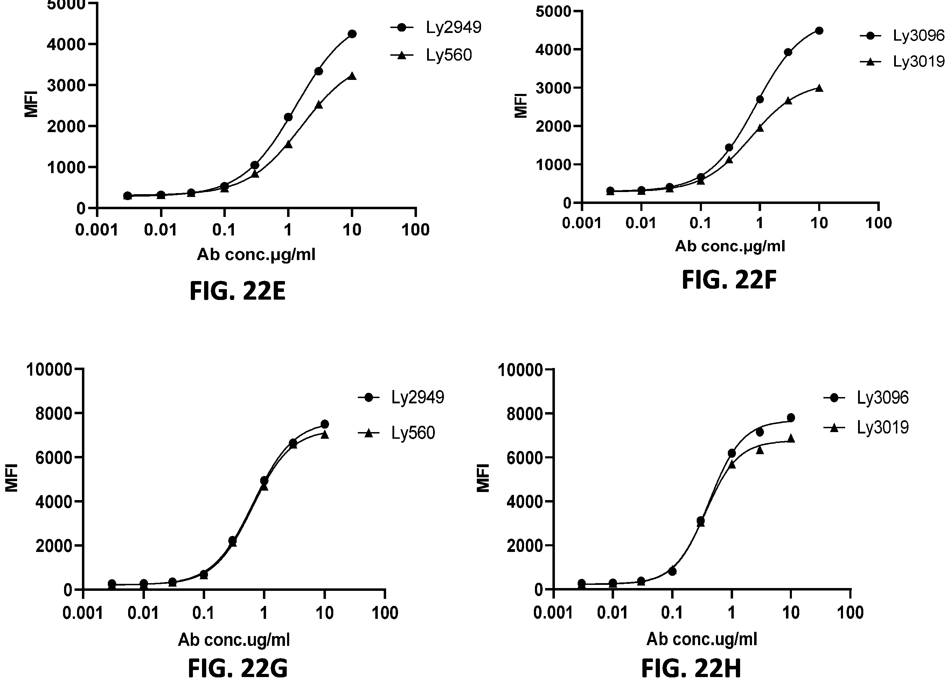

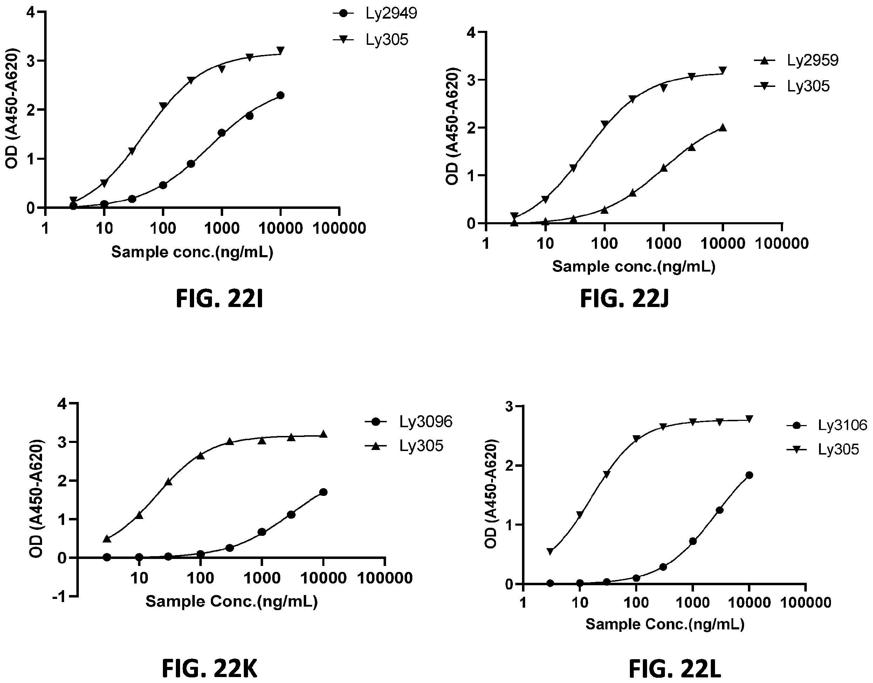

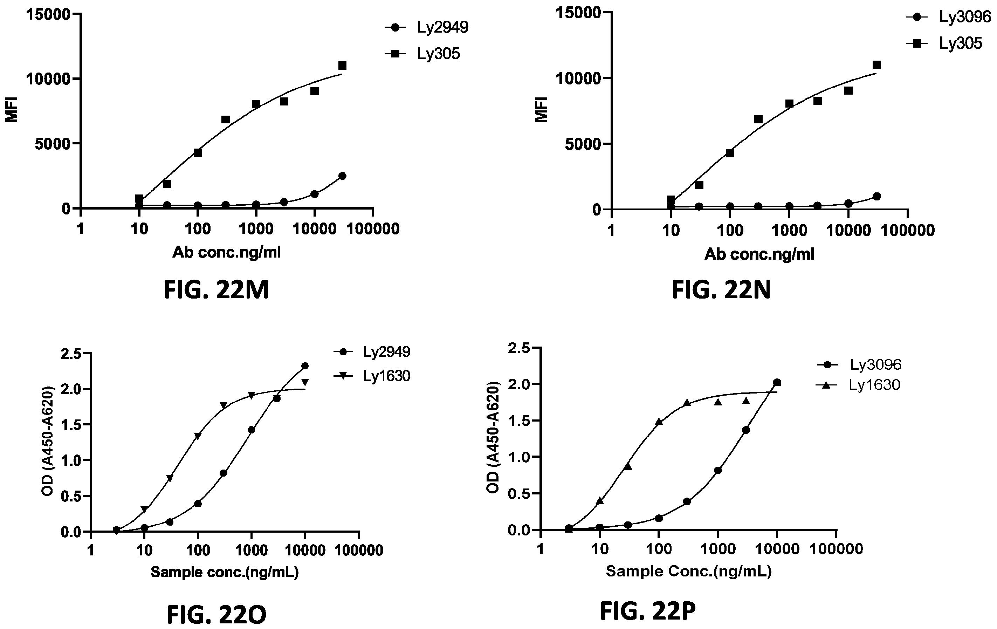

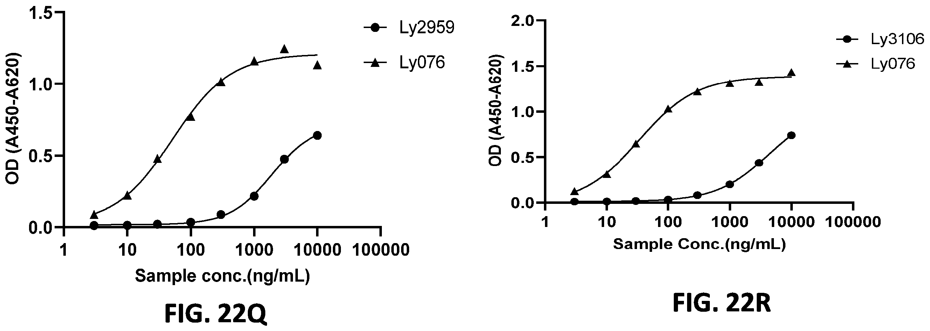

**FIGs. 22A-22R** include diagrams showing binding activities of exemplary multi-specific antibodies as indicated to BCMA, CD3, CD137 or PD-L1. **FIG. 22A**: Binding activity to human BCMA protein of antibodies Ly2949 and Ly560. **FIG. 22B**: Binding activity to human BCMA protein of antibodies Ly2959 and Ly560**.** **FIG. 22C**: Binding activity to human BCMA protein of antibodies Ly3096 and Ly560**.** **FIG. 22D**: Binding activity to human BCMA protein of antibodies Ly3106 and Ly560**.** **FIG. 22E**: Binding activity to human BCMA expressing NCI-H929 cells of antibodies Ly2949 and Ly560**.** **FIG. 22F**: Binding activity to human BCMA expressing NCI-H929 cells of antibodies Ly3096 and Ly3019**.** **FIG. 22G**: Binding activity to human BCMA overexpressing HEK293 cells of antibodies Ly2949 and Ly560. **FIG. 22H**: Binding activity to human BCMA overexpressing HEK293 cells of antibodies Ly3096 and Ly3019. **FIG. 22I**: Binding activity to human CD3D/E protein of antibodies Ly2949 and Ly305. **FIG. 22J**: Binding activity to human CD3D/E protein of antibodies Ly2959 and Ly305**. FIG. 22K**: Binding activity to human CD3D/E protein of antibodies Ly3096 and Ly305**.** **FIG. 22L**: Binding activity to human CD3D/E protein of antibodies Ly3106 and Ly305**.** **FIG. 22M**: Binding activity to CD3 expressing Jurkat cells of antibodies Ly2949, and Ly305. **FIG. 22N**: Binding activity to CD3 expressing Jurkat cells of antibodies Ly3096 and Ly305**.** **FIG. 22O**: Binding activity to human CD137 protein of antibodies Ly2949 and Ly1630. **FIG. 22P**: Binding activity to human CD137 protein of antibodies Ly3096 and Ly1630**.** **FIG. 22Q**: Binding activity to human PD-L1 protein of antibodies Ly2959 and Ly076. **FIG. 22R**: Binding activity to human PD-L1 protein of antibodies Ly3106 and Ly076. Parental anti- BCMA mAb Ly560, anti-CD3 mAb Ly305, anti- PD-L1 mAb Ly076, and anti-CD137 mAb Ly1630 were used as controls.

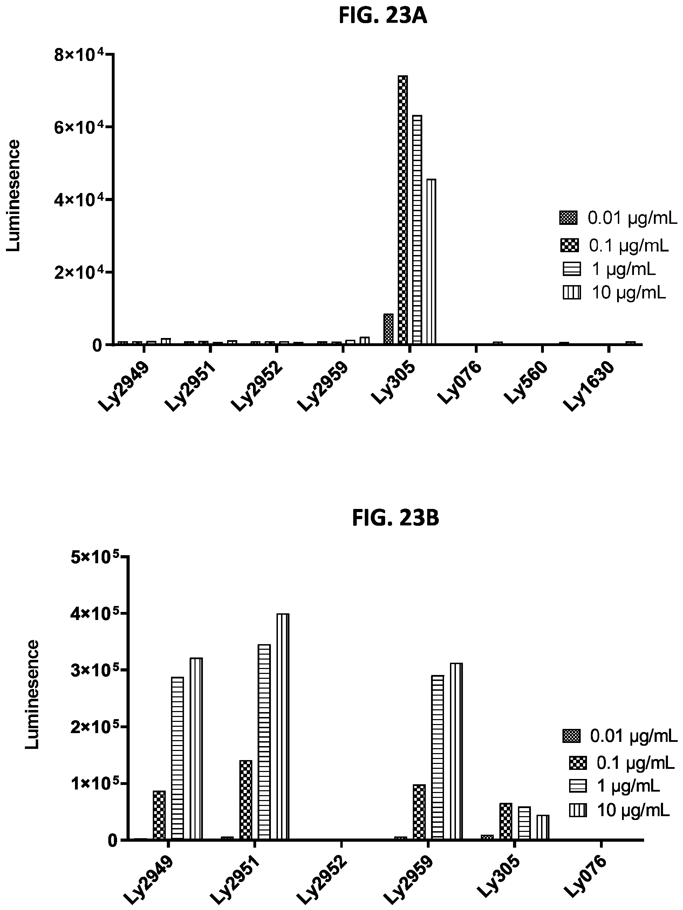

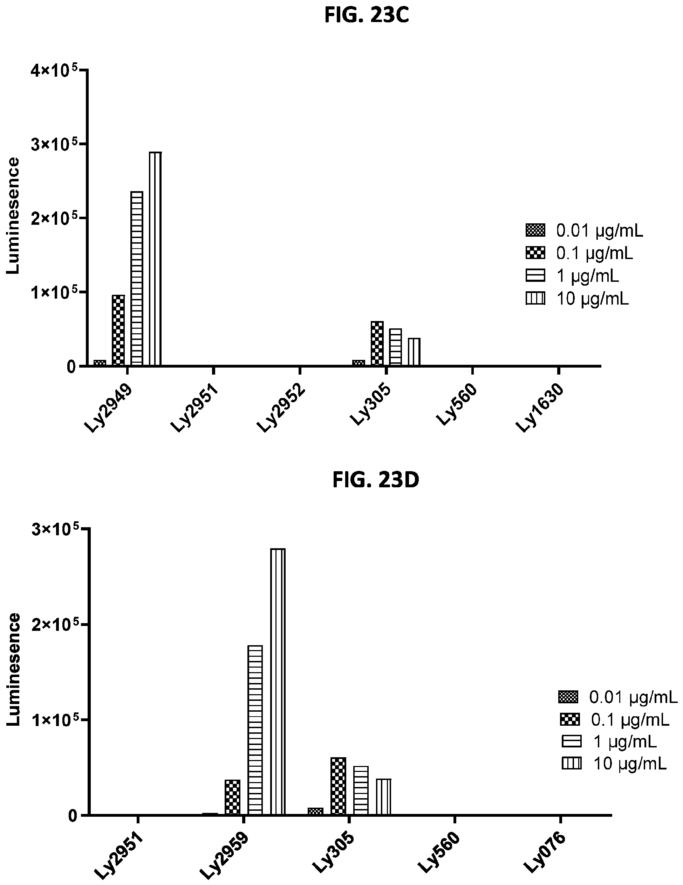

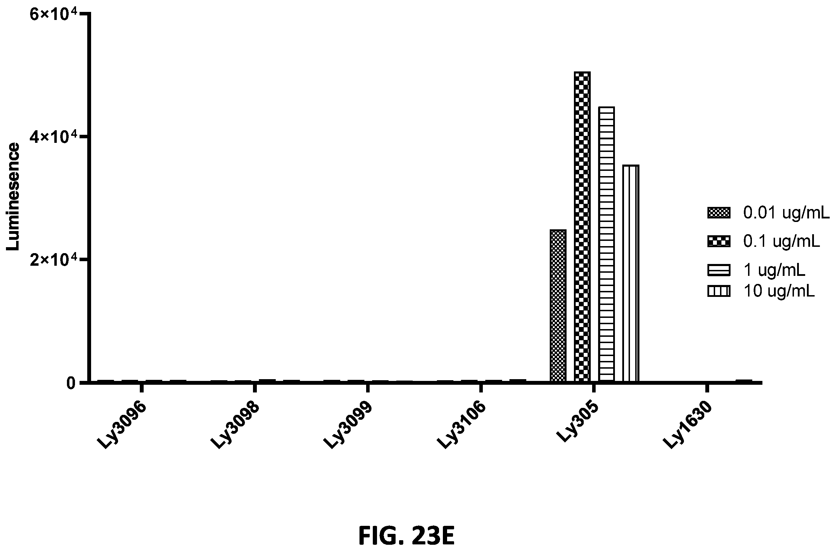

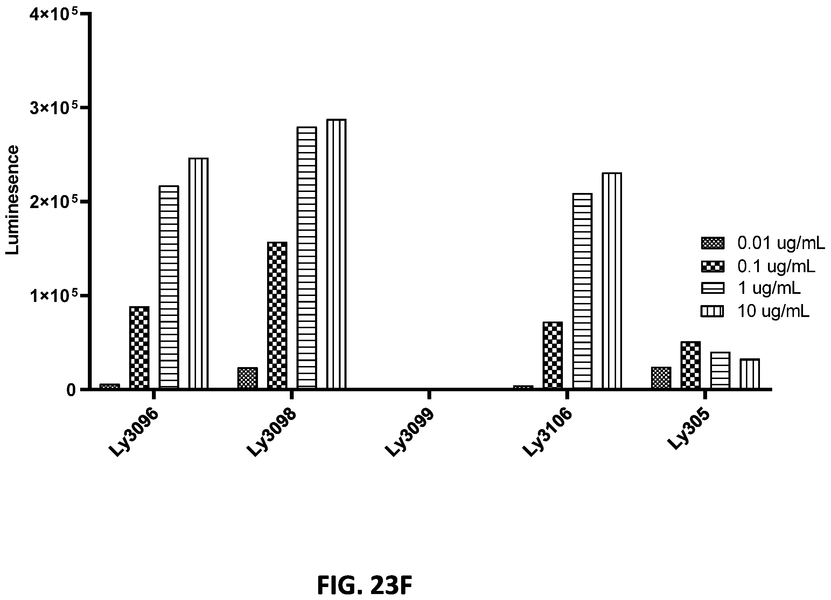

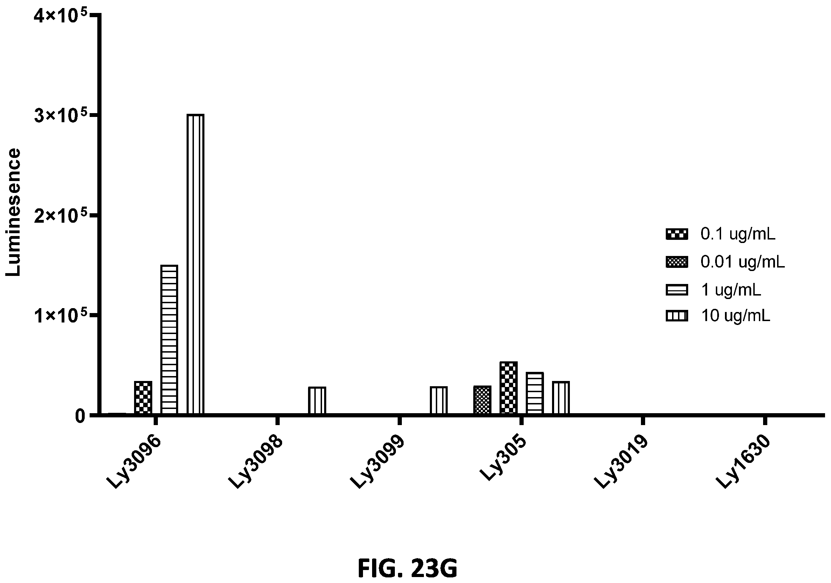

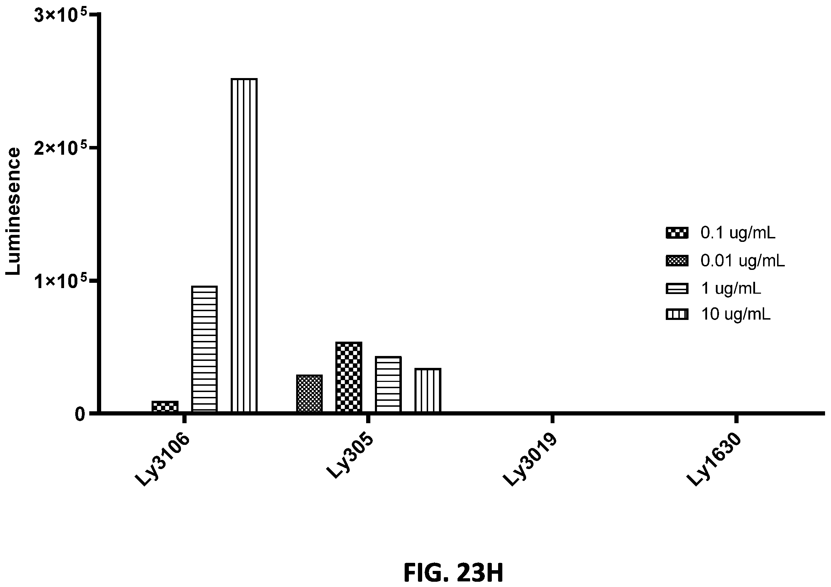

**FIGs. 23A-23H** include charts showing activation of human CD3 signaling by exemplary multi-specific antibodies as indicated in a Jurkat-NFAT-luminescence reporter asssay. **FIGs. 23A-23D**: agonistic activity of antibodies Ly2949, Ly2951, Ly2952, Ly2959, anti-PD-L1 mAb Ly076, anti-BCMA mAb Ly560, anti-CD137 mAb Ly1630 and anti-CD3 mAb Ly305 alone (**A**), co-cultured with BCMA-expressing NCI-H929 cells (**B**), co-cultured with CD137 overexpressing CHO cells (**C**), co-cultured with PD-L1 overexpressing CHO cells (**D**). **FIGs. 23E-23G**: agonistic activity of antibodies Ly3096, Ly3098, Ly3099, Ly3106, anti-CD137 mAb Ly1630 and anti-CD3 mAb Ly305 alone (**E**), co-cultured with NCI-H929 cells (**F**), co-cultured with CD137 overexpressing CHO cells (**G**), co-cultured with PD-L1 overexpressing CHO cells (**H**). The luminescence produced by reporter cells indicates CD3 activation.

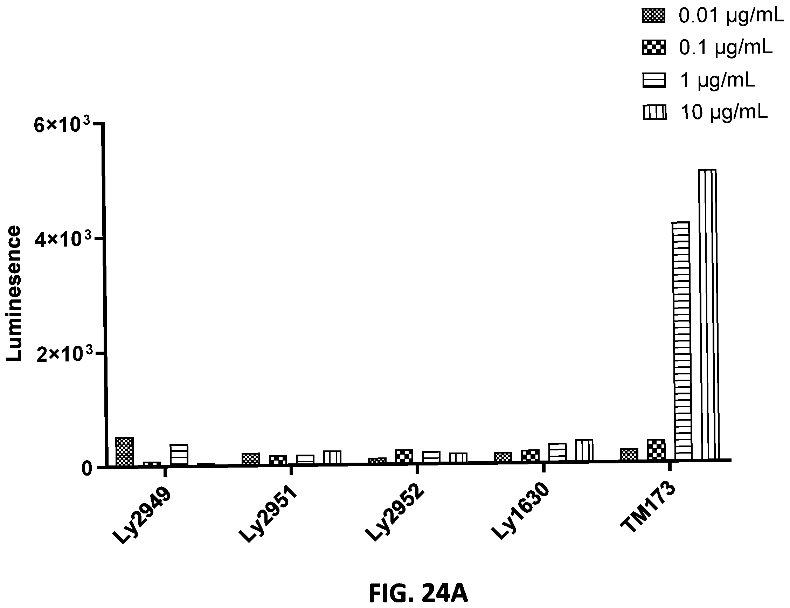

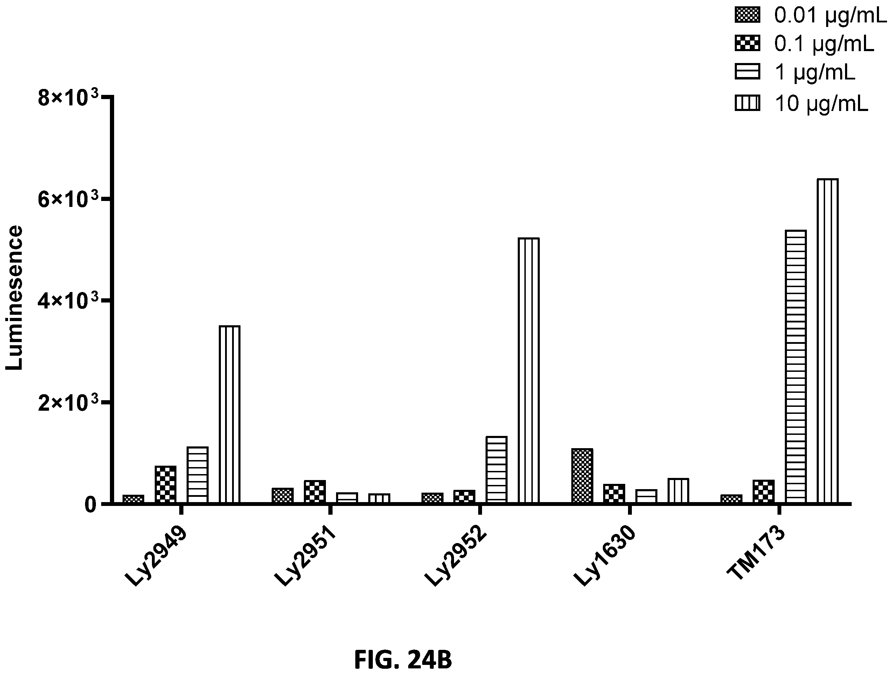

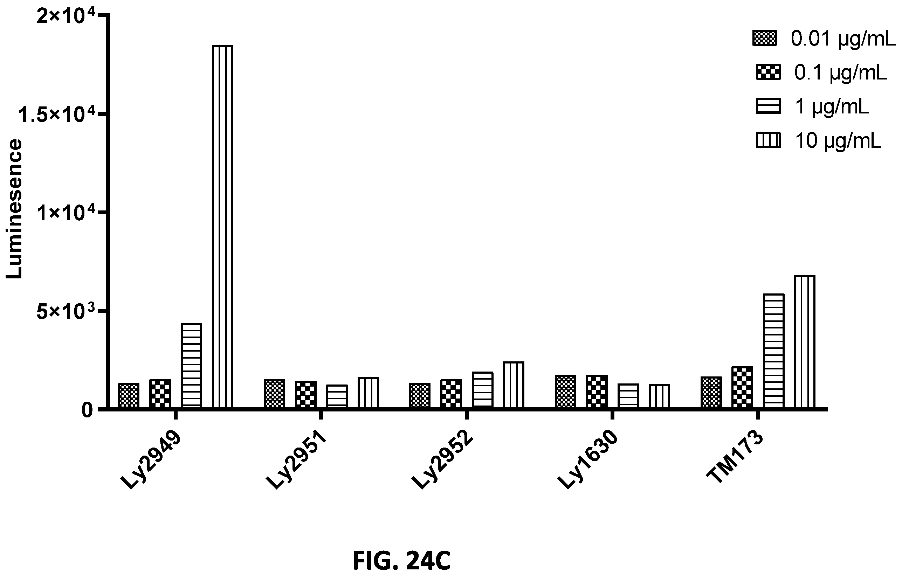

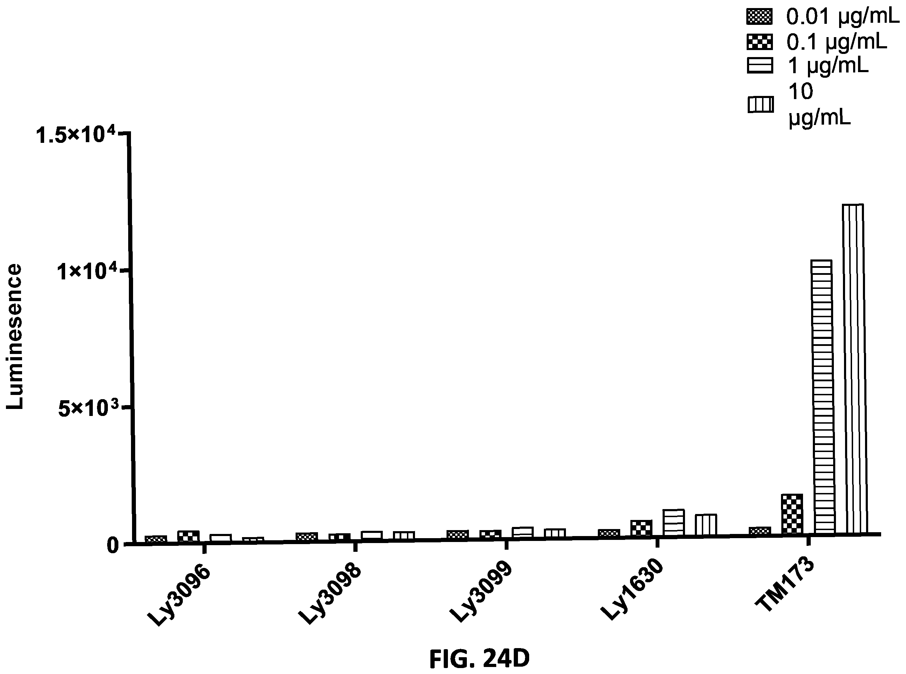

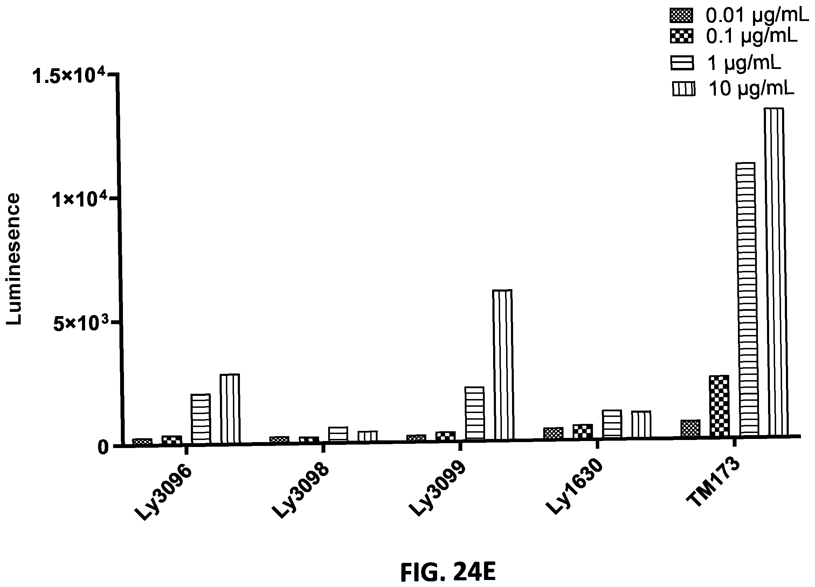

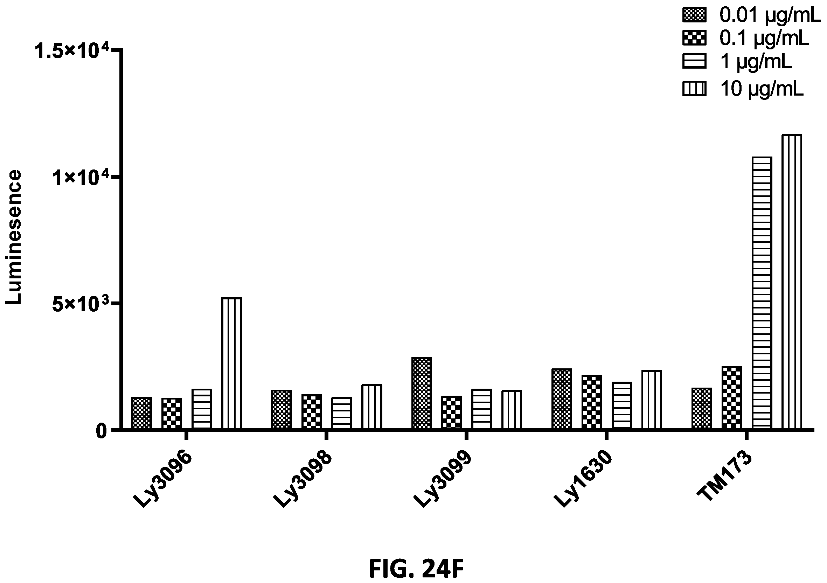

**FIGs. 24A-24F** include charts showing activation of human CD137 signaling by exemplary multi-specific antibodies as indicated in a CD137 reporter assay. **FIGs. 24A-24C**: agonistic activity of antibodies Ly2949, Ly2951, Ly2952, anti-CD137 mAb Ly1630 and anti-CD137 mAb TM173 alone (**A**), co-cultured with NCI-H929 cells (**B**), co-cultured with Jurkat cells (**C**). **FIGs. 24D-24F**: agonistic activity of antibodies Ly3096, Ly3098, Ly3099, Ly1630 and TM173 alone (**D**), co-cultured with NCI-H929 cells (**E**), co-cultured with Jurkat cells (**F**). The luminescence produced by reporter cells indicates CD137 activation.
**FIG. 25** is a diagram showing anti-tumor activities of antibodies Ly2949 and Ly2323 in human PBMC engrafted mouse model bearing NCI-H929 tumor cells.
**FIG. 26** is a diagram showing anti-tumor activities of antibodies Ly2949, Ly2951 and Ly2313 in LL2-huBCMA bearing mouse models transplanted with bone marrow from human CD3 and CD137 knock-in mice.
**FIG. 27** is a diagram showing anti-tumor activities of antibodies Ly3096, Ly3098 and Ly2314 in EL4-huBCMA bearing mouse models transplanted with bone marrow from human CD3 and CD137 knock-in mice.
**FIG. 28** is a diagram showing anti-tumor activities of antibodies Ly2949, Ly2959 and Ly2313 in human PBMC engrafted mouse model bearing NCI-H929 tumor cells.
**FIG. 29** is a diagram showing anti-tumor activities of antibodies Ly3098, Ly3106 and Ly2314 in human PBMC engrafted mouse model bearing MM.1R tumor cells.
**FIG. 30A-30F** include diagrams showing CD3 activation of anti-CD3 mAb in a Jurkat-NFAT-luminescence reporter assay alone **(A)**, with parent CHO cells **(B)**, with CHO cells expressing FCGRI **(C)**, with CHO cells expressing FCGRIIA **(D)**, with CHO cells expressing FCGRIIB **(E)**, with CHO cells expressing FCGRIIIA **(F)**. Tested antibodies were anti-CD3 mAb carrying different Fc variants as described here: Ly2863: 237 deletion; Ly2864: P329G; Ly2865: 237 deletion + P329G; Ly2873: L234A+ L235A + 237 deletion; Ly1761: IgG1 wt.
**FIG. 31** is a diagram showing CD3 activation of exemplary multi-specific antibodies in a Jurkat-NFAT-luminescence reporter assay alone **(A)**, with parent CHO cells **(B)**, with CHO cells expressing FCGRI **(C)**, with CHO cells expressing FCGRIIA **(D)**, with CHO cells expressing FCGRIIB **(E)**, with CHO cells expressing FCGRIIIA **(F)**. Tested antibodies were multi-specific antibodies carrying different Fc variants as described here: Ly2600: 237 deletion + P329G; Ly1963: L234A+ L235A + 237 deletion. Anti-CD3 mAb Ly305 was used as control.
**FIG. 32** is a diagram showing CD3 activation of exemplary multi-specific antibodies in a Jurkat-NFAT-luminescence reporter assay alone (**A**), with parent CHO cells (**B**), with CHO cells expressing FCGRI (**C**), with CHO cells expressing FCGRIIA (**D**), with CHO cells expressing FCGRIIB (**E**), with CHO cells expressing FCGRIIIA (**F**). Tested antibodies were multi-specific antibodies carrying different Fc variants as described here: Ly2601: 237 deletion + P329G; Ly1967: L234A+ L235A + 237 deletion. Anti-CD3 mAb Ly305 was used as control.

### DETAILED DESCRIPTION OF THE INVENTION

Bispecific antibodies (bsAb) targeting a tumor associated antigen (TAA) and CD3 can induce T cell activation in tumor microenvironment (TME), resulting in immune protection against tumors. However, sole CD3 activation has limitations in cancer therapy. First, pan-T cells activation via CD3 can stimulate high levels of cytokine release, which can predispose to cytokine release syndrome, which is a serious side effect of T cell engager therapy. Second, CD3 stimulation alone may promote T cell anergy or activation induced T cell death, thereby limiting the efficacy or durability of the anti-tumor response. Anti-TAA/CD3 bsAbs have shown some success in treating hematologic malignancies. However, a significant portion of the patients does not respond to treatment or eventually relapse even with initial responses.

The present disclosures provide multi-specific antibodies comprising binding moieties for two immune receptors, CD3/CD137 or CD3/PD-L1, and binding moieties to TAA(s). CD137 activation is expected to elicit a secondary signal to promote the survival of activated T cells, which may be triggered by CD3 activation. Compared with corresponding CD3 bsAb, the inclusion of a monovalent agonistic anti-CD137 moiety in exemplary multi-specific antibodies provided herein showed stronger *in vivo* antitumor efficacy. In vitro, dual CD3/CD137 activation also increased the activation of CD8 T cells, which play essential roles in protection against tumors. Enhanced CD8 T cell activation is accompanied with reduced cytokine release, suggesting a larger therapeutic window. Further, combining CD3-binding moiety with anti-PD-L1 moiety is expected to enhance anti-tumor efficacy as evidenced by the experimental data provided herein.

Further, the multi-specific antibodies provided herein may comprise an Fc fragment to connect the binding moieties therein, for example, connecting the binding moiety to CD3 and the binding moiety to CD137 or PD-L1. The Fc fragment may contain one or more mutations relative to the wild-type parent, for example, a deletion at position 237 and an amino acid substitution at position 329, following the EU numbering system (*e.g.,* Fc variant 237Δ/P329G). As shown herein, such a Fc variant doesn't bind to Fc receptors or induce Fc receptors mediated cross-linking effect thereby reducing the risk of inducing systemic immune responses or Fc effector function, which may lead to undesired side effects.

Accordingly, provided herein are multi-specific antibodies targeting tumor antigens and immune receptors (e.g., CD3/CD137 or CD3/PD-L1) and optionally involving Fc variant with little or no binding to Fc receptors, nucleic acid(s) encoding such, and host cells comprising the encoding nucleic acid(s), which can be used to product the multi-specific antibodies. Also provided herein are uses of the multi-specific antibodies in cancer therapy.

### I. Multi-Specific Antibodies

The multi-specific antibodies disclosed herein comprise antigen-binding moieties specific to two immune receptors, for example, CD3/CD137 or CD3/PD-L1, and one or more antigen-binding moieties to tumor associated antigen(s). Each of the antigen-binding moieties specific to the immune receptors is in the format of Fv, comprising a heavy chain variable domain (V_{H}) and a light chain variable domain (V_{L}). The two antigen-binding moieties specific to the immune receptors can be connected via a Fc fragment, which may comprise a hinge domain and a CH2 domain. In some instances, the Fc fragment comprises the 237Δ deletion and the amino acid substitution at position P329 (e.g., P329G), following the EU numbering system. The antigen-binding moieties specific to the TAA may be in any suitable format, for example, single chain variable fragment (scFv) or Fab. In some instances, the antigen-binding moieties are in connection with the antigen-binding moiety specific to CD3 (anti-CD3 moiety), for example, via a peptide linker. In some instances, the multi-specific antibody provided herein is multi-valent. An exemplary illustration of the multi-specific antibody is provided in

### FIG. 1.

As used herein, multi-specific antibodies refer to antibodies capable of binding to two or more target antigens. In some examples, the multi-specific antibody disclosed herein may be a bi-specific antibody, *i.e.,* binding to two different target antigens or binding to two different epitopes of a target antigen. In some examples, the multi-specific antibody disclosed herein may be a tri-specific antibody, *e.g*., binding to three different target antigens or epitopes. Alternatively, the multi-specific antibody disclosed herein may be a tetra-specific antibody, *e.g*., binding to four different target antigens or epitopes.

As used herein, multi-valent antibodies refer to antibodies having two or more antigen binding sites. In some examples, the multi-specific antibody disclosed herein may have two antigen binding sites. In some examples, the multi-specific antibody disclosed herein may have three antigen binding sites. In some examples, the multi-specific antibody disclosed herein may have four antigen binding sites.

### A. Antigen-Binding Moieties

Each of the antigen-binding moieties specific to CD3 (anti-CD3 moiety) and specific to CD137 (anti-CD137 moiety) or PD-L1 (anti-PD-L1 moiety) is in Fv format. An Fv fragment is a monovalent antibody fragment containing a V_{H} chain and a V_{L} chain, which are separate polypeptides. In some embodiments, the multi-specific antibody provided herein comprises an anti-CD3 moiety and an anti-CD137 moiety. In other embodiments, the multi-specific antibody provided herein comprises an anti-CD3 moiety and an anti-PD-L1 moiety.

Each of the antigen-binding moieties specific to TAAs (anti-TAA moiety) may be in any suitable form, including, but not limited to, intact (*i.e.,* full-length) antibodies, antigen-binding fragments thereof (such as Fab, Fab', F(ab')₂, Fv, tribody, triFabs, tandem linked Fabs, a Fab-Fv, tandem linked V domains, tandem linked scFvs, and among other formats), single chain antibodies (scFv antibodies), single domain antibody such as VHH, cross Fab, and tetravalent antibodies. In some examples, the anti-TAA moiety or moieties in the multi-specific antibodies disclosed here can be in Fab format. In other examples, the anti-TAA moiety or moieties in the multi-specific antibodies disclosed here can be in cross Fab format. Alternatively or in addition, the anti-TAA moiety or moieties in the multi-specific antibodies disclosed here can be in scFv format.

Any scFv fragment in a multi-specific antibody may be in V_{H}→V_{L} orientation. Alternatively, it can be in the V_{L}→V_{H} orientation. A single-domain antibody (sdAb), also known as a nanobody, is an antibody fragment consisting of a single monomeric variable antibody domain. In some embodiments, the single-domain antibody can be a heavy chain only (VHH) fragment, which may be derived from a camelid antibody.

A Fab fragment typically contains two separate chains, one chain containing a heavy chain variable region (VH) in connection with a heavy chain constant region fragment such as CH1 and the other chain containing a light chain variable region (VL) in connection with a light chain constant region (*e.g.,* Cκ or Cλ). A cross Fab fragment has a similar two-chain structure as a Fab fragment but has the connection between VH/VL and the heavy/light chain constant region fragment swapped. A cross Fab fragment includes a first chain containing a VH connected to a light chain constant region (*e.g*., Cκ or Cλ) and a second chain containing a VL connected to a heavy chain constant region fragment such as CH1.

A tumor associated antigen (TAA) refers to an antigen produced by tumor cells. TAAs are tumor markers for identifying tumor cells and therapeutic targets for use in cancer therapy. Exemplary TAAs include, but are not limited to, B7H3, CD19, CD20, PSMA, HER2, CEA, BCMA, p53, p53mut, DLL3, MET, EGFR, B7H4, CD20, FGF, HER2, HER3, BCMA, P53mut, MSLN, EPCAM, ROR1, MAGE (*e.g.*, MAGE-A1, MAGE-A3, MAGE-A4, MAGE-A10, MAGE-A12, MAGE-B, or MAGE-C), SSX2, CAGE, GAGE, NY-ESO-1, SPANX-A, SPANX-C, SPANX-D, PRAME, PECAM, ICAM-3 and HLA-DR, PI3K, RAS, RAF, MEK, and ERK. In some instances, the multi-specific antibody comprises one or more anti-TAA moieties capable of binding to B7H3, CD19, CD20, PSMA, HER2, CEA, BCMA, P53mut, DLL3, MET, and EGFR. In specific examples, the TAA is HER2, CEA, or BCMA.

In some examples, the multi-specific antibody provided herein comprises one anti-TAA moiety, for example, binding to B7H3, CD19, CD20, PSMA, HER2, CEA, BCMA, P53mut, DLL3, MET, and EGFR (*e.g.*, HER2, CEA, or BCMA). In other examples, the multi-specific antibody provided herein comprises two anti-TAA moieties, for example, binding to B7H3, CD19, CD20, PSMA, HER2, CEA, BCMA, P53mut, DLL3, MET, and EGFR (*e.g.*, HER2, CEA, or BCMA). The two anti-TAA moieties may bind to two different TAAs. Alternatively, the two anti-TAA moieties may bind to one TAA, e.g., binding to different epitopes of the TAA. In other examples, a multi-specific antibody provided herein comprises two identical anti-TAA moieties.

An antibody that "specifically binds" to an antigen or an epitope is a term well understood in the art. A molecule is said to exhibit "specific binding" if it reacts more frequently, more rapidly, with greater duration and/or with greater affinity with a particular target antigen than it does with alternative targets. An antibody "specifically binds" to a target antigen or epitope if it binds with greater affinity, avidity, more readily, and/or with greater duration than it binds to other substances. For example, an antibody that specifically (or preferentially) binds to an antigen (e.g., those listed above) or an antigenic epitope therein is an antibody that binds this target antigen with greater affinity, avidity, more readily, and/or with greater duration than it binds to other antigens or other epitopes in the same antigen. It is also understood with this definition that, for example, an antibody that specifically binds to a first target antigen may or may not specifically or preferentially bind to a second or third target antigen. As such, "specific binding" or "preferential binding" does not necessarily require (although it can include) exclusive binding. In some examples, an antibody that "specifically binds" to a target antigen or an epitope thereof may not bind to other antigens or other epitopes in the same antigen *(i.e.*, only baseline binding activity can be detected in a conventional method). Alternatively, or in addition, the antibodies described herein may specifically binds the human antigen or a fragment thereof as relative to the monkey counterpart, or *vice versa* (*e.g.*, having a binding affinity at least 10-fold higher to one antigen than the other as determined in the same assay under the same assay conditions). In other instances, the antibodies described herein may cross-react to human and a non-human antigen (*e.g.*, monkey), *e.g.*, the difference in binding affinity to the human and the non-human antigen is less than 5-fold, *e.g.*, less than 2-fold, or substantially similar.

In some embodiments, an antigen binding moiety in any of the multi-specific antibodies as described herein has a suitable binding affinity for the target antigen(s) (*e.g.,* the immune cell receptors such as CD3/CD137 or CD3/PD-L1 or a TAA as disclosed herein) or antigenic epitopes thereof. As used herein, "binding affinity" refers to the apparent association constant or K_{A}. The K_{A} is the reciprocal of the dissociation constant (K_{D}). The antibody described herein may have a binding affinity (K_{D}) of at least 10⁻⁵, 10⁻⁶, 10⁻⁷, 10⁻⁸, 10⁻⁹, 10⁻¹⁰ M, or lower for the target antigen or antigenic epitope. An increased binding affinity corresponds to a decreased K_{D}. Higher affinity binding of an antibody for a first antigen relative to a second antigen can be indicated by a higher K_{A} (or a smaller numerical value K_{D}) for binding the first antigen than the K_{A} (or numerical value K_{D}) for binding the second antigen. In such cases, the antibody has specificity for the first antigen (*e.g.*, a first protein in a first conformation or mimic thereof) relative to the second antigen (*e.g.*, the same first protein in a second conformation or mimic thereof; or a second protein). Differences in binding affinity (*e.g.*, for specificity or other comparisons) can be at least 1.5, 2, 3, 4, 5, 10, 15, 20, 37.5, 50, 70, 80, 91, 100, 500, 1000, 10,000 or 10⁵-fold. In some embodiments, any of the antibodies may be further affinity matured to increase the binding affinity of the antibody to the target antigen or antigenic epitope thereof.

Binding affinity (or binding specificity) can be determined by a variety of methods including equilibrium dialysis, equilibrium binding, gel filtration, ELISA, surface plasmon resonance, or spectroscopy (*e.g.*, using a fluorescence assay). Exemplary conditions for evaluating binding affinity are in HBS-P buffer (10 mM HEPES pH7.4, 150 mM NaCl, 0.005% (v/v) Surfactant P20). These techniques can be used to measure the concentration of bound binding protein as a function of target protein concentration. The concentration of bound binding protein ([Bound]) is generally related to the concentration of free target protein ([Free]) by the following equation: $\left[Bound\right] = \left[Free\right] / \left(Kd+\left[Free\right]\right)$

It is not always necessary to make an exact determination of K_{A}, though, since sometimes it is sufficient to obtain a quantitative measurement of affinity, *e.g.*, determined using a method such as ELISA or FACS analysis, is proportional to K_{A}, and thus can be used for comparisons, such as determining whether a higher affinity is, *e.g.*, 2-fold higher, to obtain a qualitative measurement of affinity, or to obtain an inference of affinity, *e.g.*, by activity in a functional assay, *e.g.*, an *in vitro* or *in vivo* assay.

### Exemplary Parent Antibodies

The antigen binding moieties of a multi-specific antibody as disclosed herein may be derived from the parent antibody specific to any of the immune cell receptor CD3, CD137, and/or PD-L1, or TAA target antigens as disclosed herein. Exemplary parent antibodies, from which any of the antigen binding moieties are derived, are provided in **Table 1** below (heavy chain and light chain CDRs based on the Kabat scheme are identified in boldface).

For example, the anti-CD3 moiety may be derived from parent anti-CD3 clone Ly305. The anti-CD137 moiety may be derived from parent anti-CD137 clone Ly1630. Alternatively, the anti-CD137 moiety may be derived from parent anti-CD137 clone TM173. The anti-PD-L1 moiety may be derived from parent anti-PD-L1 clone Ly076. Alternatively, the anti-PD-L1 moiety may be derived from parent anti-PD-L1 clone Ly2530.

Alternatively or in addition, the multi-specific antibody provided herein may comprise an anti-HER2 moiety derived from the anti-HER2 parent clone Ly591 or TM737. In some instances, the multi-specific antibody may comprise a first anti-HER2 moiety derived from Ly591 and a second anti-HER2 moiety derived from TM737.

In some instances, the multi-specific antibody provided herein may comprise an anti-CEA moiety derived from the anti-CEA parent clone Ly312.

In some instances, the multi-specific antibody provided herein may comprise an anti-BCMA moiety derived from the anti-BCMA parent clone Ly560 or Ly3019. In some instances, the multi-specific antibody may comprise a first anti-BCMA moiety derived from Ly560 and a second anti-BCMA moiety derived from Ly3019.

### Exemplary Antigen-Binding Moieties

The antigen-binding moieties in any of the multi-specific antibodies disclosed herein may be derived from any of the corresponding parent antibodies, *e.g.*, those provided in Table 1 below.

As used herein, an antigen binding moiety in a multi-specific antibody "derived from" a parent antibody means that the parent antibody is used as a starting material for making one antigen binding moiety in the multi-specific antibody. The antigen binding moiety may comprise the same heavy chain and/or light chain CDRs as those of the parent antibody. Two antibodies having the same V_{H} and/or V_{L} CDRs means that their CDRs are identical when determined by the same approach (*e.g.*, the Kabat definition, the Chothia definition, the AbM definition, and/or the contact definition as known in the art).

In some instances, an antigen binding moiety derived from a parent antibody may be a functional variant of the parent antibody. Such functional variants are substantially similar to the reference antibody, both structurally and functionally. A functional variant comprises substantially the same V_{H} and V_{L} CDRs as the reference antibody. For example, it may comprise only up to 5 (*e.g.*, 4, 3, 2, or 1) amino acid residue variations in the total heavy chain CDR regions of the reference antibody and/or comprise only up to 5 (*e.g.*, 4, 3, 2, or 1) amino acid residue variations in the total light chain CDR regions of the reference antibody. In some examples, the functional variant may comprise up to 8 (*e.g.*, 7, 6, 5, 4, 3, 2, or 1) amino acid residue variations in the total heavy and light chain CDRs relative to those of the reference antibody. Such functional variants may bind the same epitope of the antigen targeted by the parent antibody with substantially similar affinity (*e.g.*, having a K_{D} value in the same order). Alternatively, or in addition, the amino acid residue variations are conservative amino acid residue substitutions as disclosed herein.

In some embodiments, an antigen binding moiety in a multi-specific antibody as disclosed herein may comprise heavy chain CDRs that are at least 80% (*e.g.*, 85%, 90%, 95%, or 98%) sequence identity, individually or collectively, as compared with the V_{H} CDRs of the corresponding parent antibody. Alternatively, or in addition, the antigen binding moiety may comprise light chain CDRs that are at least 80% (*e.g.*, 85%, 90%, 95%, or 98%) sequence identity, individually or collectively, as compared with the V_{L} CDRs as the parent antibody.

In other embodiments, the antigen binding moiety may comprise heavy chain CDRs that are at least 80% (*e.g.*, 85%, 90%, 95%, or 98%) sequence identity, individually or collectively, as compared with the V_{H} CDRs of the corresponding parent antibody. Alternatively, or in addition, the antigen binding moiety may comprise light chain CDRs that are at least 80% (*e.g.*, 85%, 90%, 95%, or 98%) sequence identity, individually or collectively, as compared with the V_{L} CDRs as the parent antibody.

The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of the invention. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (*e.g*., XBLAST and NBLAST) can be used.

Alternatively, or in addition, the amino acid residue variations can be conservative amino acid residue substitutions. As used herein, a "conservative amino acid substitution" refers to an amino acid substitution that does not alter the relative charge or size characteristics of the protein in which the amino acid substitution is made. Variants can be prepared according to methods for altering polypeptide sequence known to one of ordinary skill in the art such as are found in references which compile such methods, *e.g*. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

### B. Format of Multi-Specific Antibodies

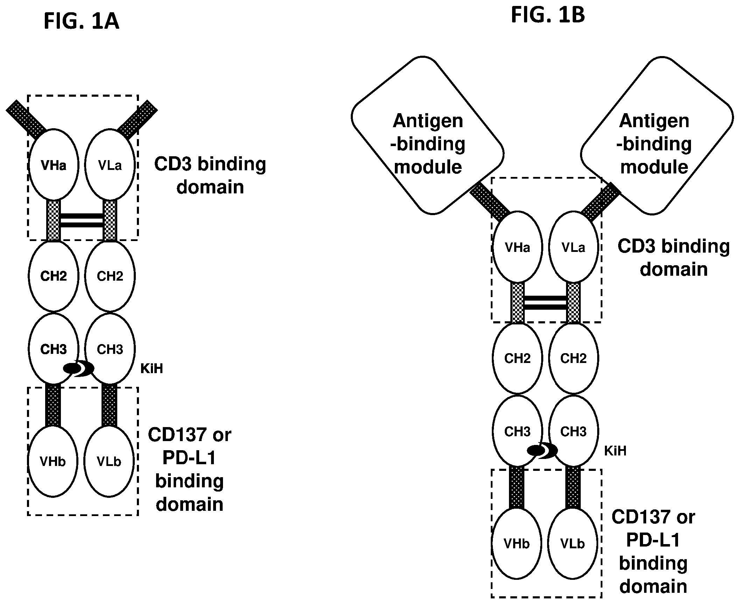

The multi-specific antibody disclosed herein may comprise a dual immune modulating construct formed by the two antigen binding moieties specific to the two immune receptors, CD3/CD137 or CD3/PD-L1, and one or more anti-TAA moieties, which can be in a suitable format such as Fab. See, *e.g.,* **FIGs. 1A** and **1B****.**

### (a) Dual Immune Modulating Construct

In the dual immune modulating construct, both the anti-CD3 moiety and the anti-CD137 or anti-PD-L1 moiety are in Fv format and the two antigen binding moieties are connected via an Fc fragment (*e.g*., an Fc variant such as those provided herein). An exemplary illustration is provided in **FIG. 1A****.**

### Fv Binding Moieties

The anti-CD3 Fv fragment disclosed herein comprises a V_{H} and a V_{L} on separate chains. Similarly, the anti-CD137 or anti-PD-L1 Fv fragment disclosed herein comprises a V_{H} and a V_{L} on separate chains. In some instances, the V_{H} of the anti-CD3 Fv may be linked to the V_{H} of the anti-CD137 or anti-PD-L1 Fv via a first Fc fragment and the V_{L} of the anti-CD3 Fv is linked to the V_{L} of the anti-CD137 or anti-PD-L1 via a second Fc fragment. In other instances, the V_{H} of the anti-CD3 Fv may be linked to the V_{L} of the anti-CD137 or anti-PD-L1 Fv via a first Fc fragment and the V_{L} of the anti-CD3 Fv is linked to the V_{H} of the anti-CD137 or anti-PD-L1 via a second Fc fragment.

The anti-CD3 Fv fragment, the anti-CD137 Fv fragment, and the anti-PD-L1 Fv fragment may be derived from any of the corresponding parent antibodies provided in **Table 1** below. See also above disclosures.

In some examples, the first and second Fc fragments are identical. In other examples, the first and second Fc fragments are different (*e.g.*, comprising matched mutations for, *e.g.*, enhancing heterodimerization over homodimerization and/or reducing protein A binding).

### Fc fragments

The Fc fragment for use to connect the anti-CD3 and anti-CD137 or anti-PD-L1 moieties may comprise a hinge domain and a CH2 domain, and optionally a CH3 domain from a suitable immunoglobin (Ig) molecule, for example, an IgG molecule. In some instances, the Fc fragment is derived from an IgG1 molecule. In some examples, the Fc fragment is a fragment of a wild-type Ig molecule (*e.g.*, IgG such as IgG1). Alternatively, the Fc fragment may contain more or more mutations relative to the wildtype counterpart.

In some embodiments, the Fc fragment is an Fc variant comprising one or more mutations relative to the wild-type counterpart. Such mutations may modulate binding affinity and selectivity to Fc receptors. In some instances, an Fc fragment may contain an amino acid substitution at one or more of positions 267, 273, 328, and 329. In some instances, the one or more mutations may be amino acid substitutions at one or more of positions 239, 265, 297, 329, 330, and 332. In one example, a heavy chain constant region fragment such as a Fc fragment may contain (i) a deletion at the position 237, and (ii) two amino acid substitutions at position 234 (*e.g.*, L234A) and 235 (*e.g.*, L235A). In another example, the heavy chain constant region fragments may comprise one or more of the following: (i) a deletion at the position 237, (ii) two amino acid substitutions selected from L234A, L235A and P329G, (iii) a deletion at position 237 and amino acid substitutions of D265A and N297A, (iv) amino acid substitutions S239D, A330L and I332E, and (v) a deletion at the position 237 and the amino acid substitution P329G. In some examples, an Fc fragment as disclosed herein may comprise (i) an amino acid substitution at position 329, which optionally is P329G, (ii) amino acid substitutions at positions 265 and 297, which optionally are D265A and N297A, (iii) amino acid substitutions at positions 239, 330, and 332, which optionally are S239D, A330L and I332E, or a combination of any one of (i)-(iii).

In one example, the Fc variant may have a deletion at position 237 (237D) and an amino acid residue substitution at position P329 (*e.g*., P329G). Such an Fc variant may comprise the amino acid sequence of SEQ ID NO: 91. Such Fc variant, its encoding nucleic acids, and antibodies comprising such are also within the scope of the present disclosure.

Additional mutations in the Fc fragment for modulating Fc receptor binding activities can be found in, *e.g*., US-2020-0392227.

Alternatively or in addition, the Fc fragment for use in constructing the multi-specific antibody provided herein may contain one or more mutations that enhance heterodimer formation. Examples include "knobs-into-holes" (Ridgway et al., Protein Engineering, 9 (7), pp. 617-21 (1996); Merchant et al., Nature Biotechnology, 16 (7), pp. 677-681 (1998)), electrostatics (Gunasekaran et al., Journal of Biological Chemistry, 285 (25), pp. 19637-19646 (2010)) or negative state designs (Kreudenstein et al., mAbs, 5 (5), pp. 646-654 (2013); Leaver-Fay et al., Structure, 24 (4), pp. 641-651 (2016)) (charged mutations). Other examples can be found in, *e.g.,* Brinkmann et al., MABS (2017), 9(2):182-212.

In some examples, the mutation may be at positions 366 (*e.g*., T366W or T366S), 368 *(e.g.,* L368A), and/or 407 *(e.g.,* G407V). In specific examples, one heavy chain constant region fragment in a multi-specific antibody may contain mutations at position 366 (*e.g*., T366W) and 407 *(e.g.,* G407V) and a second heavy chain constant region fragment in the same multi-specific antibody may contain mutations at positions 366 *(e.g.,* T366S), 368 *(e.g.,* L368A), and 407 (G407V). Unless explicitly pointed out, all numbers referring to positions in an Ig molecule follow the EU numbering system.

In some instances, mutations that reduce binding affinity to Protein A may be introduced into one or both of the heavy chain Fc regions in a multi-specific antibody to facilitate purification of the multi-specific antibodies. Such mutations are known in the art. See, *e.g*., Tustian et al., mAbs 8:828-838 (2016).

Exemplary CH3 domain sequences comprising a Knob mutation, a Hole mutation, and mutations affecting protein A binding are provided in **Table 1** below.

The dual immune modulating constructs for use in making the multi-specific antibodies provided herein comprises an anti-CD3 Fv fragment and an anti-CD137 or anti-PD-L1 Fv fragment, which are connected via an Fc fragment such as the Fc variant disclosed herein (*e.g*., an Fc variant having the 237Δ/P329G mutations). In some instances, the dual immune modulating construct contains two polypeptides. The first polypeptide comprises the VH of the anti-CD3 Fv, a first Fc fragment as disclosed herein, and the VH of the anti-CD137 or anti-PD-L1 moiety and the second polypeptide comprises the VL of the anti-CD3 Fv, a second Fc fragment as disclosed herein, and the VL of the anti-CD137 or anti-PD-L1 moiety. Alternatively, the first polypeptide comprises the VH of the anti-CD3 Fv, a first Fc fragment as disclosed herein, and the VL of the anti-CD137 or anti-PD-L1 moiety and the second polypeptide comprises the VL of the anti-CD3 Fv, a second Fc fragment as disclosed herein, and the VH of the anti-CD137 or anti-PD-L1 moiety. The first and second Fc fragments may be identical. Alternatively, they may be different (e.g., comprising the matched Knob/Hole mutations as also disclosed herein). In some instances, a peptide linker (*e.g.*, those listed in **Table 1** below) may be inserted between an antibody fragment and the Fc fragment.

In some specific examples, a dual immune modulating construct targeting CD3 and CD137 used in the multi-specific antibodies disclosed herein may comprise a first polypeptide comprising the amino acid sequence of SEQ ID NO: 3 and a second polypeptide comprising the amino acid sequence of SEQ ID NO: 4. In other specific examples, a dual immune modulating construct targeting CD3 and PD-L1 used in the multi-specific antibodies disclosed herein may comprise a first polypeptide comprising the amino acid sequence of SEQ ID NO: 5 and a second polypeptide comprising the amino acid sequence of SEQ ID NO: 6.

### (b) Anti-TAA Moieties

The one or more anti-TAA moieties in the multi-specific antibodies may be in any suitable format, see, *e.g.*, those disclosed herein. In some examples, the anti-TAA moieties are in Fab format.

The anti-TAA moieties can be linked to the anti-CD3 Fv moiety in the multi-specific antibodies via, *e.g.*, a peptide linker. In some examples, the multi-specific antibody may contain one anti-TAA moiety, which may be linked to either the VH chain of the anti-CD3 Fv or the VL chain of the anti-CD3 Fv. In some instances, the anti-TAA moiety is in Fab format, which comprises a VH-CH1 chain and a VL-CL chain. In some instances, the VH-CH1 chain may be linked to the VH chain of the anti-CD3 Fv and the VL-CL chain can be paired with the VH-CH1 chain via one or more disulfide bonds. Alternatively, the VL-CL chain can be linked to the VH chain of the anti-CD3 Fv and the VH-CL chain can be paired with the VL-CL chain via one or more disulfide bond. In other instances, the VH-CH1 chain may be linked to the VL chain of the anti-CD3 Fv and the VL-CL chain can be paired with the VH-CH1 chain via one or more disulfide bonds. Alternatively, the VL-CL chain can be linked to the VL chain of the anti-CD3 Fv and the VH-CL chain can be paired with the VL-CL chain via one or more disulfide bond.

In some embodiments, the multi-specific antibodies provided herein may comprise two anti-TAA moieties, *e.g.*, in Fab format, each of which can be linked to the VH and VL chains of the anti-CD3 Fv moiety in the multi-specific antibodies.

In some examples, the anti-TAA moieties in the multi-specific antibodies may be derived from the parent antibodies provided in **Table 1** below. See also disclosures above.

### (c) Peptide Linkers

The antigen binding moieties in the multi-specific antibodies provided herein, or fragments thereof, may be connected via peptide linkers. A peptide linker may be located between two fragments in a polypeptide of a multi-specific antibody as disclosed herein, for example, between the V_{H} and V_{L} portions in a scFv fragment, between the V_{H} or V_{L} of a Fv fragment and a chain of another antigen binding moiety, or between the VH or VL of a Fv fragment and an Fc fragment.

Any of the peptide linkers described herein can comprise naturally occurring amino acids and/or non-naturally occurring amino acids. Non-naturally occurring amino acids can include protected amino acids such as naturally occurring amino acids protected with groups such as acetyl, formyl, tosyl, nitro and the like. Non-limiting examples of non-naturally occurring amino acids include azidohomoalanine, homopropargylglycine, homoallylglycine, p-bromophenylalanine, p-iodophenylalanine, azidophenylalanine, acetylphenylalanine or ethynylephenylalanine, amino acids containing an internal alkene such as trans-crotylalkene, serine allyl ether, allyl glycine, propargyl glycine, vinyl glycine, pyrrolysine, N-sigma-o-azidobenzyloxycarbonyl-L-Lysine (AzZLys), N-sigma-propargyloxycarbonyl-L-Lysine, N-sigma-2-azidoethoxycarbonyl-L-Lysine, N-sigma-tert-butyloxycarbonyl-L-Lysine (BocLys), N-sigma-allyloxycarbonyl-L-Lysine (AlocLys), N-sigma-acetyl-L-Lysine (AcLys), N-sigma-benzyloxycarbonyl-L-Lysine (ZLys), N-sigma-cyclopentyloxycarbonyl-L-Lysine (CycLys), N-sigma-D-prolyl-L-Lysine, N-sigma-nicotinoyl-L-Lysine (NicLys), N-sigma-N-Me-anthraniloyl-L-Lysine (NmaLys), N-sigma-biotinyl-L-Lysine, N- sigma-9-fluorenylmethoxycarbonyl-L-Lysine, N-sigma-methyl-L-Lysine, N-sigma-dimethyl-L- Lysine, N-sigma-multimethyl-L-Lysine, N-sigma-isopropyl-L-Lysine, N-sigma-dansyl-L-Lysine, N-sigma-o,p-dinitrophenyl-L-Lysine, N-sigma-p-toluenesulfonyl-L-Lysine, N-sigma-DL-2-amino- 2carboxyethyl-L-Lysine, N-sigma-phenylpyruvamide-L-Lysine, N-sigma-pyruvamide-L-Lysine, azidohomoalanine, homopropargylglycine, homoallylglycine, p-bromophenylalanine, p-iodophenylalanine, azidophenylalanine, acetylphenylalanine or ethynylephenylalanine, amino acids containing and an internal alkene such as trans-crotylalkene, serine allyl ether, allyl glycine, propargyl glycine, and vinyl glycine.

The peptide linkers provided herein may contain about 5-160 amino acid residues, for example, about 10-120 amino acid residues, about 10-100 amino acid residues, about 10-80 amino acid residues, about 10-60 amino acid residues, about 10-50 amino acid residues, about 10-40 amino acid residues, about 10-30 amino acid residues, or about 10-20 amino acid residues.

In some embodiments, the peptide linker can be a flexible peptide linker, which typically contains small, flexible amino acid residues so as to connect various domains in the multi-specific antibody without affecting their binding activity. In some examples, the flexible peptide linker is a Gly-rich linker, for example, comprising the motif of (GxS)n, in which X is an integer of 1, 2, 3, 4, 5, or 6 and n is an integer of 1, 2, 3, 4, 5, 6, 7, 8, 9, or 10. Exemplary peptide linkers are provided in **Table 1** below, any of which can be used for constructing the multi-specific antibodies disclosed herein.

In some embodiments, the peptide linker may be a so-called rigid peptide linker, which contains at least one cysteine residues (*e.g.*, 1 or 2 cysteine residues) such as it can form disulfide bonds with another rigid peptide linker. Use of one or more pairs of rigid peptide linkers in the multi-specific antibodies disclosed herein can facilitate dimer formation across the multiple polypeptides of the antibody via disulfide formation, thereby forming an intact multi-chain antibody molecule.

In some instances, the rigid peptide linker may be derived from a hinge domain of an IgG molecule (positions 216-230 or a fragment thereof, following the EU numbering system), for example, an IgG1 molecule, taking advantage of the disulfide bond formation capacity of such fragments. In some examples, such a peptide linker is a fragment of a wild-type IgG molecule (*e.g.*, a human IgG1 molecule). Alternatively, the rigid peptide linker may contain one or more mutations relative to the wild-type counterpart.

In some examples, the rigid peptide linker may contain only the hinge domain of an IgG molecule or a fragment thereof. In other examples, the rigid peptide linker may contain the hinge domain or a fragment thereof and a Gly-rich fragment (*e.g.*, those disclosed herein), which can be linked to either the N-terminus and/or the C-terminus of the hinge domain or the fragment thereof. Examples of the rigid peptide linkers for use in any of the multi-specific antibodies disclosed herein are provided in **Table 1** below. See also International Application No. PCT/US2023/061051.

### C. Exemplary Multi-Specific Antibodies

In some examples, the multi-specific antibody disclosed herein comprises a dual immune modulating construct targeting CD3 and CD137, or targeting CD3 and PD-L1. Such an immune modulating construct may have the structure illustrated in **FIG. 1A**. An exemplary anti-CD3/CD137 dual immune modulating construct may comprise a first polypeptide comprising the amino acid sequence of SEQ ID NO: 3 and a second polypeptide comprising the amino acid sequence of SEQ ID NO: 4. An exemplary anti-CD3/PD-L1 construct may comprise a first polypeptide comprising the amino acid sequence of SEQ ID NO: 5 and a second polypeptide comprising the amino acid sequence of SEQ ID NO: 6.

The dual immune modulating construct may be connected to one or more anti-TAA moieties to produce the multi-specific antibodies disclosed herein. See, *e.g.*, the structure illustrated in **FIG. 1B**. In some instances, the anti-TAA moiety is a Fab fragment comprising a VH-CH1 chain and a VL-CL chain. The anti-TAA moiety can be connected to the anti-CD3 moiety. For example, the VH-CH1 chain of the anti-TAA Fab can be linked to the VH or the VL of the anti-CD3 Fv fragment in the dual immune modulating construct, and the VL-CL chain can pair with the VH-CH1 fragment to form the TAA binding moiety. A peptide linker may be inserted between the anti-TAA chain and the anti-CD3 chain.

In some examples, a multi-specific antibody provided herein may comprise (i) a first polypeptide comprising, from N-terminus to C-terminus, a VH-CH1 chain of an anti-TAA Fab, a VH of an anti-CD3 moiety, an Fc fragment, and a VH of the anti-CD137 or anti-PD-L1 moiety, (ii) a second polypeptide comprising, from N-terminus to C-terminus, the VH-CH1 chain of the anti-TAA Fab, a VL of the anti-CD3 moiety, an Fc fragment, and a VL of the anti-CD137 or anti-PD-L1 moiety, and (iii) a third polypeptide comprising a VL-CL of the anti-TAA Fab. One or more peptide linkers such as those provided in **Table 1** below can be used to link any of the two adjacent antibody fragments in any of the polypeptides of the multi-specific antibody.

In other examples, a multi-specific antibody provided herein may comprise (i) a first polypeptide comprising, from N-terminus to C-terminus, a VH-CH1 chain of a first anti-TAA Fab, a VH of an anti-CD3 moiety, an Fc fragment, and a VH of the anti-CD137 or anti-PD-L1 moiety, (ii) a second polypeptide comprising, from N-terminus to C-terminus, aVH-CH1 chain of a second anti-TAA Fab, a VL of the anti-CD3 moiety, an Fc fragment, and a VL of the anti-CD137 or anti-PD-L1 moiety, (iii) a third polypeptide comprising a VL-CL of the first anti-TAA Fab, and (iv) a fourth polypeptide comprising a VL-CL chain of the second anti-TAA Fab. One or more peptide linkers such as those provided in **Table 1** below can be used to link any of the two adjacent antibody fragments in any of the polypeptides of the multi-specific antibody.

Exemplary multi-specific antibodies as disclosed herein are provided in **Table 2** below. In specific examples, the exemplary multi-specific antibodies provided herein include Ly3151 (anti-HER2/CD3/CD137), Ly3188 (anti-HER2/CD3/PD-L1), Ly2909 (anti-CEA/CD3/CD137), Ly3060 (anti-CEA/CD3/PD-L1), Ly2949 (anti-BCMA/CD3/CD137), Ly3096 (anti-BCMA/CD3/CD137), Ly3106 (anti-BCMA/CD3/PD-L1), Ly2959 (BCMA/CD3/PD-L1), Ly3232 (anti-HER2/CD3/CD137), Ly3233 (anti-HER2/CD3/CD137), Ly3234 (anti-HER2/CD3/CD137), Ly3235 (anti-HER2/CD3/CD137), Ly3236 (anti-HER2/CD3/CD137) , Ly3353 (anti-HER2/CD3/PD-L1) , Ly3354 (anti-HER2/CD3/PD-L1) , Ly3355 (anti-HER2/CD3/PD-L1), Ly3356 (anti-HER2/CD3/PD-L1) and Ly3357 (anti-HER2/CD3/PD-L1).

### II. Methods for Preparation of Multi-Specific Antibodies

Any of the multi-specific antibodies, including bi-specific antibodies and tri-specific antibodies as described herein can be made by any method known in the art. See, for example, Harlow and Lane, (1998) Antibodies: A Laboratory Manual, Cold Spring Harbor Laboratory, New York.

In some embodiments, the multi-specific antibody may be produced via, *e.g.*, conventional recombinant technology as exemplified below.

Nucleic acids encoding the multiple chains of a multi-specific antibody as described herein can be cloned into one expression vector, each nucleotide sequence being in operable linkage to a suitable promoter. In one example, each of the nucleotide sequences encoding the heavy chain and light chain is in operable linkage to a distinct prompter. Alternatively, the nucleotide sequences encoding the multiple chains can be in operable linkage with a single promoter, such that both heavy and light chains are expressed from the same promoter. When necessary, an internal ribosomal entry site (IRES) can be inserted between the heavy chain and light chain encoding sequences.

In some examples, the nucleotide sequences encoding the multiple chains of the antibody are cloned into two or more vectors, which can be introduced into the same or different cells. When the multiple chains are expressed in different cells, each of them can be isolated from the host cells expressing such and the isolated multiple chains can be mixed and incubated under suitable conditions allowing for the formation of the multi-chain antibody.

Generally, a nucleic acid sequence encoding one or all chains of an antibody can be cloned into a suitable expression vector in operable linkage with a suitable promoter using methods known in the art. For example, the nucleotide sequence and vector can be contacted, under suitable conditions, with a restriction enzyme to create complementary ends on each molecule that can pair with each other and be joined together with a ligase. Alternatively, synthetic nucleic acid linkers can be ligated to the termini of a gene. These synthetic linkers contain nucleic acid sequences that correspond to a particular restriction site in the vector. The selection of expression vectors/promoter would depend on the type of host cells for use in producing the antibodies.

A variety of promoters can be used for expression of the antibodies described herein, including, but not limited to, cytomegalovirus (CMV) intermediate early promoter, a viral LTR such as the Rous sarcoma virus LTR, HIV-LTR, HTLV-1 LTR, the simian virus 40 (SV40) early promoter, E. coli lac UV5 promoter, and the herpes simplex tk virus promoter.

Regulatable promoters can also be used. Such regulatable promoters include those using the lac repressor from E. coli as a transcription modulator to regulate transcription from lac operator-bearing mammalian cell promoters (Brown, M. et al., Cell, 49:603-612 (1987)), those using the tetracycline repressor (tetR) (Gossen, M., and Bujard, H., Proc. Natl. Acad. Sci. USA 89:5547-5551 (1992); Yao, F. et al., Human Gene Therapy, 9:1939-1950 (1998); Shockelt, P., et al., Proc. Natl. Acad. Sci. USA, 92:6522-6526 (1995)). Other systems include FK506 dimer, VP16 or p65 using astradiol, RU486, diphenol murislerone, or rapamycin. Inducible systems are available from Invitrogen, Clontech and Ariad.

Regulatable promoters that include a repressor with the operon can be used. In one embodiment, the lac repressor from E. coli can function as a transcriptional modulator to regulate transcription from lac operator-bearing mammalian cell promoters (M. Brown et al., Cell, 49:603-612 (1987); Gossen and Bujard (1992); M. Gossen et al., Natl. Acad. Sci. USA, 89:5547-5551 (1992)) combined the tetracycline repressor (tetR) with the transcription activator (VP 16) to create a tetR-mammalian cell transcription activator fusion protein, tTa (tetR-VP 16), with the tetO-bearing minimal promoter derived from the human cytomegalovirus (hCMV) major immediate-early promoter to create a tetR-tet operator system to control gene expression in mammalian cells. In one embodiment, a tetracycline inducible switch is used. The tetracycline repressor (tetR) alone, rather than the tetR-mammalian cell transcription factor fusion derivatives can function as potent trans-modulator to regulate gene expression in mammalian cells when the tetracycline operator is properly positioned downstream for the TATA element of the CMVIE promoter (Yao et al., Human Gene Therapy, 10(16):1392-1399 (2003)). One particular advantage of this tetracycline inducible switch is that it does not require the use of a tetracycline repressor-mammalian cells transactivator or repressor fusion protein, which in some instances can be toxic to cells (Gossen et al., Natl. Acad. Sci. USA, 89:5547-5551 (1992); Shockett et al., Proc. Natl. Acad. Sci. USA, 92:6522-6526 (1995)), to achieve its regulatable effects.

Additionally, the vector can contain, for example, some or all of the following: a selectable marker gene, such as the neomycin gene for selection of stable or transient transfectants in mammalian cells; enhancer/promoter sequences from the immediate early gene of human CMV for high levels of transcription; transcription termination and RNA processing signals from SV40 for mRNA stability; SV40 polyoma origins of replication and ColE1 for proper episomal replication; internal ribosome binding sites (IRESes), versatile multiple cloning sites; and T7 and SP6 RNA promoters for in vitro transcription of sense and antisense RNA. Suitable vectors and methods for producing vectors containing transgenes are well known and available in the art.

Examples of polyadenylation signals useful to practice the methods described herein include, but are not limited to, human collagen I polyadenylation signal, human collagen II polyadenylation signal, and SV40 polyadenylation signal.

One or more vectors (e.g., expression vectors) comprising nucleic acids encoding any of the antibodies may be introduced into suitable host cells for producing the antibodies. The host cells can be cultured under suitable conditions for expression of the antibody or any polypeptide chain thereof. Such antibodies or polypeptide chains thereof can be recovered by the cultured cells (*e.g.*, from the cells or the culture supernatant) via a conventional method, *e.g.*, affinity purification. If necessary, polypeptide chains of the antibody can be incubated under suitable conditions for a suitable period of time allowing for production of the antibody.

In some embodiments, methods for preparing an antibody described herein involve a recombinant expression vector that encodes all of the multiple chains of a multi-specific antibody as also described herein. The recombinant expression vector can be introduced into a suitable host cell (*e.g.*, a dhfr- CHO cell) by a conventional method, *e.g.*, calcium phosphate-mediated transfection. Positive transformant host cells can be selected and cultured under suitable conditions allowing for the expression of the multiple polypeptide chains (*e.g.*, three or four) that form the antibody, which can be recovered from the cells or from the culture medium. When necessary, the multiple chains recovered from the host cells can be incubated under suitable conditions allowing for the formation of the multi-chain antibody.

In one example, two or more recombinant expression vectors are provided, each encoding one or more of the multiple chains of the antibody. The two or more recombinant expression vectors can be introduced into a suitable host cell *(e.g.,* dhfr- CHO cell) by a conventional method, *e.g*., calcium phosphate-mediated transfection. Alternatively, each of the expression vectors can be introduced into a suitable host cell. Positive transformants can be selected and cultured under suitable conditions allowing for the expression of the polypeptide chains of the antibody. When the two or more expression vectors are introduced into the same host cells, the antibody produced therein can be recovered from the host cells or from the culture medium. If necessary, the polypeptide chains can be recovered from the host cells or from the culture medium and then incubated under suitable conditions allowing for formation of the antibody. When the two or more expression vectors are introduced into different host cells, each of them can be recovered from the corresponding host cells or from the corresponding culture media. The multiple polypeptide chains can then be incubated under suitable conditions for formation of the antibody.

Standard molecular biology techniques are used to prepare the recombinant expression vector, transfect the host cells, select for transformants, culture the host cells and recovery of the antibodies from the culture medium. For example, some antibodies can be isolated by affinity chromatography with a Protein A or Protein G coupled matrix.

Any of the nucleic acids encoding the multiple chains of a multi-specific antibody as disclosed herein, vectors (*e.g*., expression vectors) containing such; and host cells comprising the vectors are within the scope of the present disclosure.

### III. Pharmaceutical Compositions

Any of the multi-specific antibodies disclosed herein, as well as the encoding nucleic acids or nucleic acid sets, vectors comprising such, or host cells comprising the vectors, as described herein can be mixed with a pharmaceutically acceptable carrier (excipient) to form a pharmaceutical composition for use in treating a target disease. "Acceptable" means that the carrier must be compatible with the active ingredient of the composition (and preferably, capable of stabilizing the active ingredient) and not deleterious to the subject to be treated. Pharmaceutically acceptable excipients (carriers) including buffers, which are well known in the art. See, *e.g*., Remington: The Science and Practice of Pharmacy 20th Ed. (2000) Lippincott Williams and Wilkins, Ed. K. E. Hoover.

The pharmaceutical compositions to be used in the present methods can comprise pharmaceutically acceptable carriers, excipients, or stabilizers in the form of lyophilized formulations or aqueous solutions. (Remington: The Science and Practice of Pharmacy 20th Ed. (2000) Lippincott Williams and Wilkins, Ed. K. E. Hoover). Acceptable carriers, excipients, or stabilizers are nontoxic to recipients at the dosages and concentrations used, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextran; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN, PLURONICS or polyethylene glycol (PEG).

In some examples, the pharmaceutical composition described herein comprises liposomes containing the antibodies (or the encoding nucleic acids) which can be prepared by methods known in the art, such as described in Epstein, et al., Proc. Natl. Acad. Sci. USA 82:3688 (1985); Hwang, et al., Proc. Natl. Acad. Sci. USA 77:4030 (1980); and U.S. Pat. Nos. 4,485,045 and 4,544,545. Liposomes with enhanced circulation time are disclosed in U.S. Pat. No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

The antibodies, or the encoding nucleic acid(s), may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are known in the art, see, *e.g.*, Remington, The Science and Practice of Pharmacy 20th Ed. Mack Publishing (2000).

In other examples, the pharmaceutical composition described herein can be formulated in sustained-release format. Suitable examples of sustained-release preparations include semipermeable matrices of solid hydrophobic polymers containing the antibody, matrices are in the form of shaped articles, *e.g.*, films, or microcapsules. Examples of sustained-release matrix include polyesters, hydrogels (for example, poly(2-hydroxyethyl-methacrylate), or poly(vinyl alcohol)), polylactides (U.S. Pat. No. 3,773,919), copolymers of L-glutamic acid and 7 ethyl-L-glutamate, non-degradable ethylene-vinyl acetate, degradable lactic acid-glycolic acid copolymers such as the LUPRON DEPOT (injectable microspheres composed of lactic acid-glycolic acid copolymer and leuprolide acetate), sucrose acetate isobutyrate, and poly-D-(-)-3-hydroxybutyric acid.

The pharmaceutical compositions to be used for *in vivo* administration must be sterile. This is readily accomplished by, for example, filtration through sterile filtration membranes. Therapeutic antibody compositions are generally placed into a container having a sterile access port, for example, an intravenous solution bag or vial having a stopper pierceable by a hypodermic injection needle.

The pharmaceutical compositions described herein can be in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient can be mixed with a pharmaceutical carrier, *e.g.*, conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable surface-active agents include, in particular, non-ionic agents, such as polyoxyethylenesorbitans (*e.g.*, TWEEN 20, 40, 60, 80 or 85) and other sorbitans (*e.g.*, SPAN 20, 40, 60, 80 or 85). Compositions with a surface-active agent will conveniently comprise between 0.05 and 5% surface-active agent and can be between 0.1 and 2.5%. It will be appreciated that other ingredients may be added, for example mannitol or other pharmaceutically acceptable vehicles, if necessary.

Suitable emulsions may be prepared using commercially available fat emulsions, such as INTRALIPID, LIPOSYN, INFONUTROl, LIPOFUNDIN and LIPIPHYSAN. The active ingredient may be either dissolved in a pre-mixed emulsion composition or alternatively it may be dissolved in an oil (*e.g*., soybean oil, safflower oil, cottonseed oil, sesame oil, corn oil or almond oil) and an emulsion formed upon mixing with a phospholipid *(e.g.* egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion can comprise fat droplets between 0.1 and 1.0 µm, particularly 0.1 and 0.5 µm, and have a pH in the range of 5.5 to 8.0.

The emulsion compositions can be those prepared by mixing an antibody with INTRALIPID or the components thereof (soybean oil, egg phospholipids, glycerol and water).

Pharmaceutical compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect.

Compositions in preferably sterile pharmaceutically acceptable solvents may be nebulized by use of gases. Nebulized solutions may be breathed directly from the nebulizing device or the nebulizing device may be attached to a face mask, tent or intermittent positive pressure breathing machine. Solution, suspension or powder compositions may be administered, preferably orally or nasally, from devices which deliver the formulation in an appropriate manner.

### IV. Therapeutic Applications

Any of the multi-specific antibodies disclosed herein, may be used in clinical settings *(e.g.,* therapeutic) or in non-clinical settings *(e.g.,* for research purposes).

In some aspects, provided herein are methods of using any of the multi-specific antibodies disclosed herein for modulating immune responses and/or for treating cancer in a subject in need of the treatment. To practice the method disclosed herein, an effective amount of the pharmaceutical composition described herein can be administered to a subject (*e.g.*, a human) in need of the treatment *via* a suitable route, such as intravenous administration, *e.g.*, as a bolus or by continuous infusion over a period of time, by intramuscular, intraperitoneal, intracerebrospinal, subcutaneous, intra-articular, intrasynovial, intrathecal, oral, inhalation or topical routes. Commercially available nebulizers for liquid formulations, including jet nebulizers and ultrasonic nebulizers are useful for administration. Liquid formulations can be directly nebulized and lyophilized powder can be nebulized after reconstitution. Alternatively, the antibodies as described herein can be aerosolized using a fluorocarbon formulation and a metered dose inhaler or inhaled as a lyophilized and milled powder.

The subject to be treated by the methods described herein can be a mammal, more preferably a human. Mammals include, but are not limited to, farm animals, sport animals, pets, primates, horses, dogs, cats, mice, and rats. A human subject who needs the treatment may be a human patient having, at risk for, or suspected of having a target disease/disorder, such as a cancer or an immune disorder such as an autoimmune disease.

Examples of cancers include, but are not limited to, breast cancer; biliary tract cancer; bladder cancer; brain cancer including glioblastomas and medulloblastomas; cervical cancer; choriocarcinoma; colon cancer; endomemultial cancer; esophageal cancer; gasmultic cancer; hematological neoplasms including acute lymphocytic and myelogenous leukemia, *e.g.*, B Cell CLL; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor.

In some instances, the multi-specific antibody for use in the treatment methods disclosed herein contain one binding arm specific to a TAA and the patient for treatment carries cancer cells expressing the target TAA. For example, the multi-specific antibody is specific to CD19 and the patient carries a CD19+ cancer. In other examples, the multi-specific antibody is specific to CD20 and the patient carries a CD20+ cancer. In other examples, the multi-specific antibody is specific to BCMA and the patient carries a BCMA+ cancer. In other examples, the multi-specific antibody is specific to B7H3 and the patient carries a B7H3+ cancer. In some examples, the multi-specific antibody is specific to HER2 and the patient carries a HER2+ cancer. In some examples, the multi-specific antibody is specific to p53mut and the patient carries a P53mut+ cancer. In some examples, the multi-specific antibody is specific to MET and the patient carries a MET+ cancer. In some examples, the multi-specific antibody is specific to PSMA and the patient carries a PSMA+ cancer. In some examples, the multi-specific antibody is specific to CEA and the patient carries a CEA+ cancer. In some examples, the multi-specific antibody is specific to EGFR and the patient carries an EGFR+ cancer. In some examples, the multi-specific antibody is specific to DLL3 and the patient carries a DLL3+ cancer. In some examples, the multi-specific antibody is specific to MAGE-A4 and the patient carries a MAGE-A4+ cancer. In some examples, the multi-specific antibody is specific to PRAME and the patient carries a PRAME+ cancer.

A subject having a target cancer can be identified by routine medical examination, *e.g.*, laboratory tests, organ functional tests, CT scans, ultrasounds, and/or genetic testing. In some embodiments, the subject to be treated by the method described herein may be a human cancer patient who has undergone or is subjecting to an anti-cancer therapy, for example, chemotherapy, radiotherapy, immunotherapy, or surgery.

A subject suspected of having any of such target disease/disorder such as cancer might show one or more symptoms of the disease/disorder. A subject at risk for the disease/disorder can be a subject having one or more of the risk factors for that disease/disorder.

As used herein, "an effective amount" refers to the amount of each active agent required to confer therapeutic effect on the subject, either alone or in combination with one or more other active agents. Determination of whether an amount of the antibody achieved the therapeutic effect would be evident to one of skill in the art. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment.

Empirical considerations, such as the half-life, generally will contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally, but not necessarily, based on treatment and/or suppression and/or amelioration and/or delay of a target disease/disorder. Alternatively, sustained continuous release formulations of an antibody may be appropriate. Various formulations and devices for achieving sustained release are known in the art.

In one example, dosages for an antibody as described herein may be determined empirically in individuals who have been given one or more administration(s) of the antibody. Individuals are given incremental dosages of the agonist. To assess efficacy of the agonist, an indicator of the disease/disorder can be followed.

Generally, for administration of any of the antibodies described herein, an initial candidate dosage can be about 2 mg/kg. For the purpose of the present disclosure, a typical daily dosage might range from about any of 0.1 µg/kg to 3 µg/kg to 30 µg/kg to 300 µg/kg to 3 mg/kg, to 30 mg/kg to 100 mg/kg or more, depending on the factors mentioned above. For repeated administrations over several days or longer, depending on the condition, the treatment is sustained until a desired suppression of symptoms occurs or until sufficient therapeutic levels are achieved to alleviate a target disease or disorder, or a symptom thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner wishes to achieve. For example, dosing from one-four times a week is contemplated. In some embodiments, dosing ranging from about 3 µg/mg to about 2 mg/kg (such as about 3 µg/mg, about 10 µg/mg, about 30 µg/mg, about 100 µg/mg, about 300 µg/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy is easily monitored by conventional techniques and assays. The dosing regimen (including the antibody used) can vary over time.

In some embodiments, for an adult patient of normal weight, doses ranging from about 0.003 to 5.00 mg/kg may be administered. In some examples, the dosage of the antibody described herein can be 10 mg/kg. The particular dosage regimen, *i.e.*, dose, timing and repetition, will depend on the particular individual and that individual's medical history, as well as the properties of the individual agents (such as the half-life of the agent, and other considerations well known in the art).

For the purpose of the present disclosure, the appropriate dosage of an antibody as described herein will depend on the specific antibody, antibodies, and/or non-antibody peptide (or compositions thereof) employed, the type and severity of the disease/disorder, whether the antibody is administered for preventive or therapeutic purposes, previous therapy, the patient's clinical history and response to the agonist, and the discretion of the attending physician. Typically, the clinician will administer an antibody, until a dosage is reached that achieves the desired result. In some embodiments, the desired result is an increase in anti-tumor immune response in the tumor microenvironment. Methods of determining whether a dosage resulted in the desired result would be evident to one of skill in the art. Administration of one or more antibodies can be continuous or intermittent, depending, for example, upon the recipient's physiological condition, whether the purpose of the administration is therapeutic or prophylactic, and other factors known to skilled practitioners. The administration of an antibody may be essentially continuous over a preselected period of time or may be in a series of spaced dose, *e.g.*, either before, during, or after developing a target disease or disorder.

As used herein, the term "treating" refers to the application or administration of a composition including one or more active agents to a subject, who has a target disease or disorder, a symptom of the disease/disorder, or a predisposition toward the disease/disorder, with the purpose to cure, heal, alleviate, relieve, alter, remedy, ameliorate, improve, or affect the disorder, the symptom of the disease, or the predisposition toward the disease or disorder.

Alleviating a target disease/disorder includes delaying the development or progression of the disease or reducing disease severity or prolonging survival. Alleviating the disease or prolonging survival does not necessarily require curative results. As used therein, "delaying" the development of a target disease or disorder means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given time frame and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detectable and assessed using standard clinical techniques as well known in the art. However, development also refers to progression that may be undetectable. For purpose of this disclosure, development or progression refers to the biological course of the symptoms. "Development" includes occurrence, recurrence, and onset. As used herein "onset" or "occurrence" of a target disease or disorder includes initial onset and/or recurrence.

Conventional methods, known to those of ordinary skill in the art of medicine, can be used to administer the pharmaceutical composition to the subject, depending upon the type of disease to be treated or the site of the disease. This composition can also be administered via other conventional routes, *e.g.*, administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, it can be administered to the subject via injectable depot routes of administration such as using 1-, 3-, or 6-month depot injectable or biodegradable materials and methods. In some examples, the pharmaceutical composition is administered intraocularly or intravitreally.

Injectable compositions may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and a physiologically acceptable excipient is infused. Physiologically acceptable excipients may include, for example, 5% dextrose, 0.9% saline, Ringer's solution or other suitable excipients. Intramuscular preparations, *e.g.*, a sterile formulation of a suitable soluble salt form of the antibody, can be dissolved and administered in a pharmaceutical excipient such as Water-for-Injection, 0.9% saline, or 5% glucose solution.

In one embodiment, an antibody is administered *via* site-specific or targeted local delivery techniques. Examples of site-specific or targeted local delivery techniques include various implantable depot sources of the antibody or local delivery catheters, such as infusion catheters, an indwelling catheter, or a needle catheter, synthetic grafts, adventitial wraps, shunts and stents or other implantable devices, site specific carriers, direct injection, or direct application. See, *e.g.*, PCT Publication No. WO 00/53211 and U.S. Pat. No. 5,981,568.

Targeted delivery of therapeutic compositions containing an antisense polynucleotide, expression vector, or subgenomic polynucleotides can also be used. Receptor-mediated DNA delivery techniques are described in, for example, Findeis et al., Trends Biotechnol. (1993) 11:202; Chiou et al., Gene Therapeutics: Methods and Applications Of Direct Gene Transfer (J. A. Wolff, ed.) (1994); Wu et al., J. Biol. Chem. (1988) 263:621; Wu et al., J. Biol. Chem. (1994) 269:542; Zenke et al., Proc. Natl. Acad. Sci. USA (1990) 87:3655; Wu et al., J. Biol. Chem. (1991) 266:338.

Therapeutic compositions containing a polynucleotide (*e.g.*, those encoding the antibodies described herein) are administered in a range of about 100 ng to about 200 mg of DNA for local administration in a gene therapy protocol. In some embodiments, concentration ranges of about 500 ng to about 50 mg, about 1 µg to about 2 mg, about 5 µg to about 500 µg, and about 20 µg to about 100 µg of DNA or more can also be used during a gene therapy protocol.

The therapeutic polynucleotides and polypeptides described herein can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters and/or enhancers. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, *e.g.*, PCT Publication Nos. WO 90/07936; WO 94/03622; WO 93/25698; WO 93/25234; WO 93/11230; WO 93/10218; WO 91/02805; U.S. Pat. Nos. 5,219,740 and 4,777,127; GB Patent No. 2,200,651; and EP Patent No. 0 345 242), alphavirusbased vectors (*e.g.*, Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, *e.g.*, PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, *e.g.*, Curiel, Hum. Gene Ther. (1992) 3:147); ligand-linked DNA (see, *e.g.*, Wu, J. Biol. Chem. (1989) 264:16985); eukaryotic cell delivery vehicles cells (see, *e.g.*, U.S. Pat. No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Pat. No. 5,580,859. Liposomes that can act as gene delivery vehicles are described in U.S. Pat. No. 5,422,120; PCT Publication Nos. WO 95/13796; WO 94/23697; WO 91/14445; and EP Patent No. 0524968. Additional approaches are described in Philip, Mol. Cell. Biol. (1994) 14:2411, and in Woffendin, Proc. Natl. Acad. Sci. (1994) 91:1581.

The particular dosage regimen, *i.e.*, dose, timing and repetition, used in the method described herein will depend on the particular subject and that subject's medical history.

In some embodiments, more than one antibody, or a combination of an antibody and another suitable therapeutic agent, may be administered to a subject in need of the treatment. The antibody can also be used in conjunction with other agents that serve to enhance and/or complement the effectiveness of the agents. Treatment efficacy for a target disease/disorder can be assessed by methods well-known in the art.

When any of the antibodies described herein is used for treating a cancer, it can be combined with an anti-cancer therapy, for example, those known in the art. Additional anti-cancer therapy includes chemotherapy, surgery, radiation, immunotherapy, gene therapy, and so forth.

Alternatively, the treatment of the present disclosure can be combined with a chemotherapeutic agent, for example, pyrimidine analogs (5-fluorouracil, floxuridine, capecitabine, gemcitabine and cytarabine), purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethyhnelamineoxaliplatin, iphosphamide, melphalan, merchlorehtamine, mitomycin, mitoxantrone, nitrosourea, plicamycin, procarbazine, taxol, taxotere, teniposide, multiethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes-dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (*e.g.*, TNP-470, genistein, bevacizumab) and growth factor inhibitors (*e.g.*, fibroblast growth factor (FGF) inhibitors); angiotensin receptor blocker; nimultic oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; and chromatin disruptors.

When any of the antibodies described herein is for use in treating an immune disorder, it can be co-used with other immunomodulatory treatments such as, *e.g.*, therapeutic vaccines (including but not limited to GVAX, DC-based vaccines, *etc*.), or checkpoint inhibitors (including but not limited to agents that block CTLA4, PD1, LAG3, TIM3, *etc*.). In some instances, the antibody can be combined with another therapy for autoimmune diseases. Examples include, but are not limited to, intravenous Ig therapy; nonsteroidal antiinflammatory drugs (NSAID); corticosteroids; cyclosporins, rapamycins, ascomycins; cyclophosphamide; azathioprene; methotrexate; brequinar; FTY 720; leflunomide; mizoribine; mycophenolic acid; mycophenolate mofetil; 15-deoxyspergualine; an immunosuppressive agent, or an adhesion molecule inhibitor.

For examples of additional useful agents see also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

When a second therapeutic agent is used, such an agent can be administered simultaneously or sequentially (in any order) with the therapeutic agent described herein. When co-administered with an additional therapeutic agent, suitable therapeutically effective dosages for each agent may be lowered due to the additive action or synergy.

### V. Kits Comprising Multi-Specific Antibodies Disclosed Herein

The present disclosure also provides kits for use in treating or alleviating a target disease, such as cancer or immune disorders as described herein. Such kits can include one or more containers comprising any of the multi-specific antibodies disclosed herein, and optionally a second therapeutic agent to be co-used with the antibody, which is also described herein.

In some embodiments, the kit can comprise instructions for use in accordance with any of the methods described herein. The included instructions can comprise a description of administration of the antibody, and optionally the second therapeutic agent, to treat, delay the onset, or alleviate a target disease as those described herein. The kit may further comprise a description of selecting an individual suitable for treatment based on identifying whether that individual has the target disease, *e.g.,* applying the diagnostic method as described herein. In still other embodiments, the instructions comprise a description of administering an antibody to an individual at risk of the target disease.

The instructions relating to the use of an antibody generally include information as to dosage, dosing schedule, and route of administration for the intended treatment. The containers may be unit doses, bulk packages (*e.g.*, multi-dose packages) or sub-unit doses. Instructions supplied in the kits of the invention are typically written instructions on a label or package insert (*e.g.*, a paper sheet included in the kit), but machine-readable instructions (*e.g.*, instructions carried on a magnetic or optical storage disk) are also acceptable.

The label or package insert indicates that the composition is used for treating, delaying the onset and/or alleviating the disease, such as cancer or immune disorders (*e.g.*, an autoimmune disease). Instructions may be provided for practicing any of the methods described herein.

The kits of this invention are in suitable packaging. Suitable packaging includes, but is not limited to, vials, bottles, jars, flexible packaging (*e.g.*, sealed Mylar or plastic bags), and the like. Also contemplated are packages for use in combination with a specific device, such as an inhaler, nasal administration device (*e.g.*, an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is an antibody as those described herein.

Kits may optionally provide additional components such as buffers and interpretive information. Normally, the kit comprises a container and a label or package insert(s) on or associated with the container. In some embodiments, the invention provides articles of manufacture comprising contents of the kits described above.

### General techniques

The practice of the present disclosure will employ, unless otherwise indicated, conventional techniques of molecular biology (including recombinant techniques), microbiology, cell biology, biochemistry, and immunology, which are within the skill of the art. Such techniques are explained fully in the literature, such as Molecular Cloning: A Laboratory Manual, second edition (Sambrook, et al., 1989) Cold Spring Harbor Press; Oligonucleotide Synthesis (M. J. Gait, ed. 1984); Methods in Molecular Biology, Humana Press; Cell Biology: A Laboratory Notebook (J. E. Cellis, ed., 1989) Academic Press; Animal Cell Culture (R. I. Freshney, ed. 1987); Introuction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds. 1993-8) J. Wiley and Sons; Methods in Enzymology (Academic Press, Inc.); Handbook of Experimental Immunology (D. M. Weir and C. C. Blackwell, eds.): Gene Transfer Vectors for Mammalian Cells (J. M. Miller and M. P. Calos, eds., 1987); Current Protocols in Molecular Biology (F. M. Ausubel, et al. eds. 1987); PCR: The Polymerase Chain Reaction, (Mullis, et al., eds. 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practice approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds. Harwood Academic Publishers, 1995); DNA Cloning: A practical Approach, Volumes I and II (D.N. Glover ed. 1985*);* Nucleic Acid Hybridization (B.D. Hames & S.J. Higgins eds.(1985»; Transcription and Translation (B.D. Hames & S.J. Higgins, eds. (1984»; Animal Cell Culture (R.I. Freshney, ed. (1986»*;* Immobilized Cells and Enzymes (lRL Press, (1986»; and B. Perbal, A practical Guide To Molecular Cloning (1984); F.M. Ausubel et al. (eds.).

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever.

### EXAMPLES

### Example 1: Construction and Production of Multi-Specific and Multivalent Antibodies

Exemplary multi-specific antibodies shown in **Table 2** were produced by recombinant technology and characterized for their antigen binding activities and bioactivities as disclosed herein.

Briefly, cDNAs encoding the dual anti-CD3/CD137 (SEQ ID NO: 3-4) or anti-CD3/PD-L1 (SEQ ID NO: 5-6) immune modulating constructs illustrated in **FIG. 1A** were used as the starting materials for making the multi-specific antibodies exampled in **FIG. 1B****.** The VH and VL sequences from parent antibody clones shown in **Table 1** were used for constructing the exemplary multi-specific antibodies. The coding sequences of the multiple chains of each exemplary multi-specific antibodies (see **Table 2)** were cloned into expression vectors, which were transfected into CHO cells for transient expression. The multi-specific antibodies produced by CHO cells were purified from the culture supernatant using Protein A affinity chromatography. Antibody properties were examined using standard protocols or as described herein.

### Example 2: Characterization of Dual Anti-CD3/CD137 Immune Modulating Constructs

This example explores activities of the dual immune modulating construct described in **Example 1** above, which binds to CD3 and CD137 and its impact on multi-specific antibodies comprising such.

Exemplary multi-specific antibodies were produced in CHO cells as described in **Example 1** above. Several multi-specific antibodies were constructed, using the anti-CD3/CD137 dual modulators as the backbone and including further binding moieties to tumor antigens such as HER2, CEA or BCMA. The format of such multi-specific antibodies is illustrated in **FIG. 1B**.

### (i) Tumor Cell Killing

Tumor cell killing by immune cells induced by exemplary anti-CEA/CD3/CD137 multi-specific antibody Ly2909, containing the dual CD3/CD137 modulating construct, is examined *in vitro*. Briefly, LS174T-Luc cells and human PBMCs were mixed and incubated in the presence of various test antibodies for 48 hours and luminescence from tumor cells was measured using kit from Promega following the instructions. Cytotoxicity of test antibodies were reversely correlated to luminescence intensity. A CEA/CD3 bispecific antibody Ly2323 is used as control.

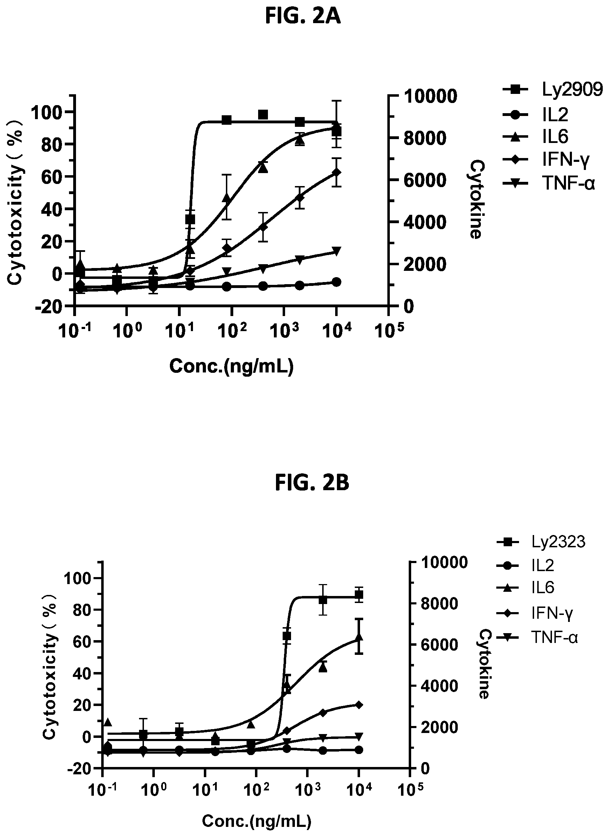

**FIGs. 2A-2B** provide a summary of the results obtained from the *in vitro* assay described above. Ly2909 showed stronger cytotoxicity than Ly2323, suggesting a contribution from the additional CD137 signaling induced by the dual immune modulating construct. In addition, less cytokine release is induced by Ly2909 than Ly2323 at their relative tumor-killing effective dosage, suggesting Ly2909 has a larger therapeutic window than that of Ly2323, as cytokine profiles can predict adverse reactions.

### (ii) CD8 T Cell Activation In Vitro

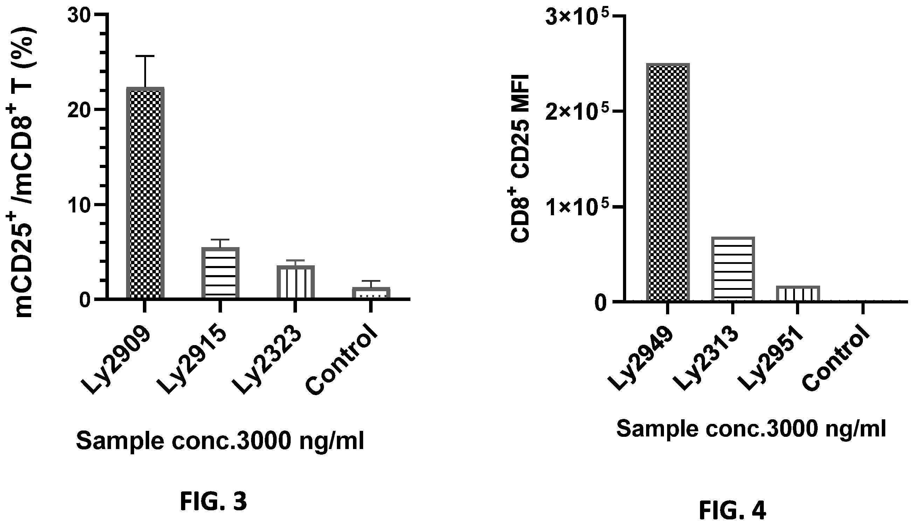

To determine whether multi-specific antibodies containing the dual CD3/CD137 immune modulating construct could enhance cellular immune functions, the *in vitro* phenotypes of T cells were evaluated. Human CEA expressing MC38 cells and splenocytes from human CD3/CD137 knock-in (KI) mice were mixed and incubated for 3 days in the presence of exemplary anti-CEA/CD3/CD137 multi-specific antibody Ly2909 or CEA/CD3 bispecific antibodies Ly2915 or Ly2323. Ly2909 induced activation of CD8 T cells in a level much greater than that induced by Ly2915 or Ly2323 as shown in **FIG. 3**. Similarly, an anti-BCMA/CD3/CD137 multi-specific antibody Ly2949 stimulates CD8 cell activation in a level much greater than that induced by BCMA /CD3 bispecific antibodies control Ly2951 or Ly2313 as shown in **FIG. 4**. These data indicate that dual CD3/CD137 activation is necessary to maximize CD8 T cell activation that is likely to enhance antitumor activity.

### (iii) Antitumor Efficacy

To determine whether multi-specific antibodies containing the dual anti-CD3/CD137 modulating construct could protect better than CD3 bispecific antibody (bsAb) against tumor growth *in vivo*, antitumor efficacy was examined in bone marrow transplanted syngeneic mouse models, inoculated with human BCMA expressing EL4, LL2 cell line respectively. Thirteen or five days after tumor cell inoculation, mice were grouped, and test articles were injected as indicated. Mice were weighed and tumor growth was measured twice weekly using calipers. Tumor volume was estimated as 1/2 x length x width².

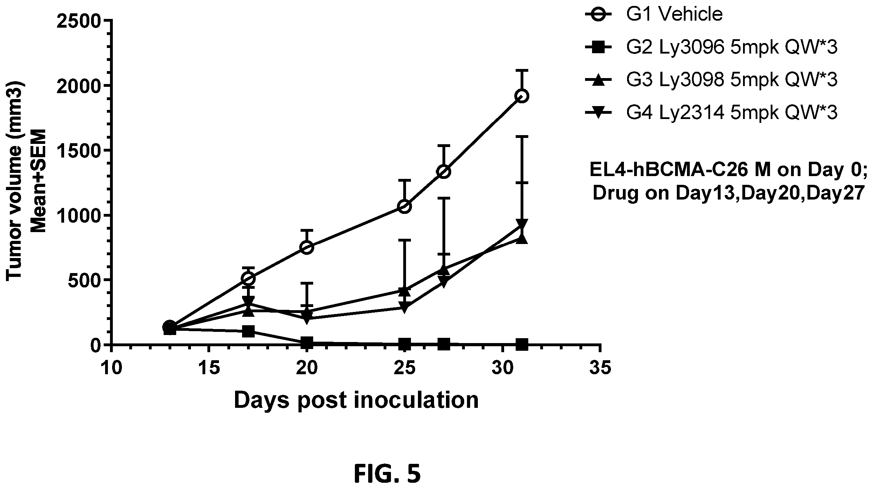

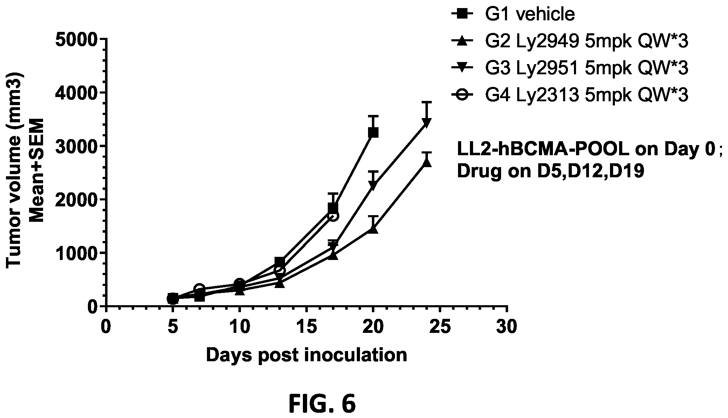

Stronger antitumor activities were achieved by Ly3096 as compared with the bispecific anti-BCMA/CD3 bsAb control Ly2314 and Ly3098, as shown in **FIG. 5**. Similarly, Ly2949 showed stronger antitumor activities than anti-BCMA CD3 bsAb control Ly2951 and Ly2313, as shown in **FIG. 6**. The data further demonstrate the contribution of dual CD3/CD137 activation to *in vivo* anti-tumor efficacy,

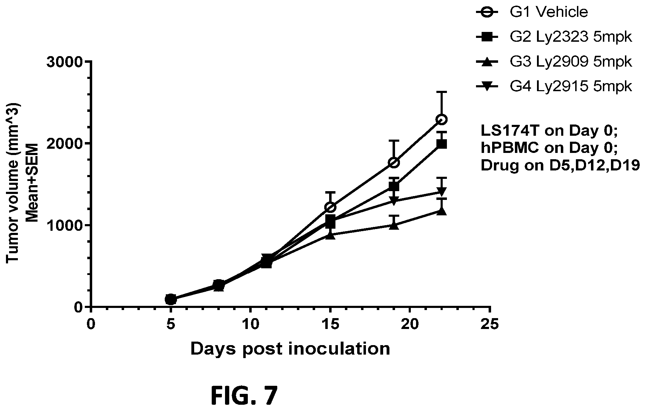

Moreover, antitumor efficacy was examined in human PBMC engrafted mouse models. Human CEA expressing LS174T tumors were inoculated *s.c.* and human PBMC were inoculated i.v. on day 0. Five days after tumor cell inoculation, mice were grouped, and test articles were injected *i.p.* weekly. Mice were weighed and tumor growth was measured twice weekly using calipers. Tumor volume was estimated as 1/2(length x width²). Stronger antitumor activity was achieved by exemplary multi-specific antibody Ly2909 as compared with anti-CEA/CD3 bsAb Ly2915 and Ly2323 as shown in **FIG. 7**, suggesting benefits of dual CD3/CD137 activation in antitumor activity.

In sum, multi-specific antibodies tested herein, containing the dual CD3/CD137 modulating construct provided in Example 1 above and binding moieties targeting a tumor antigen, showed more desired *in vitro* activities (*e.g.*, enhanced T cell activation) and stronger *in vivo* antitumor efficacy relative to bispecific antibodies lacking moieties activating the CD137 signaling.

### Example 3: Characterization of Dual Anti-CD3/PD-L1 Immune Modulating Constructs

This example explores activities of the dual immune modulating construct described in **Example 1** above, which binds to CD3 and PD-L1 and its impact on multi-specific antibodies comprising such.

Exemplary multi-specific antibodies were tested for their binding to involved tumor antigen and the immune targets CD3 and PD-L1. The cytotoxic assays were performed using a co-culture assay of tumor cells and hPBMCs.

To assess the contribution of additional PD-L1 binding effect in multi-specific antibodies in their *in vivo* antitumor efficacy, exemplary multi-specific antibodies containing the dual CD3/PD-L1 immune modulating construct and binding moieties to tumor antigens such as CEA and BCMA were constructed. Their antitumor efficacy was examined in human PBMC engrafted mouse models inoculated with cell lines expressing the corresponding tumor antigen.

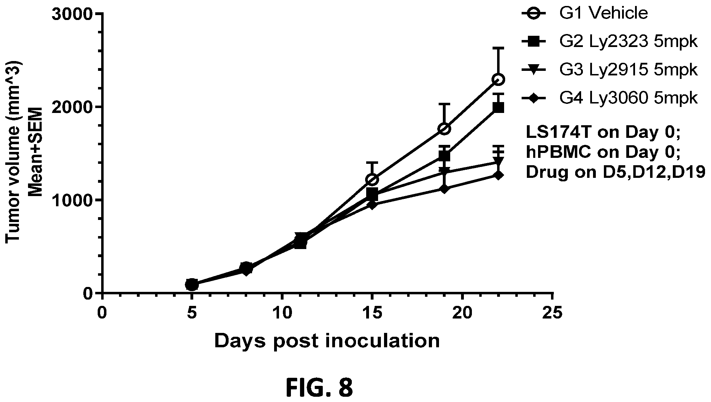

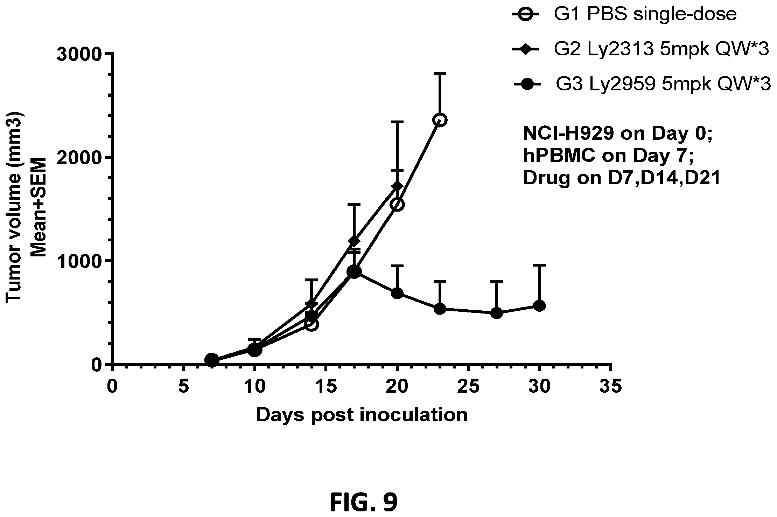

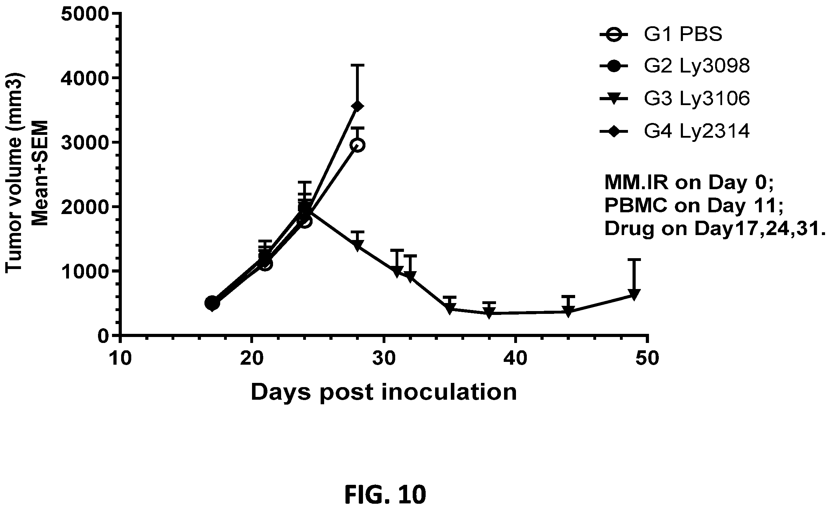

Anti-CEA/CD3/PD-L1 multi-specific antibody Ly3060 showed stronger antitumor activity than its corresponding anti-CEA/CD3 bsAb Ly2915 and the control anti-CEA/CD3 bispecific Ly2323, as shown in **FIG. 8****.** As for anti-BCMA antibodies, Ly2959 and Ly3106, containing the dual CD3/PD-L1 modulating construct and an anti-BCMA binding moiety, exhibited higher antitumor efficacy than their corresponding anti-BCMA/CD3 bsAb Ly2313 and Ly3098 or Ly2314, as shown in **FIGs 9** **and** **10****.**

The promising anti-tumor efficacy data presented in this example strongly indicate that multi-specific antibodies containing the dual CD3/PD-L1 immune modulating construct have the potential for developing effective therapeutic drugs, due to the addition of the PD-L1 binding that potentiate the cytotoxic effects of T cells against cancer cells.

### Example 4: Assessment of Multi-Specific Antibodies Targeting HER2

This example evaluates the functionalities of HER2-targeting multi-specific antibodies comprising the dual immune modulating construct disclosed herein, which bind to CD3 and CD137 or CD3 and PD-L1).

### (i) Binding to Target Antigens

Exemplary multi-specific antibodies were produced in CHO cells as described in Example 1 above. Binding of the multi-specific antibodies to the corresponding target antigens was evaluated by ELISA or FCM following a standard procedure. These antibodies bind to HER2 with high affinity, however they showed much weaker binding to CD3 and CD137 as compared to their corresponding parent anti-CD3 mAb or anti-CD137 mAb as summarized **inTable 3** below and **FIGs. 11A-11F****.**

**Table 3. Binding of Anti-HER2 Multi-Specific Antibodies to Target Antigens**

| **Antibody** | **HER2 binding** | **CD3 binding** | **CD137 binding** | **PD-L1 binding** |
|---|---|---|---|---|
| Ly305 (anti-CD3 parent) | N/A | ++++ | N/A | N/A |
| Ly1630 (anti-CD137 parent) | N/A | N/A | +++++ | N/A |
| Ly076 (anti-PD-L1 parent) | N/A | N/A | N/A | +++++ |
| Ly3151 (HER2/CD3/CD137) | ++++ | +/- | ++ | N/A |
| Ly3188 (HER2/CD3/PD-L1) | ++++ | +/- | N/A | ++ |

### (ii) Reporter Assays

A CD3 reporter assay as described in the Example 3 was performed to determine the potency of these antibodies.

The CD3 reporter assay showed a much-reduced potency (no activity at 10 µg/mL) for these antibodies in the absence of additional targets, compared to the parent anti-CD3 mAb Ly305 **(Table 4).** However, activity of the multi-specific antibodies was greatly enhanced in the presence of additional targets for crosslinking, such as tumor antigen or immune receptor **(Table 4** below and **FIGs. 12A-12F****).**

**Table 4. CD3 binding and activation**

| **Protein** | **CD3 activation alone** | **CD3 activation in the presence of TA target(s)** | **CD3 activation in the presence of CD137 or PD-L1** |
|---|---|---|---|
| Ly305 (anti-CD3 parent) | +++ | +++ | +++ |
| Ly3151 (HER2/CD3/CD137) | +/- | +++++ | ++++ |
| Ly3152 (HER2/CD3) | - | ++++ | - |
| Ly3188 (HER2/CD3/PD-L1) | - | +++++ | ++++ |

A CD137 reporter assay was performed to determine the potency of these antibodies as described in the above examples. The results from the CD137 reporter assay showed minimal activity for the antibodies tested in the absence of additional targets. However, activity was greatly enhanced when additional targets are available for crosslinking, such as tumor antigen or immune receptor (**Table 5** below and **FIGs. 13A-13C**).

**Table 5. CD137 Binding and Activation**

| **Protein** | **CD137 activation alone** | **CD137 activation in the presence of TA target(s)** | **CD3 activation in the presence of CD137 or PD-L1** |
|---|---|---|---|
| Ly1630 (anti-CD137 parent) | ++ | ++ | ++ |
| TM173 (anti-CD137) | +++++ | +++++ | +++++ |
| Ly3151 (HER2/CD3/CD137) | - | +++ | +++ |

### (iii) Tumor Cell Killing

Tumor cell killing by immune cells induced by the anti-HER2 multi-specific antibodies were examined *in vitro*.

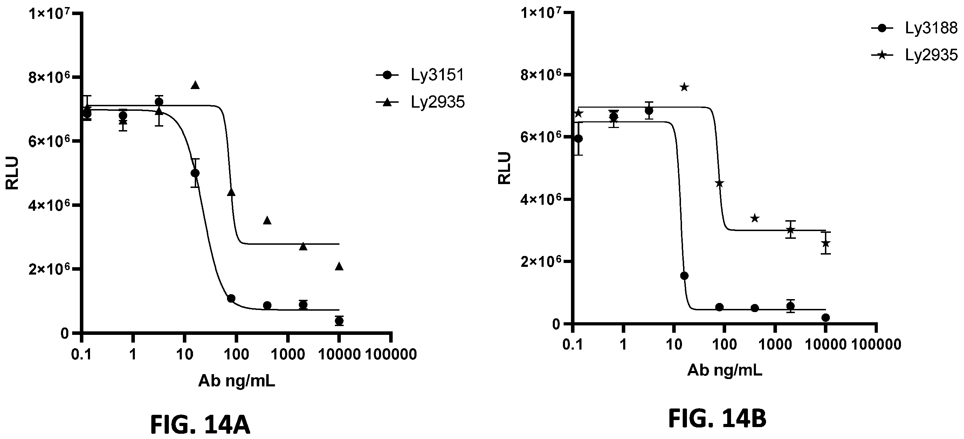

EBC-1-luc cells and human PBMCs were mixed and incubated with various test articles for 48 hours, and luminescence from tumor cells was measured using kit from Promega following the instructions. Cytotoxicity of test articles were reversely correlated to luminescence intensity. The anti-HER2 antibodies integrated with dual CD3/CD137 or CD3/PD-L1 immune modulating fragment showed significant tumor cells killing activity comparable or superior to an anti- HER2/CD3/CD28 tri-specific antibody reference Ly2935 in this assay (**FIGs. 14A-14B**).

### (iv) Antitumor Efficacy

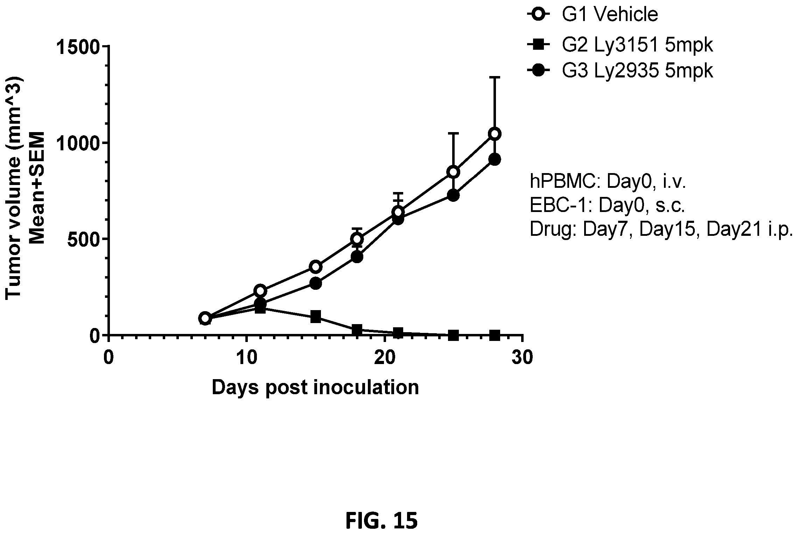

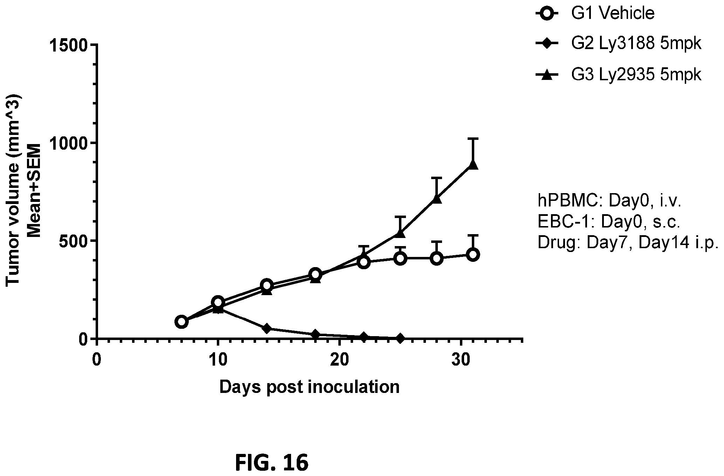

Antitumor efficacy was examined in human PBMC engrafted mouse models implanted with human HER2 expressing lung squamous cell carcinoma line cell EBC-1. Test articles were injected *i.p.*. Mice were weighed and tumor growth was measured twice weekly using calipers. Tumor volume was estimated using the formula 0.5x (length × width²). Stronger antitumor activity was achieved by anti-HER2/CD3/CD137 multi-specific antibodies Ly3151 or anti-HER2/CD3/PD-L1 multi-specific antibodies Ly3188, which comprises the dual modulating construct described above, as compared with a reference HER2/CD3/CD28 tri-specific antibody (Ly2935), as shown in **FIG. 15** and **FIG. 16** respectively.

These examples demonstrated that binding affinity to the immune target antigens (CD3 and CD137/PD-L1) was significantly reduced in the dual immune modulating construct, which leaded to minimal immune modulating activity, as compared with their parental mAbs. In multi-specific format, however, the modulation potential was strongly enhanced in the presence of addition targets in a reporter assay system. The better anti-tumor activity in vitro and in vivo were also achieved by the two exemplary multi-specific antibodies as compared with a reference HER2/CD3/CD28 tri-specific antibody (Ly2935).

### Example 5: Assessment of Multi-Specific Antibodies Targeting CEA

CEA targeted T cell engagers have the potential to mediate immune killing of a variety of solid tumors, where high level of CEA expression is common. Exemplary anti-CEA multi-specific antibody Ly2909 and Ly3060, which comprise the anti-CD3/CD137, and anti-CD3/PD-L1 dual modulating constructs provided herein, respectively, were produced following the approaches described above.

### (i) Binding to Target Antigens

Binding of the exemplary anti-CEA multi-specific antibodies to corresponding target antigens were evaluated by FCM or ELISA and the results are summarized in **Table 6** below and in **FIGs. 17A-17G**. The multi-specific antibodies exhibited binding to CEA with high affinity and significantly weaker binding affinity to immune target antigen CD3, CD137 or PD-L1.

**Table 6. Target Binding of Anti-CEA Multi-Specific Antibodies**

| **Protein** | **CEA binding** | **CD3 binding** | **CD137 binding** | **PD-L1 binding** |
|---|---|---|---|---|
| Ly305 (anti-CD3 parent) | N/A | ++++ | N/A | N/A |
| Ly1630 (anti-CD137 parent) | N/A | N/A | +++++ | N/A |
| Ly076 (anti-PD-L1 parent) | N/A | N/A | N/A | +++++ |
| Ly2909 (CEA/CD3/CD137) | ++++ | +/- | ++ | N/A |

### (ii) Reporter Assays

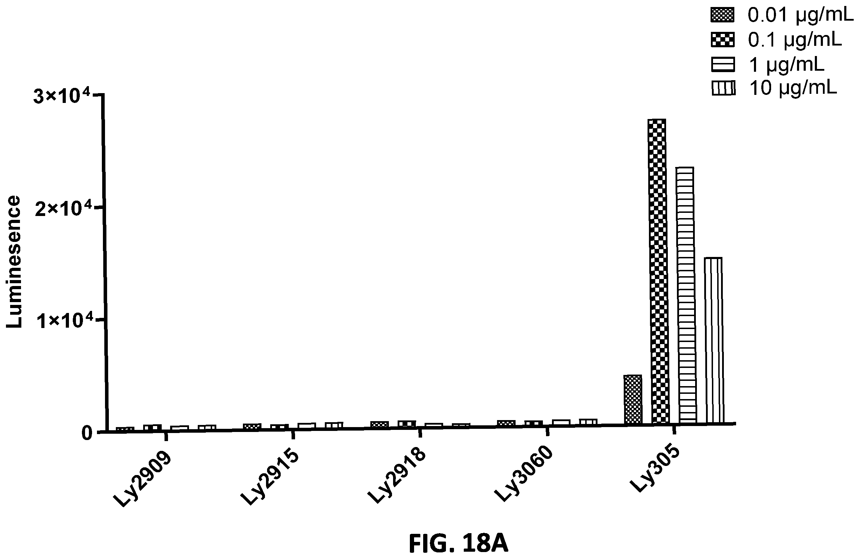

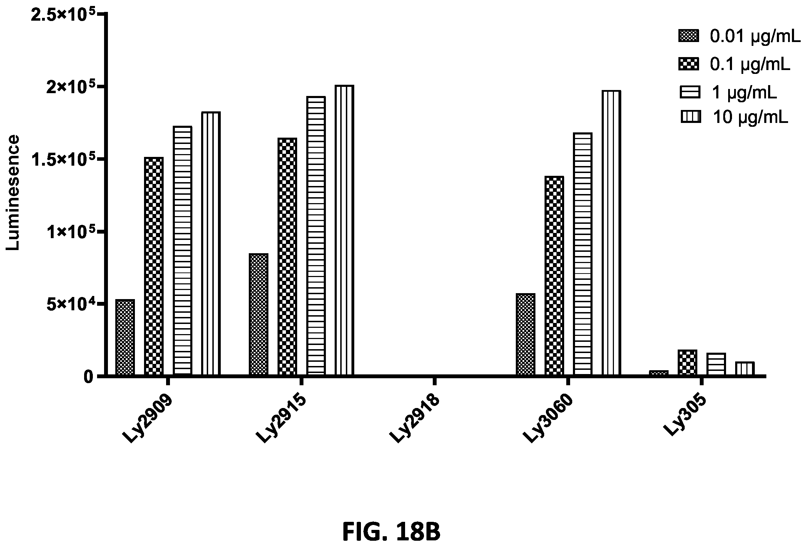

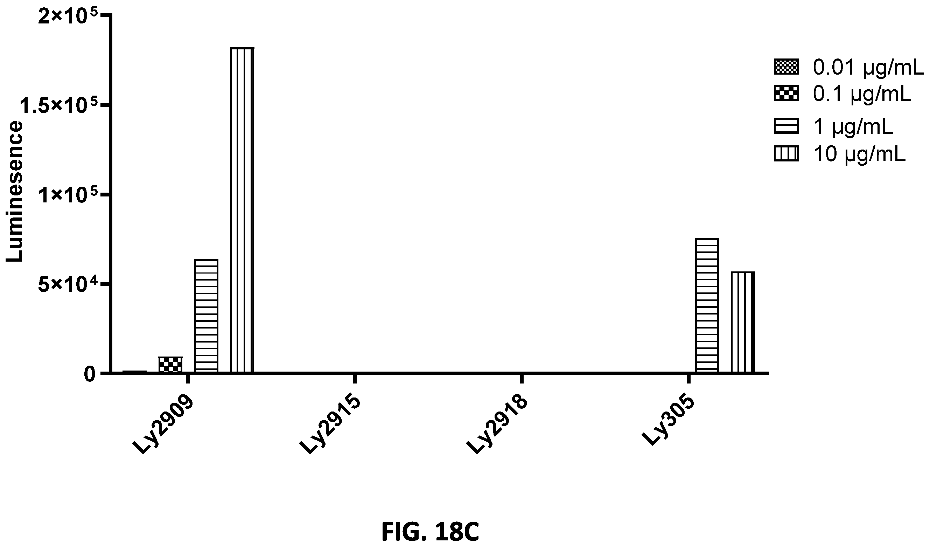

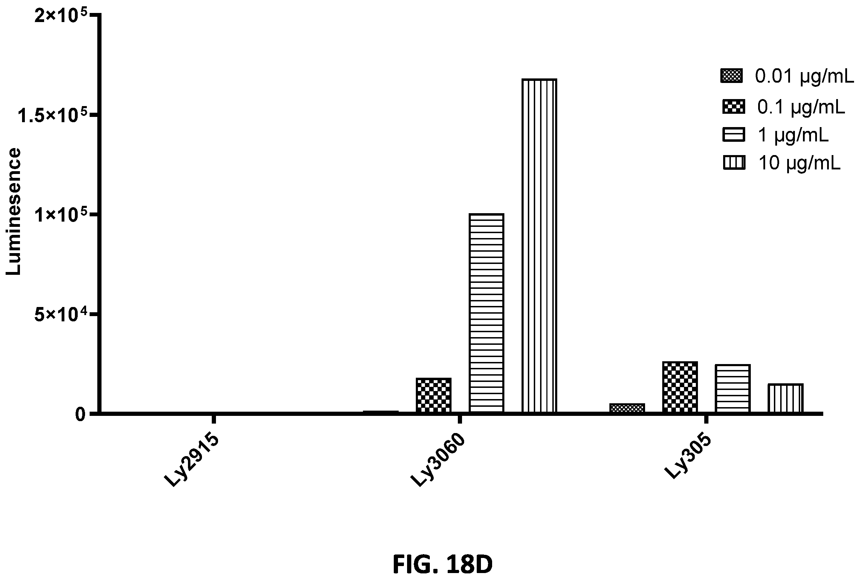

Activation of CD3 signaling by the exemplary anti-CEA multi-specific antibodies was evaluated using a reporter system and the results are summarized in **Table 7** below and in **FIGs.18A-18D****.** The presence of the CEA target antigen drastically increased CD3 activation by the multi-specific antibodies, including Ly2909 (CEA/CD3/CD137), Ly2915 (CEA/CD3) and Ly3060 (CEA/CD3/PD-L1). Binding to another immune target antigen such as CD137 or PD-L1 enhanced CD3 activation by the corresponding multi-specific antibodies.

**Table 7. Activation of CD3 signaling by anti-CEA multi-specific antibodies**

| **Protein** | **CD3 activation without co-culture** | **CD3 activation with CEA** | **CD3 activation with CD137** | **CD3 activation with PD-L1** |
|---|---|---|---|---|
| Ly305 (anti-CD3 parent) | ++ | ++ | ++ | ++ |
| Ly2909 (CEA/CD3/CD137) | - | ++++ | +++ | N/A |
| Ly3060 (CEA/CD3/PD-L1) | - | ++++ | N/A | +++ |

| | | | | |
|---|---|---|---|---|
| Note: N/A=Not Applicable | | | | |

CD137 signaling and blockade of PD-(L)1 signaling by the anti-CEA multi-specific antibodies was evaluated using a reporter system.

Activation of the immune receptor CD3 by the multi-specific antibodies is minimal but was significantly increased under cross-linking conditions, supporting the avidity mediated effect of the multi-specific antibodies.

### (iii) Tumor Cell Killing

Tumor cell killing by immune cells induced by the anti-CEA multi-specific antibodies were examined *in vitro*. LS174T-Luc or HT29-Luc cells and human PBMCs were mixed and incubated with various test antibodies for 48 hours and luminescence intensity was measured as described earlier. A CEA/CD3 bispecific antibody Ly2323 was used as control.

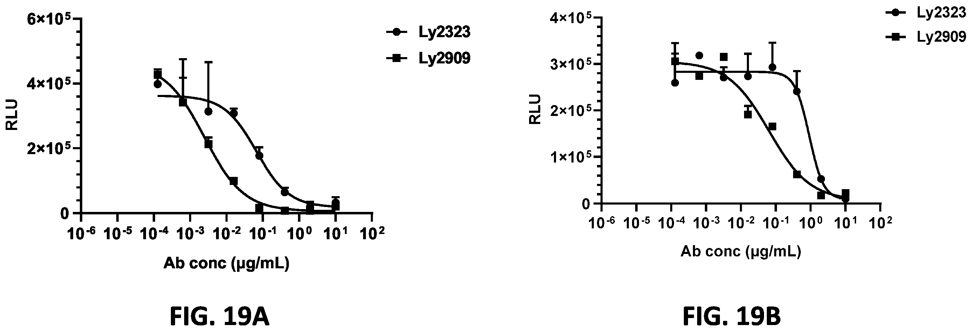

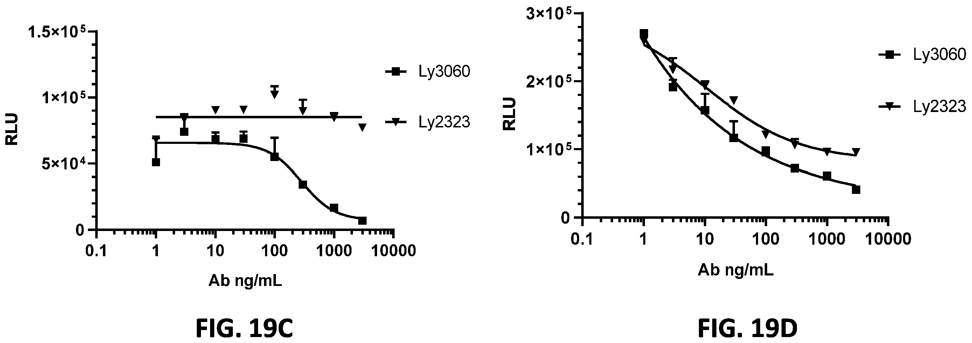

Killing of the LS174T-Luc cells and cytokines release were measured in the *in vitro* assay and the results are summarized in **FIGs. 19A-19B****.** Ly2909 (anti-CEA/CD3/CD137) showed stronger cytotoxicity than Ly2323 (anti-CEA/CD3), suggesting a contribution of the additional CD137 signaling induced by the dual immune modulating construct in the multi-specific antibody.

**FIGs. 19C-19D** provide a summary of the results obtained from the *in vitro* assay with respect to killing of LS174T-Luc or HT29-Luc cancer cells. Ly3060 (anti-CEA/CD3/PD-L1) showed stronger cytotoxicity than Ly2323 (anti-CEA/CD3), suggesting a contribution of the additional PD-L1 binding effect of the multi-specific antibody.

### (iv) Antitumor Efficacy

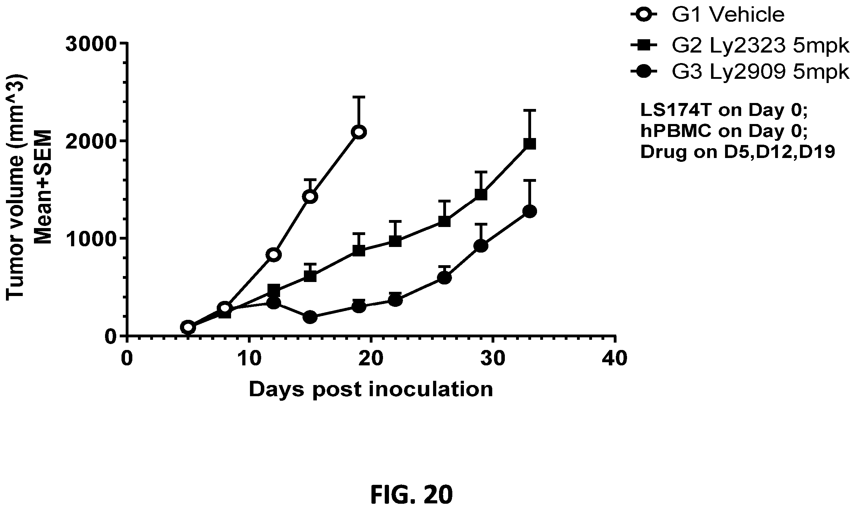

Antitumor efficacy was examined in human PBMC engrafted mouse models. Human CEA expressing LS174T tumors were inoculated *s.c.* and human PBMC were inoculated i.v. on day0. Five days after tumor inoculation, mice were grouped, and test articles were injected *i.p.* weekly. Mice were weighed and tumor growth was measured twice weekly using calipers. Tumor volume was estimated as 1/2 (length x width²). Antitumor activity of exemplary multi-specfic antibody Ly2909 (anti-CEA/CD3/CD137) is shown in **FIGs. 20**. The robust antitumor efficacy achieved by the multi-specific antibody Ly2909 suggests potential benefits arising from the dual CD3/CD137 activation, and potential advantage over the anti-CEA/CD3 bsAb Ly2323.

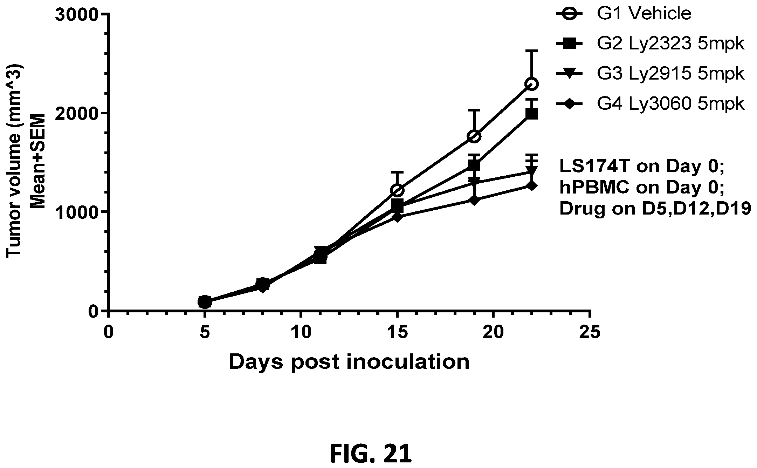

Stronger antitumor activities were also demonstrated by dual CD3/PD-L1 containing anti-CEA multi-specific antibodies Ly3060, comprising the anti-CD3/PD-L1 dual immune modulating construct, as compared with anti-CEA/CD3 bsAb Ly2915 and Ly2323, as shown in **FIG. 21**.

### Example 6: Assessment of Multi-Specific Antibodies Targeting BCMA

Bispecific antibodies targeting BCMA and other immune modulators have been evaluated for their anti-tumor efficacy in clinical trials. Exemplary anti-BCMA multi-specific antibodies comprising the dual anti-CD3/CD137 or anti-CD/PD-L1 immune modulating construct described herein were produced following the disclosures herein, including Ly2949 (anti-BCMA/CD3/CD137), Ly2959 (anti-BCMA/CD3/PD-L1), Ly3096 (anti-BCMA/CD3/CD137) and Ly3106 (anti-BCMA/CD3/PD-L1).

### (i) Binding to Target Antigens

Binding of the exemplary anti-BCMA multi-specific antibodies to corresponding target antigens was evaluated by FCM or ELISA. The results are summarized in **Table 8** below and in **FIGs. 22A-22R****.** The multi-specific antibodies exhibited high binding affinity to TAA target BCMA and significantly lower binding affinity to immune target antigens CD3 and CD137 or PD-L1.

**Table 8. Target binding of Anti-BCMA Multi-Specific Antibodies**

| **Antibody** | **BCMA binding** | **CD3 binding** | **CD137 binding** | **PD-L1 binding** |
|---|---|---|---|---|
| Ly305 (anti-CD3 parent) | N/A | ++++ | N/A | N/A |
| Ly1630 (anti-CD137 parent) | N/A | N/A | +++++ | N/A |
| Ly076 (anti-PD-L1 parent) | N/A | N/A | N/A | +++++ |
| Ly2949 (BCMA/CD3/CD137) | ++++ | + | + | N/A |
| Ly2959 (BCMA/CD3/PD-L1) | ++++ | + | N/A | + |
| Ly3096 (BCMA/CD3/CD137) | ++++ | + | + | N/A |
| Ly3106 (BCMA/CD3/PD-L1) | ++++ | + | N/A | + |

| | | | | |
|---|---|---|---|---|
| Note: N/A=Not Applicable | | | | |

### (ii) Reporter Assays

Activation of the CD3 signaling by the anti-BCMA multi-specific antibodies was evaluated using a reporter system and the results are summarized in **Table 9** below and in **FIGs.23A-23H****.** The presence of the BCMA target antigen drastically increased CD3 activation by all of the anti-BCMA multi-specific antibodies. Binding to a second immune target such as CD137 or PD-L1 enhanced CD3 activation by the corresponding multi-specific antibodies.

**Table 9. Activation of CD3 Signaling by anti-BCMA Multi-Specific Antibodies**

| **Protein** | **CD3 activation without co-culture** | **CD3 activation with BCMA** | **CD3 activation with CD137** | **CD3 activation with PD-L1** |
|---|---|---|---|---|
| Ly305 (anti-CD3 parent) | ++ | ++ | ++ | ++ |
| Ly2949 (BCMA/CD3/CD137) | - | ++++ | +++ | N/A |
| Ly2959 (BCMA/CD3/PD-L1) | - | ++++ | N/A | +++ |
| Ly3096 (BCMA/CD3/CD137) | - | ++++ | +++ | N/A |
| Ly3106 (BCMA/CD3/PD-L1) | - | ++++ | N/A | +++ |

Activation of CD137 signaling by the anti-BCMA multi-specific antibodies was evaluated using a reporter system and the results are summarized in **Table 10** below and in **FIGs. 24A-24F**.

**Table 10. Activation of CD137 Signaling by Anti-BCMA Multi-Specific Antibodies**

| **Protein** | **CD137 activation without co-culture** | **CD137 activation with BCMA** | **CD137 activation with CD3** |
|---|---|---|---|
| TM173 (anti-CD137) | +++ | +++ | +++ |
| Ly2949 (BCMA/CD3/CD137) | - | ++ | ++ |
| Ly3096 (BCMA-CD3/CD137) | - | ++ | ++ |

| | | | |
|---|---|---|---|
| Note: N/A=Not Applicable | | | |

Activation of the immune receptor CD3 or CD137 by the multi-specific antibodies is minimal but significantly increased under cross-linking conditions, supporting the avidity mediated effect of the design.

### (iv) Tumor Cell Killing and Antitumor Efficacy

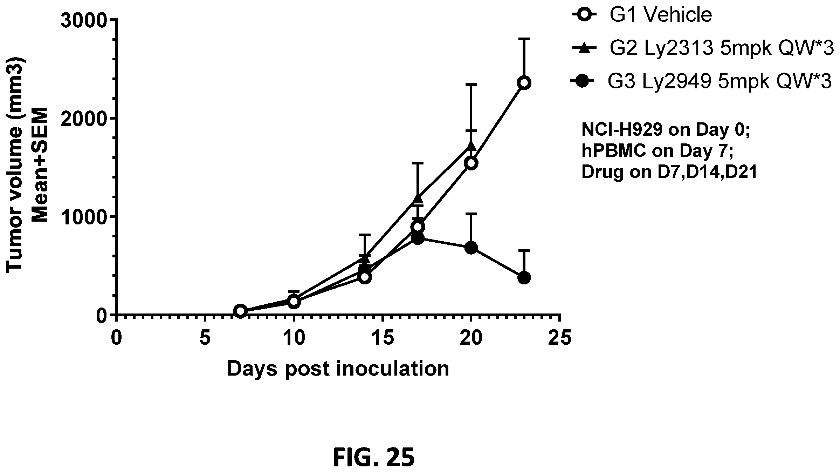

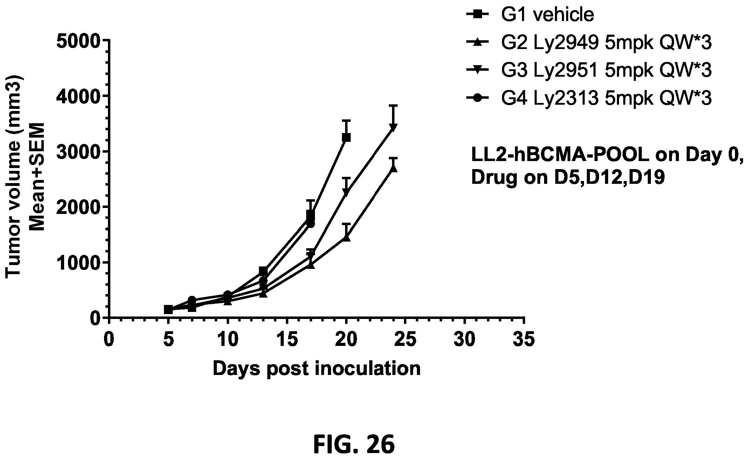

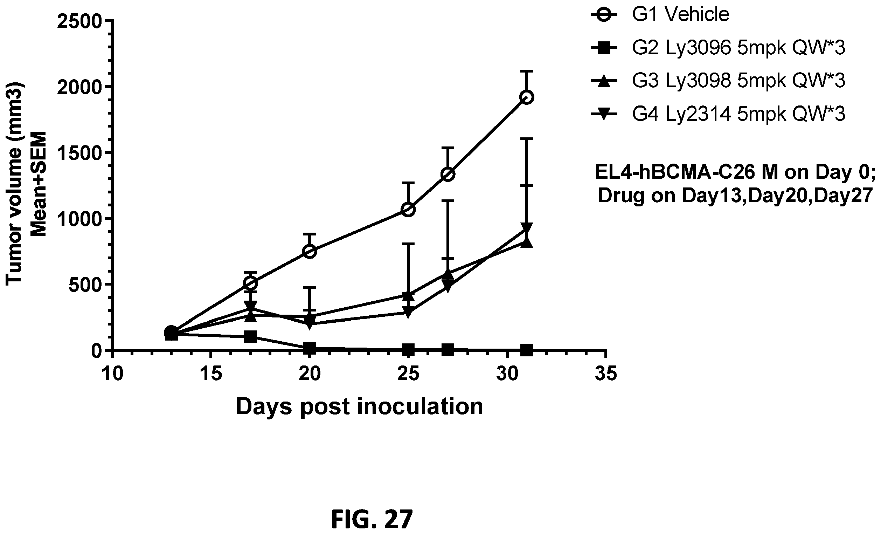

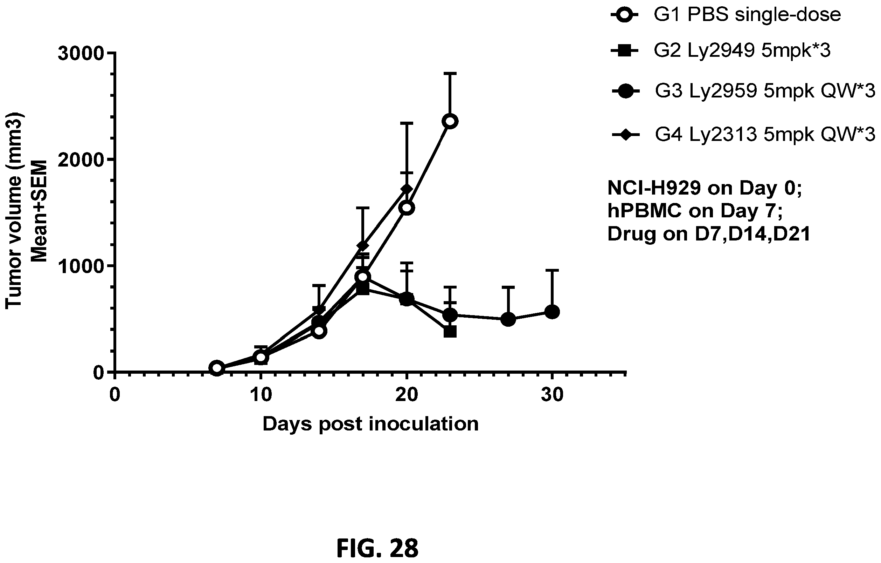

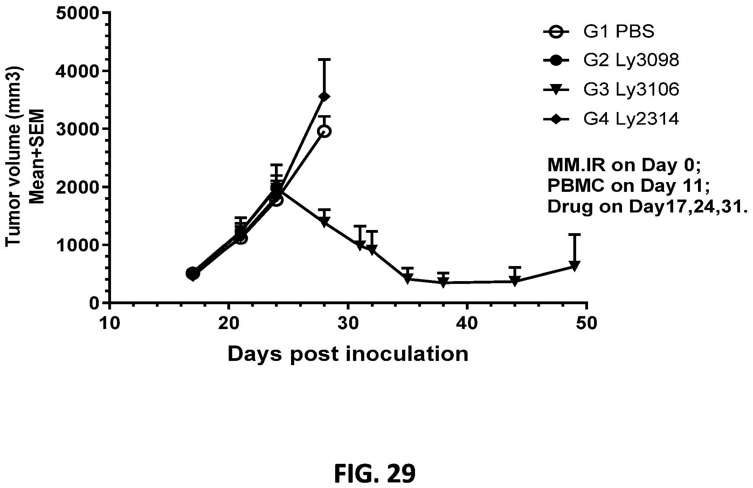

Tumor cell killing by immune cells induced by the exemplary anti-BCMA multi-specific antibodies was examined *in vitro*. Antitumor efficacy was examined in PBMC engrafted mouse model with BCMA expressing NCI-H929 and MM1.R cells or a bone marrow transplanted syngeneic mouse model with human BCMA overexpressing LL2 and EL4 cells. Stronger antitumor activities were achieved by clones Ly2949, Ly3096, Ly2959 and Ly3106, comprising the dual anti-CD3/CD137 or anti-CD3/PD-L1 dual immune modulating construct, when compared with anti-BCMA/CD3 bsAb Ly2951, Ly2314, Ly2313 or Ly3098. See **FIGs. 25-29****.**

### Example 7: Effects of Fc Variants in Antibodies

This example explores effects arising from Fc variants (*e.g*., 237 Deletion, L234A, L235A, P329G substitutions, or combinations thereof) contained in antibodies as provided herein.

A CD3 reporter assay was performed to detect if there is any cross-linking effect of the multi-specific antibodies tested herein *via* the binding of the Fc fragment contained in the multi-specific antibodies to Fc receptors. The CD3 reporter assay used in this Example comprises Jurkat/NFAT-Luc2P cells (Jurkat cells expressing a luciferase reporter driven by an NFAT response element). Briefly, the Jurkat/NFAT-Luc2P cells were harvested and aliquoted at 50000 cells/well in a 96-well plate and co-cultured with or without Fc receptor expressing cells. Test antibodies were added, and the plate were incubated for additional 6 hours at 37°C, followed by Bright-Glo^{™} Luciferase Assay (Promega Cat #E2620). NFAT-mediated luminescence in this assay corresponds to the activation of CD3 by the antibody in the absence or presence of Fc receptors.

### (i) Fc variant effects in anti-CD3 monoclonal antibodies

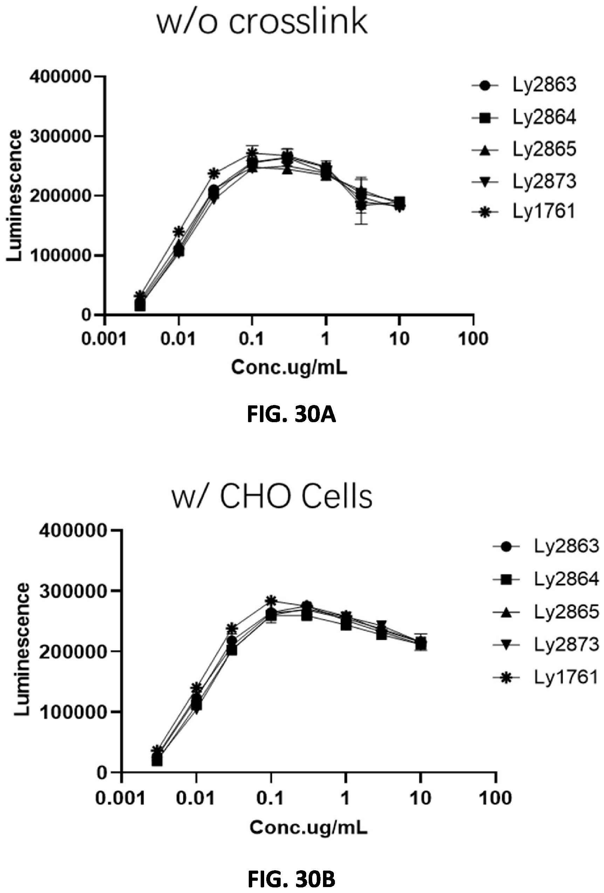

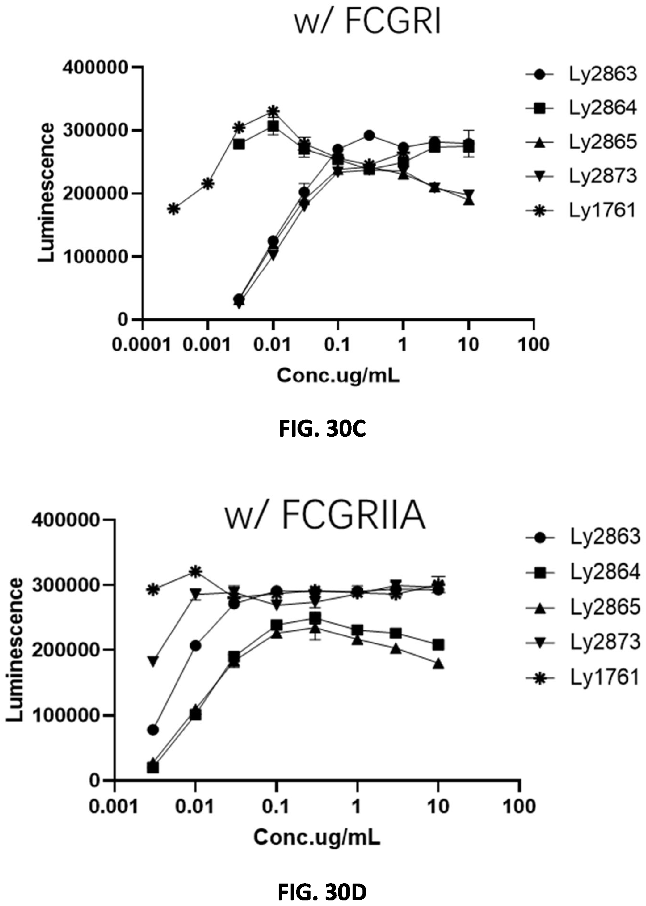

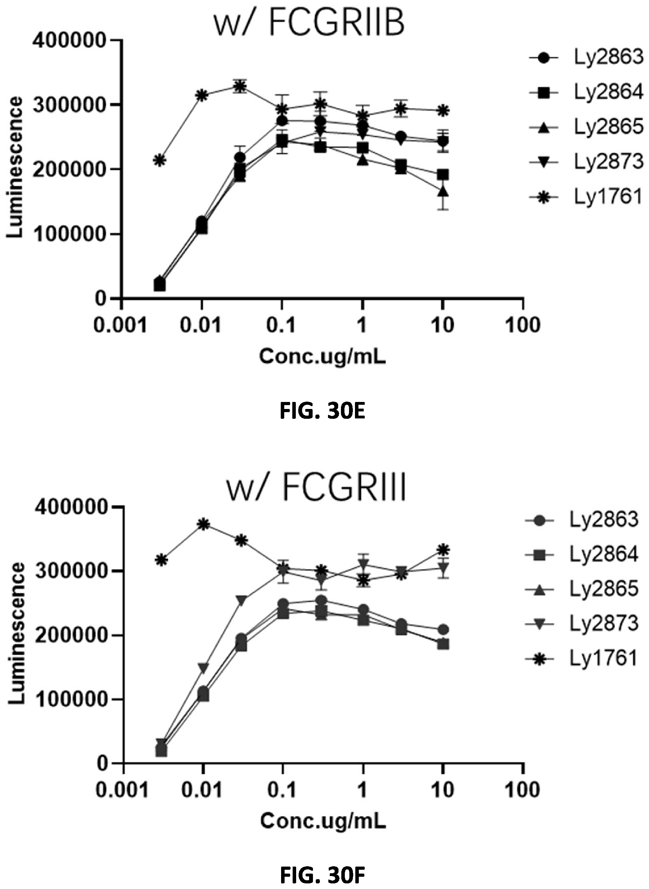

Exemplary anti-CD3 monoclonal antibodies stemming from Ly1761 (IgG1 wt) with one or more mutations in their Fc fragments, including Ly2863 (237 deletion), Ly2864 (P329G), Ly2865 (237 deletion + P329G), Ly2873 (L234A+ L235A + 237 deletion) and Ly1761 (IgG1 wt), were tested in the CD3 reporter assay. As shown in **FIGs. 30A-30F****,** the Fc variant having the combination of 237 deletion and P329G substitution doesn't have cross-linking effect due to abolished Fc-FcR binding, while Fc variants carrying the individual mutation (the 237 deletion alone and the P329G substitution alone) or the combination of L234A+ L235A + 237 deletion maintained certain levels of the cross-linking effects when co-cultured with either FCGRI or FCGRIIA expressing CHO cells for example.

### (ii) Fc variants effect in multi-specific antibodies

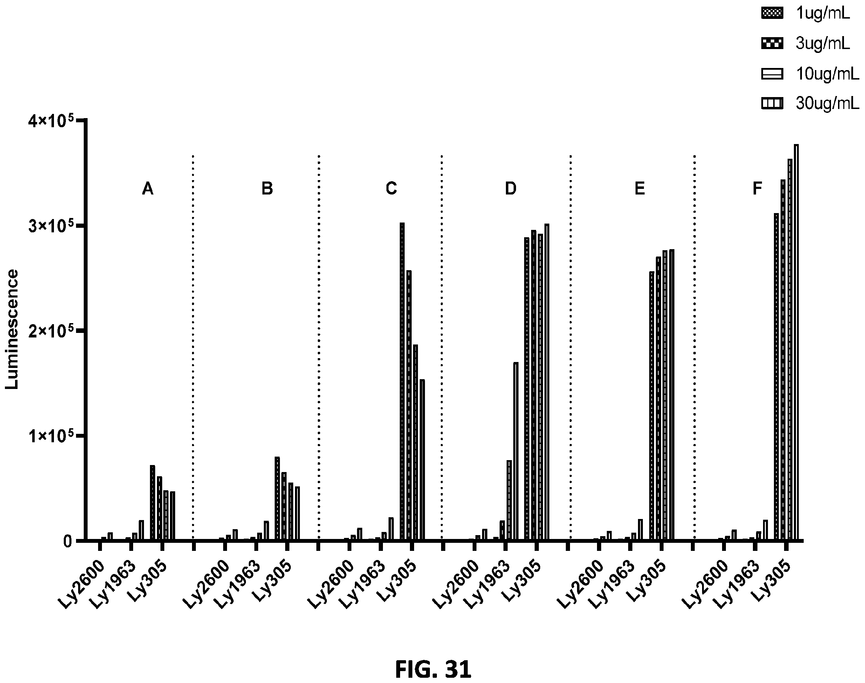

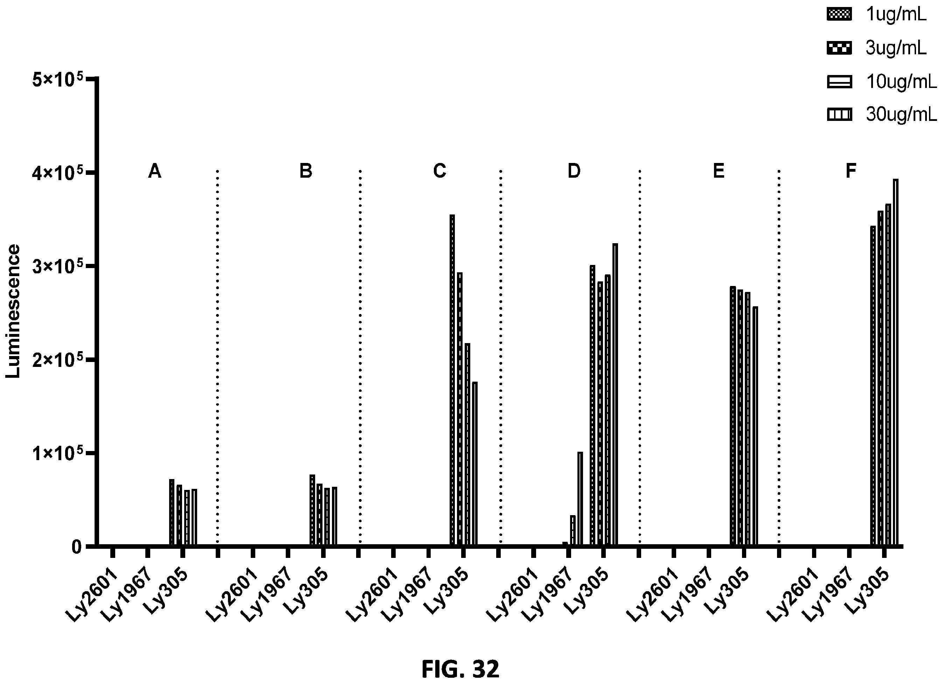

Exemplary multi-specific antibodies, Ly2600 (anti-B7H3/CD3/CD137) and Ly2601(anti-CD19/CD3/CD137), both comprising the 237 deletion and P329G mutations, were tested in the CD3 reporter assay. The sequence information of these two antibody clones is provided in **Table 2** below. Clones Ly1963 and Ly1967, both comprising the same antigen-binding moieties as Ly2600 and Ly2601, respectively, and substitutions of L234A, L235A and 237 deletion, were used as controls. As shown in **FIGs. 31** and **32****,** the combined mutations of 237 deletion and P329G in the Fc variant fragment abolished cross-linking effect arising from the Fc-FcR binding; such complete abolishment of cross-linking effect was not observed in Fc variants containing the combination of L234A+ L235A + 237 deletion when co-cultured with FCGRIIA as shown in FIG. 31D and FIG. 32D.

### SEQUENCE TABLES

**Table 1: Components in Bi/Multi-specific Antibodies**

| **Name** | **Chains** | **Sequence** | **SEQ ID NO** |
|---|---|---|---|
| Linker Motif 237Δ | | DKTHTCPPCPAPELLGP | 1 |
| Linker 237Δ LALA | | DKTHTCPPCPAPEAAGP | 29 |
| CH2 (P329G) | | SVFLFPPKPKDTLMISRTPEVTCVVVDVSHEDPEVKFNWYVDG VEVHNAKTKPREEQYNSTYRVVSVLTVLHQDWLNGKEYKCK VSNKALGAPIEKTISKAK | 2 |
| CH2 (WT) | | SVFLFPPKPKDTLMISRTPEVTCVVVDVSHEDPEVKFNWYVDG VEVHNAKTKPREEQYNSTYRVVSVLTVLHQDWLNGKEYKCK VSNKALPAPIEKTISKAK | 90 |
| Fc fragment (hinge-CH2; 237Δ/P329G) | | DKTHTCPPCPAPELLGPSVFLFPPKPKDTLMISRTPEVTCVVVD VSHEDPEVKFNWYVDGVEVHNAKTKPREEQYNSTYRVVSVLT VLHQDWLNGKEYKCKVSNKALGAPIEKTISKAK | 91 |
| CH3, wild-type | | GQPREPQVYTLPPSREEMTKNQVSLTCLVKGFYPSDIAVEWES NGQPENNYKTTPPVLDSDGSFFLYSKLTVDKSRWQQGNVFSCS VMHEALHNHYTQKSLSLSPG | 92 |
| CH3, Knob mutation | | GQPREPQVYTLPPSREEMTKNQVSLWCLVKGFYPSDIAVEWES NGQPENNYKTTPPVLDSDGSFFLYSKLTVDKSRWQQGNVFSCS VMHEALHNHYTQKSLSLSPG | 93 |
| CH3, Hole mutation Protein A binding mutation | | GQPREPQVYTLPPSREEMTKNQVSLSCAVKGFYPSDIAVEWES NGQPENNYKTTPPVLDSDGSFFLVSKLTVDKSRWQQGNVFSCS VMHEALHNRFTQKSLSLSPG | 94 |
| CH3, Hole mutation | | GQPREPQVYTLPPSREEMTKNQVSLSCAVKGFYPSDIAVEWES NGQPENNYKTTPPVLDSDGSFFLVSKLTVDKSRWQQGNVFSCS VMHEALHNHYTQKSLSLSPG | 95 |
| G₄S linker | | GGGGS | 96 |
| (G₄S)₂ linker | | GGGGSGGGGS | 97 |
| (G₄S)₃ linker | | GGGGSGGGGSGGGGS | 98 |
| (G₄S)₄ linker | | GGGGSGGGGSGGGGSGGGGS | 99 |
| (G₄S)₅ linker | | GGGGSGGGGSGGGGSGGGGSGGGGS | 100 |
| Linker | | GGGGSDKTHTCPPCPAPELLGP | 101 |
| Dual binding motif to CD3 and CD137 | 1st | EVQLVESGGGLVQPGGSLRLSCAASGFTFSTYAMNWVRQAPG | 3 |
| | | KGLEWVGRIRSKYNNYATYYADSVKGRFTISRDDSKNSLYLQ MNSLKTEDTAVYYCVRHGNFGNSYVSWFAYWGQGTLVTVSS GGGGSDKTHTCPPCPAPELLGPSVFLFPPKPKDTLMISRTPEVT CVVVDVSHEDPEVKFNWYVDGVEVHNAKTKPREEQYNSTYR VVSVLTVLHQDWLNGKEYKCKVSNKALGAPIEKTISKAKGQP REPQVYTLPPSREEMTKNQVSLWCLVKGFYPSDIAVEWESNG QPENNYKTTPPVLDSDGSFFLYSKLTVDKSRWQQGNVFSCSV MHEALHNHYTQKSLSLSPGKGGGGSGGGGSGGGGSGGGGSQ VQLVQSGAEVKKPGASVKVSCKASGYTFAGFEMHWVRQAPG QGLEWMGAIDPKTGGTDYNQKFKDRVTMTRDTSISTAYMELS RLRSDDTAVYYCARDLGYFDVWGQGTLVTVSS | |
| | 2nd | QAVVTQEPSLTVSPGGTVTLTCRSSTGAVTTSNYANWVQQKP GQAPRGLIGGTNKRAPWTPARFSGSLLGGKAALTITGAQAEDE ADYYCALWYSNLWVFGGGTKLTVLGGGGSDKTHTCPPCPAPE LLGPSVFLFPPKPKDTLMISRTPEVTCVVVDVSHEDPEVKFNW YVDGVEVHNAKTKPREEQYNSTYRVVSVLTVLHQDWLNGKE YKCKVSNKALGAPIEKTISKAKGQPREPQVYTLPPSREEMTKN QVSLSCAVKGFYPSDIAVEWESNGQPENNYKTTPPVLDSDGSF FLVSKLTVDKSRWQQGNVFSCSVMHEALHNRFTQKSLSLSPG KGGGGSGGGGSGGGGSGGGGSDIQMTQSPSSLSASVGDRVTIT CRASQDIRSNLNWYQQKPGGAVKLLIYYTSRLHSGVPSRFSGS GSGTDYTLTISSLQPEDFATYFCQQSEKLPRTFGGGTKVEIR | 4 |
| Dual binding motif to CD3 and PD-L1 | 1st | EVQLVESGGGLVQPGGSLRLSCAASGFTFSTYAMNWVRQAPG KGLEWVGRIRSKYNNYATYYADSVKGRFTISRDDSKNSLYLQ MNSLKTEDTAVYYCVRHGNFGNSYVSWFAYWGQGTLVTVSS GGGGSDKTHTCPPCPAPELLGPSVFLFPPKPKDTLMISRTPEVT CVVVDVSHEDPEVKFNWYVDGVEVHNAKTKPREEQYNSTYR VVSVLTVLHQDWLNGKEYKCKVSNKALGAPIEKTISKAKGQP REPQVYTLPPSREEMTKNQVSLWCLVKGFYPSDIAVEWESNG QPENNYKTTPPVLDSDGSFFLYSKLTVDKSRWQQGNVFSCSV MHEALHNHYTQKSLSLSPGKGGGGSGGGGSGGGGSGGGGSQ VQLVQSGAEVKKPGASVKVSCKASGYTFTDFWMSWVRQAPG QGLEWMGQIYPNTGTTHSNEKFKGRVTMTRDTSTSTVYMELS SLRSEDTAVYYCARSYHISTTPNWFAYWGQGTLVTVSS | 5 |
| | 2nd | QAVVTQEPSLTVSPGGTVTLTCRSSTGAVTTSNYANWVQQKP GQAPRGLIGGTNKRAPWTPARFSGSLLGGKAALTITGAQAEDE ADYYCALWYSNLWVFGGGTKLTVLGGGGSDKTHTCPPCPAPE LLGPSVFLFPPKPKDTLMISRTPEVTCVVVDVSHEDPEVKFNW YVDGVEVHNAKTKPREEQYNSTYRVVSVLTVLHQDWLNGKE YKCKVSNKALGAPIEKTISKAKGQPREPQVYTLPPSREEMTKN QVSLSCAVKGFYPSDIAVEWESNGQPENNYKTTPPVLDSDGSF FLVSKLTVDKSRWQQGNVFSCSVMHEALHNRFTQKSLSLSPG KGGGGSGGGGSGGGGSGGGGSDIQMTQSPSSLSASVGDRVTIT CKASQNVYKKLEWYQQKPGKVPKVLIYHTNILQTGVPSRFSGS GSGTDYTLTISSLQPEDVATYYCYQWNSGPTFGGGTKVEIK | 6 |
| Ly305 Anti-CD3 | VH | EVQLVESGGGLVQPGGSLRLSCAASGFTFSTYAMNWVRQAP GKGLEWVGRIRSKYNNYATYYADSVKGRFTISRDDSKNSLYL QMNSLKTEDTAVYYCVRHGNFGNSYVSWFAYWGQGTLVTV SS | 7 |
| | VL | QAVVTQEPSLTVSPGGTVTLTCRSSTGAVTTSNYANWVQQKP GQAPRGLIGGTNKRAPWTPARFSGSLLGGKAALTITGAQAED EADYYCALWYSNLWVFGGGTKLTVL | 8 |
| Ly1761 Anti-CD3 | VH | | 30 |
| | VL | | 33 |
| Ly1630 Anti-CD137 | VH | QVQLVQSGAEVKKPGASVKVSCKASGYTFAGFEMHWVRQA PGQGLEWMGAIDPKTGGTDYNQKFKDRVTMTRDTSISTAYM ELSRLRSDDTAVYYCARDLGYFDVWGQGTLVTVSS | 9 |
| | VL | DIQMTQSPSSLSASVGDRVTITCRASQDIRSNLNWYQQKPGGA VKLLIYYTSRLHSGVPSRFSGSGSGTDYTLTISSLQPEDFATYFC QQSEKLPRTFGGGTKVEIR | 10 |
| TM173 Anti-CD137 | VH | | 34 |
| | VL | | 37 |
| Ly076 Anti-PD-L1 | VH | QVQLVQSGAEVKKPGASVKVSCKASGYTFTDFWMSWVRQA PGQGLEWMGQIYPNTGTTHSNEKFKGRVTMTRDTSTSTVYM ELSSLRSEDTAVYYCARSYHISTTPNWFAYWGQGTLVTVSS | 11 |
| | VL | DIQMTQSPSSLSASVGDRVTITCKASQNVYKKLEWYQQKPGK VPKVLIYHTNILQTGVPSRFSGSGSGTDYTLTISSLQPEDVATY YCYQWNSGPTFGGGTKVEIK | 12 |
| Ly2530 Anti-PD-L1 | VH | | 48 |
| | VL | | 51 |
| Ly591 Anti-HER2 (a) | VH | | 13 |
| | VL | | 14 |
| TM737 Anti-HER2 (b) | VH | | 15 |
| | VL | | 16 |
| Ly312 Anti-CEA | VH | EVQLVESGGGLVQPGGSLRLSCAASGFNIKDTYMHWVRQAP GKGLEWVARIDPANGNSKYADSVKGRFTISADTSKNTAYLQ MNSLRAEDTAVYYCAPFGYYVSDYAMAYWGQGTLVTVSS | 17 |
| | VL | DIQLTQSPSSLSASVGDRVTITCRAGESVDIFGVGFLHWYQQK PGKAPKLLIYRASNLESGVPSRFSGSGSRTDFTLTISSLQPEDFA TYYCQQTNEDPYTFGQGTKVEIK | 18 |
| Ly560 Anti-BCMA (a) | VH | QVQLVQSGAEVKKPGASVKVSCKASGYTFTNHIIHWVRQAP GQGLEWMGYINPYPGYHAYNEKFQGRATMTSDTSTSTVYME LSSLRSEDTAVYYCARDGYYRDTDVLDYWGQGTLVTVSS | 19 |
| | VL | DIQMTQSPSSLSASVGDRVTITCQASQDISNYLNWYQQKPGKA | 20 |
| | | PKLLIYYTSRLHTGVPSRFSGSGSGTDFTFTISSLEPEDIATYYC QQGNTLPWTFGQGTKVEIK | |
| Ly3019 Anti-BCMA (b) | VH | QLQLQESGPGLVKPSETLSLTCTVSGGSISSGSYFWGWIRQPPG KGLEWIGSIYYSGITYYNPSLKSRVTISVDTSKNQFSLKLSSVT AADTAVYYCARHDGAVAGLFDYWGQGTLVTVSS | 21 |
| | VL | SYVLTQPPSVSVAPGQTARITCGGNNIGSKSVHWYQQPPGQAP VVVVYDDSDRPSGIPERFSGSNSGNTATLTISRVEAGDEAVYY CQVWDSSSDHVVFGGGTKLTVL | 22 |

**Table 2: Sequences for Exemplary Bi-Multi-Specific Antibodies**

| **Name** | **Chains** | **Sequence** | **SEQ ID NO** |
|---|---|---|---|
| Ly3151 | HC1 | EVQLVESGGGLVQPGGSLRLSCAASGFTFTDYTMDWVRQAPGKGL EWVADVNPNSGGSIYNQRFKGRFTLSVDRSKNTLYLQMNSLRAED TAVYYCARNLGPSFYFDYWGQGTLVTVSSASVAAPSVFIFPPSDEQL KSGTASVVCLLNNFYPREAKVQWKVDNALQSGNSQESVTEQDSKD STYSLSSTLTLSKADYEKHKVYACEVTHQGLSSPVTKSFNRGECGG GGSGGGGSGGGGSGGGGSEVQLVESGGGLVQPGGSLRLSCAASGF TFSTYAMNWVRQAPGKGLEWVGRIRSKYNNYATYYADSVKGRFTI SRDDSKNSLYLQMNSLKTEDTAVYYCVRHGNFGNSYVSWFAYWG QGTLVTVSSGGGGSDKTHTCPPCPAPELLGPSVFLFPPKPKDTLMISR TPEVTCVVVDVSHEDPEVKFNWYVDGVEVHNAKTKPREEQYNSTY RVVSVLTVLHQDWLNGKEYKCKVSNKALGAPIEKTISKAKGQPREP QVYTLPPSREEMTKNQVSLWCLVKGFYPSDIAVEWESNGQPENNY KTTPPVLDSDGSFFLYSKLTVDKSRWQQGNVFSCSVMHEALHNHY TQKSLSLSPGKGGGGSGGGGSGGGGSGGGGSQVQLVQSGAEVKKP GASVKVSCKASGYTFAGFEMHWVRQAPGQGLEWMGAIDPKTGGT DYNQKFKDRVTMTRDTSISTAYMELSRLRSDDTAVYYCARDLGYF DVWGQGTLVTVSS | 23 |
| HER2 /CD3 /CD137 | | | |
| | HC2 | EVQLVESGGGLVQPGGSLRLSCAASGFNIKDTYIHWVRQAPGKGLE WVARIYPTNGYTRYADSVKGRFTISADTSKNTAYLQMNSLRAEDT AVYYCSRWGGDGFYAMDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGGS GGGGSGGGGSGGGGSQAVVTQEPSLTVSPGGTVTLTCRSSTGAVTT SNYANWVQQKPGQAPRGLIGGTNKRAPWTPARFSGSLLGGKAALT ITGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT YFCQQSEKLPRTFGGGTKVEIR | 24 |
| | LC1 | DIQMTQSPSSLSASVGDRVTITCKASQDVSIGVAWYQQKPGKAPKL LIYSASYRYTGVPSRFSGSGSGTDFTLTISSLQPEDFATYYCQQYYIY PYTFGQGTKVEIKSSASTKGPSVFPLAPSSKSTSGGTAALGCLVKDY FPEPVTVSWNSGALTSGVHTFPAVLQSSGLYSLSSVVTVPSSSLGTQ TYICNVNHKPSNTKVDKKVEPKSC | 25 |
| | LC2 | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGQGTKVEIKRTVAAPSVFIFPPSDEQLKSGTASVVCLLNNFYPRE AKVQWKVDNALQSGNSQESVTEQDSKDSTYSLSSTLTLSKADYEK HKVYACEVTHQGLSSPVTKSFNRGEC | 26 |
| Ly3188 | HC1 | EVQLVESGGGLVQPGGSLRLSCAASGFTFTDYTMDWVRQAPGKGL EWVADVNPNSGGSIYNQRFKGRFTLSVDRSKNTLYLQMNSLRAED TAVYYCARNLGPSFYFDYWGQGTLVTVSSASVAAPSVFIFPPSDEQL KSGTASVVCLLNNFYPREAKVQWKVDNALQSGNSQESVTEQDSKD STYSLSSTLTLSKADYEKHKVYACEVTHQGLSSPVTKSFNRGECGG GGSGGGGSGGGGSGGGGSEVQLVESGGGLVQPGGSLRLSCAASGF TFSTYAMNWVRQAPGKGLEWVGRIRSKYNNYATYYADSVKGRFTI SRDDSKNSLYLQMNSLKTEDTAVYYCVRHGNFGNSYVSWFAYWG QGTLVTVSSGGGGSDKTHTCPPCPAPELLGPSVFLFPPKPKDTLMISR TPEVTCVVVDVSHEDPEVKFNWYVDGVEVHNAKTKPREEQYNSTY RVVSVLTVLHQDWLNGKEYKCKVSNKALGAPIEKTISKAKGQPREP QVYTLPPSREEMTKNQVSLWCLVKGFYPSDIAVEWESNGQPENNY KTTPPVLDSDGSFFLYSKLTVDKSRWQQGNVFSCSVMHEALHNHY TQKSLSLSPGKGGGGSGGGGSGGGGSGGGGSQVQLVQSGAEVKKP GASVKVSCKASGYTFTDFWMSWVRQAPGQGLEWMGQIYPNTGTT HSNEKFKGRVTMTRDTSTSTVYMELSSLRSEDTAVYYCARSYHIST TPNWFAYWGQGTLVTVSS | 27 |
| HER2 /CD3 /PD-L1 | | | |
| | HC2 | EVQLVESGGGLVQPGGSLRLSCAASGFNIKDTYIHWVRQAPGKGLE WVARIYPTNGYTRYADSVKGRFTISADTSKNTAYLQMNSLRAEDT AVYYCSRWGGDGFYAMDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGGS GGGGSGGGGSGGGGSQAVVTQEPSLTVSPGGTVTLTCRSSTGAVTT SNYANWVQQKPGQAPRGLIGGTNKRAPWTPARFSGSLLGGKAALT ITGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCKASQNVYKKLEWYQ QKPGKVPKVLIYHTNILQTGVPSRFSGSGSGTDYTLTISSLQPEDVAT YYCYQWNSGPTFGGGTKVEIK | 28 |
| | LC1 | DIQMTQSPSSLSASVGDRVTITCKASQDVSIGVAWYQQKPGKAPKL LIYSASYRYTGVPSRFSGSGSGTDFTLTISSLQPEDFATYYCQQYYIY PYTFGQGTKVEIKSSASTKGPSVFPLAPSSKSTSGGTAALGCLVKDY FPEPVTVSWNSGALTSGVHTFPAVLQSSGLYSLSSVVTVPSSSLGTQ TYICNVNHKPSNTKVDKKVEPKSC | 25 |
| | LC2 | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGQGTKVEIKRTVAAPSVFIFPPSDEQLKSGTASVVCLLNNFYPRE AKVQWKVDNALQSGNSQESVTEQDSKDSTYSLSSTLTLSKADYEK HKVYACEVTHQGLSSPVTKSFNRGEC | 26 |
| Ly3152 | HC1 | EVQLVESGGGLVQPGGSLRLSCAASGFTFTDYTMDWVRQAPGKGL EWVADVNPNSGGSIYNQRFKGRFTLSVDRSKNTLYLQMNSLRAED TAVYYCARNLGPSFYFDYWGQGTLVTVSSASVAAPSVFIFPPSDEQL KSGTASVVCLLNNFYPREAKVQWKVDNALQSGNSQESVTEQDSKD | 31 |
| HER2 /CD3 | | | |
| | | STYSLSSTLTLSKADYEKHKVYACEVTHQGLSSPVTKSFNRGECGG GGSGGGGSGGGGSGGGGSEVQLVESGGGLVQPGGSLRLSCAASGF TFSTYAMNWVRQAPGKGLEWVGRIRSKYNNYATYYADSVKGRFTI SRDDSKNSLYLQMNSLKTEDTAVYYCVRHGNFGNSYVSWFAYWG QGTLVTVSSGGGGSDKTHTCPPCPAPELLGPSVFLFPPKPKDTLMISR TPEVTCVVVDVSHEDPEVKFNWYVDGVEVHNAKTKPREEQYNSTY RVVSVLTVLHQDWLNGKEYKCKVSNKALGAPIEKTISKAKGQPREP QVYTLPPSREEMTKNQVSLWCLVKGFYPSDIAVEWESNGQPENNY KTTPPVLDSDGSFFLYSKLTVDKSRWQQGNVFSCSVMHEALHNHY TQKSLSLSPGK | |
| | HC2 | EVQLVESGGGLVQPGGSLRLSCAASGFNIKDTYIHWVRQAPGKGLE WVARIYPTNGYTRYADSVKGRFTISADTSKNTAYLQMNSLRAEDT AVYYCSRWGGDGFYAMDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGGS GGGGSGGGGSGGGGSQAVVTQEPSLTVSPGGTVTLTCRSSTGAVTT SNYANWVQQKPGQAPRGLIGGTNKRAPWTPARFSGSLLGGKAALT ITGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGK | 32 |
| | LC1 | DIQMTQSPSSLSASVGDRVTITCKASQDVSIGVAWYQQKPGKAPKL LIYSASYRYTGVPSRFSGSGSGTDFTLTISSLQPEDFATYYCQQYYIY PYTFGQGTKVEIKSSASTKGPSVFPLAPSSKSTSGGTAALGCLVKDY FPEPVTVSWNSGALTSGVHTFPAVLQSSGLYSLSSVVTVPSSSLGTQ TYICNVNHKPSNTKVDKKVEPKSC | 25 |
| | LC2 | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGQGTKVEIKRTVAAPSVFIFPPSDEQLKSGTASVVCLLNNFYPRE AKVQWKVDNALQSGNSQESVTEQDSKDSTYSLSSTLTLSKADYEK HKVYACEVTHQGLSSPVTKSFNRGEC | 26 |
| Ly2935 | HC1 | EVQLVESGGGLVQPGGSLRLSCAASGFNIKDTYIHWVRQAPGKGLE WVARIYPTNGYTRYADSVKGRFTISADTSKNTAYLQMNSLRAEDT AVYYCSRWGGDGFYAMDYWGQGTLVTVSSASTKGPSVFPLAPCSR STSESTAALGCLVKDYFPEPVTVSWNSGALTSGVHTFPAVLQSSGL YSLSSVVTVPSSSLGTKTYTCNVDHKPSNTKVDKRVESKYGPPCPPC PAPEFLGGPSVFLFPPKPKDTLMISRTPEVTCVVVDVSQEDPEVQFN WYVDGVEVHNAKTKPREEQFNSTYRVVSVLTVLHQDWLNGKEYK CKVSNKGLPSSIEKTISKAKGQPREPQVYTLPPCQEEMTKNQVSLWC LVKGFYPSDIAVEWESNGQPENNYKTTPPVLDSDGSFFLYSKLTVD KSRWQEGNVFSCSVMHEALHNHYTQKSLSLSLGK | 35 |
| HER2 /CD3 /CD28 | | | |
| | HC2 | QVQLVQSGAEVVKPGASVKVSCKASGYTFTSYYIHWVRQAPGQGL EWIGSIYPGNVNTNYAQKFQGRATLTVDTSISTAYMELSRLRSDDT AVYYCTRSHYGLDWNFDVWGKGTTVTVSSSQVQLVESGGGVVQP GRSLRLSCAASGFTFTKAWMHWVRQAPGKQLEWVAQIKDKSNSY ATYYADSVKGRFTISRDDSKNTLYLQMNSLRAEDTAVYYCRGVYY ALSPFDYWGQGTLVTVSSRTASTKGPSVFPLAPCSRSTSESTAALGC LVKDYFPEPVTVSWNSGALTSGVHTFPAVLQSSGLYSLSSVVTVPSS SLGTKTYTCNVDHKPSNTKVDKRVESKYGPPCPPCPAPEFLGGPSVF LFPPKPKDTLMISRTPEVTCVVVDVSQEDPEVQFNWYVDGVEVHN | 36 |
| | | AKTKPREEQFNSTYRVVSVLTVLHQDWLNGKEYKCKVSNKGLPSSI EKTISKAKGQPREPQVCTLPPSQEEMTKNQVSLSCAVKGFYPSDIAV EWESNGQPENNYKTTPPVLDSDGSFFLVSKLTVDKSRWQEGNVFSC SVMHEALHNHYTQKSLSLSLGK | |
| | LC1 | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGQGTKVEIKRTVAAPSVFIFPPSDEQLKSGTASVVCLLNNFYPRE AKVQWKVDNALQSGNSQESVTEQDSKDSTYSLSSTLTLSKADYEK HKVYACEVTHQGLSSPVTKSFNRGEC | 26 |
| | LC2 | DIVMTQTPLSLSVTPGQPASISCKSSQSLVHNNANTYLSWYLQKPGQ SPQSLIYKVSNRFSGVPDRFSGSGSGTDFTLKISRVEAEDVGVYYCG QGTQYPFTFGSGTKVEIKGQPKAAPDIQMTQSPSSLSASVGDRVTIT CQASQNIYVWLNWYQQKPGKAPKLLIYKASNLHTGVPSRFSGSGSG TDFTLTISSLQPEDIATYYCQQGQTYPYTFGQGTKLEIKTKGPSRTVA APSVFIFPPSDEQLKSGTASVVCLLNNFYPREAKVQWKVDNALQSG NSQESVTEQDSKDSTYSLSSTLTLSKADYEKHKVYACEVTHQGLSS PVTKSFNRGEC | 38 |
| Ly2909 | HC1 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSEVQLVESGGGLVQPGGSLRLSCAASGFTF STYAMNWVRQAPGKGLEWVGRIRSKYNNYATYYADSVKGRFTISR DDSKNSLYLQMNSLKTEDTAVYYCVRHGNFGNSYVSWFAYWGQG TLVTVSSGGGGSDKTHTCPPCPAPELLGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREPQ VYTLPPSREEMTKNQVSLWCLVKGFYPSDIAVEWESNGQPENNYK TTPPVLDSDGSFFLYSKLTVDKSRWQQGNVFSCSVMHEALHNHYT QKSLSLSPGKGGGGSGGGGSGGGGSGGGGSQVQLVQSGAEVKKPG ASVKVSCKASGYTFAGFEMHWVRQAPGQGLEWMGAIDPKTGGTD YNQKFKDRVTMTRDTSISTAYMELSRLRSDDTAVYYCARDLGYFD VWGQGTLVTVSS | 39 |
| CEA /CD3 /CD137 | | | |
| | HC2 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSQAVVTQEPSLTVSPGGTVTLTCRSSTGAV TTSNYANWVQQKPGQAPRGLIGGTNKRAPWTPARFSGSLLGGKAA LTITGAQAEDEADYYCALWYSNLWVFGGGTKLTVLGGGGSDKTHT CPPCPAPELLGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSREEMTKNQVSL SCAVKGFYPSDIAVEWESNGQPENNYKTTPPVLDSDGSFFLVSKLTV DKSRWQQGNVFSCSVMHEALHNRFTQKSLSLSPGKGGGGSGGGGS GGGGSGGGGSDIQMTQSPSSLSASVGDRVTITCRASQDIRSNLNWY QQKPGGAVKLLIYYTSRLHSGVPSRFSGSGSGTDYTLTISSLQPEDFA TYFCQQSEKLPRTFGGGTKVEIR | 40 |
| | LC1 | | 41 |
| Ly2323 | HC1 | QVQLVQSGAEVKKPGASVKVSCKASGYTFTEFGMNWVRQAPGQG LEWMGWINTKTGEATYVEEFKGRVTFTTDTSTSTAYMELRSLRSDD TAVYYCARWDFAYYVEAMDYWGQGTTVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCDGG GGSGGGGSEVQLLESGGGLVQPGGSLRLSCAASGFTFSTYAMNWV RQAPGKGLEWVSRIRSKYNNYATYYADSVKGRFTISRDDSKNTLYL QMNSLRAEDTAVYYCVRHGNFGNSYVSWFAYWGQGTLVTVSSAS VAAPSVFIFPPSDEQLKSGTASVVCLLNNFYPREAKVQWKVDNALQ SGNSQESVTEQDSKDSTYSLSSTLTLSKADYEKHKVYACEVTHQGL SSPVTKSFNRGECDKTHTCPPCPAPEAAGGPSVFLFPPKPKDTLMISR TPEVTCVVVDVSHEDPEVKFNWYVDGVEVHNAKTKPREEQYNSTY RVVSVLTVLHQDWLNGKEYKCKVSNKALGAPIEKTISKAKGQPREP QVYTLPPCRDELTKNQVSLWCLVKGFYPSDIAVEWESNGQPENNY KTTPPVLDSDGSFFLYSKLTVDKSRWQQGNVFSCSVMHEALHNHY TQKSLSLSPGK | 42 |
| CEA /CD3 | | | |
| | HC2 | QVQLVQSGAEVKKPGASVKVSCKASGYTFTEFGMNWVRQAPGQG LEWMGWINTKTGEATYVEEFKGRVTFTTDTSTSTAYMELRSLRSDD TAVYYCARWDFAYYVEAMDYWGQGTTVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPEAAGGPSVFLFPPKPKDTLMISRTPEVTCVVVDVSHED PEVKFNWYVDGVEVHNAKTKPREEQYNSTYRVVSVLTVLHQDWL NGKEYKCKVSNKALGAPIEKTISKAKGQPREPQVCTLPPSRDELTKN QVSLSCAVKGFYPSDIAVEWESNGQPENNYKTTPPVLDSDGSFFLVS KLTVDKSRWQQGNVFSCSVMHEALHNHYTQKSLSLSPGK | 43 |
| | LC1 | QAVVTQEPSLTVSPGGTVTLTCGSSTGAVTTSNYANWVQEKPGQAF RGLIGGTNKRAPGTPARFSGSLLGGKAALTLSGAQPEDEAEYYCAL WYSNLWVFGGGTKLTVLSSASTKGPSVFPLAPSSKSTSGGTAALGC LVKDYFPEPVTVSWNSGALTSGVHTFPAVLQSSGLYSLSSVVTVPSS SLGTQTYICNVNHKPSNTKVDKKVEPKSC | 44 |
| | LC2 | DIQMTQSPSSLSASVGDRVTITCKASAAVGTYVAWYQQKPGKAPKL LIYSASYRKRGVPSRFSGSGSGTDFTLTISSLQPEDFATYYCHQYYTY PLFTFGQGTKLEIKRTVAAPSVFIFPPSDEQLKSGTASVVCLLNNFYP REAKVQWKVDNALQSGNSQESVTEQDSKDSTYSLSSTLTLSKADYE KHKVYACEVTHQGLSSPVTKSFNRGEC | 45 |
| Ly3060 | HC1 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSEVQLVESGGGLVQPGGSLRLSCAASGFTF STYAMNWVRQAPGKGLEWVGRIRSKYNNYATYYADSVKGRFTISR DDSKNSLYLQMNSLKTEDTAVYYCVRHGNFGNSYVSWFAYWGQG TLVTVSSGGGGSDKTHTCPPCPAPELLGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREPQ | 46 |
| CEA /CD3 /PD-L1 | | | |
| | | VYTLPPSREEMTKNQVSLWCLVKGFYPSDIAVEWESNGQPENNYK TTPPVLDSDGSFFLYSKLTVDKSRWQQGNVFSCSVMHEALHNHYT QKSLSLSPGKGGGGSGGGGSGGGGSGGGGSQVQLVQSGAEVKKPG ASVKVSCKASGYTFTDFWMSWVRQAPGQGLEWMGQIYPNTGTTH SNEKFKGRVTMTRDTSTSTVYMELSSLRSEDTAVYYCARSYHISTTP NWFAYWGQGTLVTVSS | |
| | HC2 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSQAVVTQEPSLTVSPGGTVTLTCRSSTGAV TTSNYANWVQQKPGQAPRGLIGGTNKRAPWTPARFSGSLLGGKAA LTITGAQAEDEADYYCALWYSNLWVFGGGTKLTVLGGGGSDKTHT CPPCPAPELLGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSREEMTKNQVSL SCAVKGFYPSDIAVEWESNGQPENNYKTTPPVLDSDGSFFLVSKLTV DKSRWQQGNVFSCSVMHEALHNRFTQKSLSLSPGKGGGGSGGGGS GGGGSGGGGSDIQMTQSPSSLSASVGDRVTITCKASQNVYKKLEWY QQKPGKVPKVLIYHTNILQTGVPSRFSGSGSGTDYTLTISSLQPEDVA TYYCYQWNSGPTFGGGTKVEIK | 47 |
| | LC1 | DIQLTQSPSSLSASVGDRVTITCRAGESVDIFGVGFLHWYQQKPGKA PKLLIYRASNLESGVPSRFSGSGSRTDFTLTISSLQPEDFATYYCQQT NEDPYTFGQGTKVEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC | 41 |
| Ly2915 | HC1 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSEVQLVESGGGLVQPGGSLRLSCAASGFTF STYAMNWVRQAPGKGLEWVGRIRSKYNNYATYYADSVKGRFTISR DDSKNSLYLQMNSLKTEDTAVYYCVRHGNFGNSYVSWFAYWGQG TLVTVSSGGGGSDKTHTCPPCPAPELLGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREPQ VYTLPPSREEMTKNQVSLWCLVKGFYPSDIAVEWESNGQPENNYK TTPPVLDSDGSFFLYSKLTVDKSRWQQGNVFSCSVMHEALHNHYT QKSLSLSPGK | 49 |
| CEA /CD3 | | | |
| | HC2 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSQAVVTQEPSLTVSPGGTVTLTCRSSTGAV TTSNYANWVQQKPGQAPRGLIGGTNKRAPWTPARFSGSLLGGKAA LTITGAQAEDEADYYCALWYSNLWVFGGGTKLTVLGGGGSDKTHT CPPCPAPELLGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSREEMTKNQVSL | 50 |
| | | SCAVKGFYPSDIAVEWESNGQPENNYKTTPPVLDSDGSFFLVSKLTV DKSRWQQGNVFSCSVMHEALHNRFTQKSLSLSPGK | |
| | LC1 | DIQLTQSPSSLSASVGDRVTITCRAGESVDIFGVGFLHWYQQKPGKA PKLLIYRASNLESGVPSRFSGSGSRTDFTLTISSLQPEDFATYYCQQT NEDPYTFGQGTKVEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC | 41 |
| Ly2918 | HC1 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPELLGPSVFLFPPKPKDTLMISRTPEVTCVVVDVSHEDPE VKFNWYVDGVEVHNAKTKPREEQYNSTYRVVSVLTVLHQDWLNG KEYKCKVSNKALGAPIEKTISKAKGQPREPQVYTLPPSREEMTKNQ VSLWCLVKGFYPSDIAVEWESNGQPENNYKTTPPVLDSDGSFFLYS KLTVDKSRWQQGNVFSCSVMHEALHNHYTQKSLSLSPGKGGGGSG GGGSGGGGSGGGGSQVQLVQSGAEVKKPGASVKVSCKASGYTFA GFEMHWVRQAPGQGLEWMGAIDPKTGGTDYNQKFKDRVTMTRD TSISTAYMELSRLRSDDTAVYYCARDLGYFDVWGQGTLVTVSS | 52 |
| CEA /CD137 | | | |
| | HC2 | EVQLVESGGGLVQPGGSLRLSCAASGFNIKDTYMHWVRQAPGKGL EWVARIDPANGNSKYADSVKGRFTISADTSKNTAYLQMNSLRAED TAVYYCAPFGYYVSDYAMAYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPELLGPSVFLFPPKPKDTLMISRTPEVTCVVVDVSHEDPE VKFNWYVDGVEVHNAKTKPREEQYNSTYRVVSVLTVLHQDWLNG KEYKCKVSNKALGAPIEKTISKAKGQPREPQVYTLPPSREEMTKNQ VSLSCAVKGFYPSDIAVEWESNGQPENNYKTTPPVLDSDGSFFLVSK LTVDKSRWQQGNVFSCSVMHEALHNRFTQKSLSLSPGKGGGGSGG GGSGGGGSGGGGSDIQMTQSPSSLSASVGDRVTITCRASQDIRSNLN WYQQKPGGAVKLLIYYTSRLHSGVPSRFSGSGSGTDYTLTISSLQPE DFATYFCQQSEKLPRTFGGGTKVEIR | 53 |
| | LC1 | DIQLTQSPSSLSASVGDRVTITCRAGESVDIFGVGFLHWYQQKPGKA PKLLIYRASNLESGVPSRFSGSGSRTDFTLTISSLQPEDFATYYCQQT NEDPYTFGQGTKVEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC | 44 |
| Ly2949 | HC1 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSEVQLVESGGGLVQPGGSLRLSCAASGFTF STYAMNWVRQAPGKGLEWVGRIRSKYNNYATYYADSVKGRFTISR DDSKNSLYLQMNSLKTEDTAVYYCVRHGNFGNSYVSWFAYWGQG TLVTVSSGGGGSDKTHTCPPCPAPELLGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREPQ | 55 |
| BCMA(a) /CD3 /CD137 | | | |
| | | VYTLPPSREEMTKNQVSLWCLVKGFYPSDIAVEWESNGQPENNYK TTPPVLDSDGSFFLYSKLTVDKSRWQQGNVFSCSVMHEALHNHYT QKSLSLSPGKGGGGSGGGGSGGGGSGGGGSQVQLVQSGAEVKKPG ASVKVSCKASGYTFAGFEMHWVRQAPGQGLEWMGAIDPKTGGTD YNQKFKDRVTMTRDTSISTAYMELSRLRSDDTAVYYCARDLGYFD VWGQGTLVTVSS | |
| | HC2 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSQAVVTQEPSLTVSPGGTVTLTCRSSTGAV TTSNYANWVQQKPGQAPRGLIGGTNKRAPWTPARFSGSLLGGKAA LTITGAQAEDEADYYCALWYSNLWVFGGGTKLTVLGGGGSDKTHT CPPCPAPELLGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSREEMTKNQVSL SCAVKGFYPSDIAVEWESNGQPENNYKTTPPVLDSDGSFFLVSKLTV DKSRWQQGNVFSCSVMHEALHNRFTQKSLSLSPGKGGGGSGGGGS GGGGSGGGGSDIQMTQSPSSLSASVGDRVTITCRASQDIRSNLNWY QQKPGGAVKLLIYYTSRLHSGVPSRFSGSGSGTDYTLTISSLQPEDFA TYFCQQSEKLPRTFGGGTKVEIR | 56 |
| | LC1 | DIQMTQSPSSLSASVGDRVTITCQASQDISNYLNWYQQKPGKAPKL LIYYTSRLHTGVPSRFSGSGSGTDFTFTISSLEPEDIATYYCQQGNTLP WTFGQGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC | 57 |
| Ly3096 | HC1 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCGGGGSGGGG SGGGGSGGGGSEVQLVESGGGLVQPGGSLRLSCAASGFTFSTYAMN WVRQAPGKGLEWVGRIRSKYNNYATYYADSVKGRFTISRDDSKNS LYLQMNSLKTEDTAVYYCVRHGNFGNSYVSWFAYWGQGTLVTVS SGGGGSDKTHTCPPCPAPELLGPSVFLFPPKPKDTLMISRTPEVTCVV VDVSHEDPEVKFNWYVDGVEVHNAKTKPREEQYNSTYRVVSVLT VLHQDWLNGKEYKCKVSNKALGAPIEKTISKAKGQPREPQVYTLPP SREEMTKNQVSLWCLVKGFYPSDIAVEWESNGQPENNYKTTPPVL DSDGSFFLYSKLTVDKSRWQQGNVFSCSVMHEALHNHYTQKSLSL SPGKGGGGSGGGGSGGGGSGGGGSQVQLVQSGAEVKKPGASVKV SCKASGYTFAGFEMHWVRQAPGQGLEWMGAIDPKTGGTDYNQKF KDRVTMTRDTSISTAYMELSRLRSDDTAVYYCARDLGYFDVWGQG TLVTVSS | 58 |
| BCMA(b) /CD3 /CD137 | | | |
| | HC2 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCGGGGSGGGG SGGGGSGGGGSQAVVTQEPSLTVSPGGTVTLTCRSSTGAVTTSNYA NWVQQKPGQAPRGLIGGTNKRAPWTPARFSGSLLGGKAALTITGA QAEDEADYYCALWYSNLWVFGGGTKLTVLGGGGSDKTHTCPPCP | 59 |
| | | APELLGPSVFLFPPKPKDTLMISRTPEVTCVVVDVSHEDPEVKFNWY VDGVEVHNAKTKPREEQYNSTYRVVSVLTVLHQDWLNGKEYKCK VSNKALGAPIEKTISKAKGQPREPQVYTLPPSREEMTKNQVSLSCAV KGFYPSDIAVEWESNGQPENNYKTTPPVLDSDGSFFLVSKLTVDKSR WQQGNVFSCSVMHEALHNRFTQKSLSLSPGKGGGGSGGGGSGGGG SGGGGSDIQMTQSPSSLSASVGDRVTITCRASQDIRSNLNWYQQKPG GAVKLLIYYTSRLHSGVPSRFSGSGSGTDYTLTISSLQPEDFATYFCQ QSEKLPRTFGGGTKVEIR | |
| | LC1 | SYVLTQPPSVSVAPGQTARITCGGNNIGSKSVHWYQQPPGQAPVVV VYDDSDRPSGIPERFSGSNSGNTATLTISRVEAGDEAVYYCQVWDSS SDHVVFGGGTKLTVLGQPKAAPSVTLFPPSSEELQANKATLVCLISD FYPGAVTVAWKGDSSPVKAGVETTTPSKQSNNKYAASSYLSLTPEQ WKSHRSYSCQVTHEGSTVEKTVAPTECS | 60 |
| Ly3098 | HC1 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCGGGGSGGGG SGGGGSGGGGSEVQLVESGGGLVQPGGSLRLSCAASGFTFSTYAMN WVRQAPGKGLEWVGRIRSKYNNYATYYADSVKGRFTISRDDSKNS LYLQMNSLKTEDTAVYYCVRHGNFGNSYVSWFAYWGQGTLVTVS SGGGGSDKTHTCPPCPAPELLGPSVFLFPPKPKDTLMISRTPEVTCVV VDVSHEDPEVKFNWYVDGVEVHNAKTKPREEQYNSTYRVVSVLT VLHQDWLNGKEYKCKVSNKALGAPIEKTISKAKGQPREPQVYTLPP SREEMTKNQVSLWCLVKGFYPSDIAVEWESNGQPENNYKTTPPVL DSDGSFFLYSKLTVDKSRWQQGNVFSCSVMHEALHNHYTQKSLSL SPGK | 61 |
| BCMA(b) /CD3 | | | |
| | HC2 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCGGGGSGGGG SGGGGSGGGGSQAVVTQEPSLTVSPGGTVTLTCRSSTGAVTTSNYA NWVQQKPGQAPRGLIGGTNKRAPWTPARFSGSLLGGKAALTITGA QAEDEADYYCALWYSNLWVFGGGTKLTVLGGGGSDKTHTCPPCP APELLGPSVFLFPPKPKDTLMISRTPEVTCVVVDVSHEDPEVKFNWY VDGVEVHNAKTKPREEQYNSTYRVVSVLTVLHQDWLNGKEYKCK VSNKALGAPIEKTISKAKGQPREPQVYTLPPSREEMTKNQVSLSCAV KGFYPSDIAVEWESNGQPENNYKTTPPVLDSDGSFFLVSKLTVDKSR WQQGNVFSCSVMHEALHNRFTQKSLSLSPGK | 62 |
| | LC1 | SYVLTQPPSVSVAPGQTARITCGGNNIGSKSVHWYQQPPGQAPVVV VYDDSDRPSGIPERFSGSNSGNTATLTISRVEAGDEAVYYCQVWDSS SDHVVFGGGTKLTVLGQPKAAPSVTLFPPSSEELQANKATLVCLISD FYPGAVTVAWKGDSSPVKAGVETTTPSKQSNNKYAASSYLSLTPEQ WKSHRSYSCQVTHEGSTVEKTVAPTECS | 60 |
| Ly3099 | HC1 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCDKTHTCPPCP APELLGGPSVFLFPPKPKDTLMISRTPEVTCVVVDVSHEDPEVKFNWY VDGVEVHNAKTKPREEQYNSTYRVVSVLTVLHQDWLNGKEYKCK | 64 |
| BCMA(b) /CD137 | | | |
| | | VSNKALGAPIEKTISKAKGQPREPQVYTLPPSREEMTKNQVSLWCL VKGFYPSDIAVEWESNGQPENNYKTTPPVLDSDGSFFLYSKLTVDK SRWQQGNVFSCSVMHEALHNHYTQKSLSLSPGKGGGGSGGGGSGG GGSGGGGSQVQLVQSGAEVKKPGASVKVSCKASGYTFAGFEMHW VRQAPGQGLEWMGAIDPKTGGTDYNQKFKDRVTMTRDTSISTAYM ELSRLRSDDTAVYYCARDLGYFDVWGQGTLVTVSS | |
| | HC2 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCDKTHTCPPCP APELLGPSVFLFPPKPKDTLMISRTPEVTCVVVDVSHEDPEVKFNWY VDGVEVHNAKTKPREEQYNSTYRVVSVLTVLHQDWLNGKEYKCK VSNKALGAPIEKTISKAKGQPREPQVYTLPPSREEMTKNQVSLSCAV KGFYPSDIAVEWESNGQPENNYKTTPPVLDSDGSFFLVSKLTVDKSR WQQGNVFSCSVMHEALHNRFTQKSLSLSPGKGGGGSGGGGSGGGG SGGGGSDIQMTQSPSSLSASVGDRVTITCRASQDIRSNLNWYQQKPG GAVKLLIYYTSRLHSGVPSRFSGSGSGTDYTLTISSLQPEDFATYFCQ QSEKLPRTFGGGTKVEIR | 65 |
| | LC1 | SYVLTQPPSVSVAPGQTARITCGGNNIGSKSVHWYQQPPGQAPVVV VYDDSDRPSGIPERFSGSNSGNTATLTISRVEAGDEAVYYCQVWDSS SDHVVFGGGTKLTVLGQPKAAPSVTLFPPSSEELQANKATLVCLISD FYPGAVTVAWKGDSSPVKAGVETTTPSKQSNNKYAASSYLSLTPEQ WKSHRSYSCQVTHEGSTVEKTVAPTECS | 60 |
| Ly3106 | HC1 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCGGGGSGGGG SGGGGSGGGGSEVQLVESGGGLVQPGGSLRLSCAASGFTFSTYAMN WVRQAPGKGLEWVGRIRSKYNNYATYYADSVKGRFTISRDDSKNS LYLQMNSLKTEDTAVYYCVRHGNFGNSYVSWFAYWGQGTLVTVS SGGGGSDKTHTCPPCPAPELLGPSVFLFPPKPKDTLMISRTPEVTCVV VDVSHEDPEVKFNWYVDGVEVHNAKTKPREEQYNSTYRVVSVLT VLHQDWLNGKEYKCKVSNKALGAPIEKTISKAKGQPREPQVYTLPP SREEMTKNQVSLWCLVKGFYPSDIAVEWESNGQPENNYKTTPPVL DSDGSFFLYSKLTVDKSRWQQGNVFSCSVMHEALHNHYTQKSLSL SPGKGGGGSGGGGSGGGGSGGGGSQVQLVQSGAEVKKPGASVKV SCKASGYTFTDFWMSWVRQAPGQGLEWMGQIYPNTGTTHSNEKF KGRVTMTRDTSTSTVYMELSSLRSEDTAVYYCARSYHISTTPNWFA YWGQGTLVTVSS | 67 |
| BCMA(b) /CD3 /PD-L1 | | | |
| | HC2 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCGGGGSGGGG SGGGGSGGGGSQAVVTQEPSLTVSPGGTVTLTCRSSTGAVTTSNYA NWVQQKPGQAPRGLIGGTNKRAPWTPARFSGSLLGGKAALTITGA QAEDEADYYCALWYSNLWVFGGGTKLTVLGGGGSDKTHTCPPCP APELLGPSVFLFPPKPKDTLMISRTPEVTCVVVDVSHEDPEVKFNWY VDGVEVHNAKTKPREEQYNSTYRVVSVLTVLHQDWLNGKEYKCK VSNKALGAPIEKTISKAKGQPREPQVYTLPPSREEMTKNQVSLSCAV | 68 |
| | | KGFYPSDIAVEWESNGQPENNYKTTPPVLDSDGSFFLVSKLTVDKSR WQQGNVFSCSVMHEALHNRFTQKSLSLSPGKGGGGSGGGGSGGGG SGGGGSDIQMTQSPSSLSASVGDRVTITCKASQNVYKKLEWYQQKP GKVPKVLIYHTNILQTGVPSRFSGSGSGTDYTLTISSLQPEDVATYYC YQWNSGPTFGGGTKVEIK | |
| | LC1 | SYVLTQPPSVSVAPGQTARITCGGNNIGSKSVHWYQQPPGQAPVVV VYDDSDRPSGIPERFSGSNSGNTATLTISRVEAGDEAVYYCQVWDSS SDHVVFGGGTKLTVLGQPKAAPSVTLFPPSSEELQANKATLVCLISD FYPGAVTVAWKGDSSPVKAGVETTTPSKQSNNKYAASSYLSLTPEQ WKSHRSYSCQVTHEGSTVEKTVAPTECS | 60 |
| Ly2313 | HC1 | QVQLVQSGAEVKKPGASVKVSCKASGYTFTNHIIHWVRQAPGQCL EWMGYINPYPGYHAYNEKFQGRATMTSDTSTSTVYMELSSLRSED TAVYYCARDGYYRDTDVLDYWGQGTLVTVSSGGGGSGGGGSGGG GSDIQMTQSPSSLSASVGDRVTITCQASQDISNYLNWYQQKPGKAP KLLIYYTSRLHTGVPSRFSGSGSGTDFTFTISSLEPEDIATYYCQQGN TLPWTFGCGTKVEIKSGGGGSEVQLVESGGGLVQPGGSLKLSCAAS GFTFNKYAMNWVRQAPGKGLEWVARIRSKYNNYATYYADSVKDR FTISRDDSKNTAYLQMNNLKTEDTAVYYCVRHGNFGNSYISYWAY WGQGTLVTVSSGGGGSGGGGSGGGGSQTVVTQEPSLTVSPGGTVT LTCGSSTGAVTSGNYPNWVQQKPGQAPRGLIGGTKFLAPGTPARFS GSLLGGKAALTLSGVQPEDEAEYYCVLWYSNRWVFGGGTKLTVLG GGGDKTHTCPPCPAPELLGGPSVFLFPPKPKDTLMISRTPEVTCVVV DVSHEDPEVKFNWYVDGVEVHNAKTKPCEEQYGSTYRCVSVLTVL HQDWLNGKEYKCKVSNKALPAPIEKTISKAKGQPREPQVYTLPPSR EEMTKNQVSLTCLVKGFYPSDIAVEWESNGQPENNYKTTPPVLDSD GSFFLYSKLTVDKSRWQQGNVFSCSVMHEALHNHYTQKSLSLSPG GGGSGGGGSGGGGSGGGGSGGGGSGGGGSDKTHTCPPCPAPELLG GPSVFLFPPKPKDTLMISRTPEVTCVVVDVSHEDPEVKFNWYVDGV EVHNAKTKPCEEQYGSTYRCVSVLTVLHQDWLNGKEYKCKVSNK ALPAPIEKTISKAKGQPREPQVYTLPPSREEMTKNQVSLTCLVKGFY PSDIAVEWESNGQPENNYKTTPPVLDSDGSFFLYSKLTVDKSRWQQ GNVFSCSVMHEALHNHYTQKSLSLSPGK | 70 |
| BCMA(a) /CD3 | | | |
| Ly2314 | HC1 | EVQLVESGGGLVQPGGSLRLSCAASGFTFNTYAMNWVRQAPGKGL EWVARIRSKYNNYATYYAASVKGRFTISRDDSKNSLYLQMNSLKTE DTAVYYCARHGNFGNSYVSWFAYWGQGTLVTVSSASTKGPSVFPL APCSRSTSESTAALGCLVKDYFPEPVTVSWNSGALTSGVHTFPAVL QSSGLYSLSSVVTVPSSSLGTKTYTCNVDHKPSNTKVDKRVESKYG PPCPPCPAPEAAGGPSVFLFPPKPKDTLMISRTPEVTCVVVDVSQEDP EVQFNWYVDGVEVHNAKTKPREEQFNSTYRVVSVLTVLHQDWLN GKEYKCKVSNKGLPSSIEKTISKAKGQPREPQVYTLPPSQEEMTKNQ VSLTCLVKGFYPSDIAVEWESNGQPENNYKTTPPVLDSDGSFLLYSK LTVDKSRWQEGNVFSCSVMHEALHNHYTQKSLSLSLGK | 71 |
| BCMA(b) /CD3 | | | |
| | HC2 | QLQLQESGPGLVKPSETLSLTCTVSGGSISSGSYFWGWIRQPPGKGL EWIGSIYYSGITYYNPSLKSRVTISVDTSKNQFSLKLSSVTAADTAVY YCARHDGAVAGLFDYWGQGTLVTVSSASTKGPSVFPLAPCSRSTSE STAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLGK | 72 |
| | LC1 | QTVVTQEPSLTVSPGGTVTLTCRSSTGAVTTSNYANWVQQKPGQAP RGLIGGTNKRAPGTPARFSGSLLGGKAALTLSGVQPEDEAEYYCAL WYSNLWVFGGGTKLTVLGQPKAAPSVTLFPPSSEELQANKATLVCL ISDFYPGAVTVAWKADSSPVKAGVETTTPSKQSNNKYAASSYLSLT PEQWKSHRSYSCQVTHEGSTVEKTVAPTECS | 73 |
| | LC2 | SYVLTQPPSVSVAPGQTARITCGGNNIGSKSVHWYQQPPGQAPVVV VYDDSDRPSGIPERFSGSNSGNTATLTISRVEAGDEAVYYCQVWDSS SDHVVFGGGTKLTVLGQPKAAPSVTLFPPSSEELQANKATLVCLISD FYPGAVTVAWKGDSSPVKAGVETTTPSKQSNNKYAASSYLSLTPEQ WKSHRSYSCQVTHEGSTVEKTVAPTECS | 60 |
| Ly2951 | HC1 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSEVQLVESGGGLVQPGGSLRLSCAASGFTF STYAMNWVRQAPGKGLEWVGRIRSKYNNYATYYADSVKGRFTISR DDSKNSLYLQMNSLKTEDTAVYYCVRHGNFGNSYVSWFAYWGQG TLVTVSSGGGGSDKTHTCPPCPAPELLGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREPQ VYTLPPSREEMTKNQVSLWCLVKGFYPSDIAVEWESNGQPENNYK TTPPVLDSDGSFFLYSKLTVDKSRWQQGNVFSCSVMHEALHNHYT QKSLSLSPGK | 75 |
| BCMA(a) /CD3 | | | |
| | HC2 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSQAVVTQEPSLTVSPGGTVTLTCRSSTGAV TTSNYANWVQQKPGQAPRGLIGGTNKRAPWTPARFSGSLLGGKAA LTITGAQAEDEADYYCALWYSNLWVFGGGTKLTVLGGGGSDKTHT CPPCPAPELLGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSREEMTKNQVSL SCAVKGFYPSDIAVEWESNGQPENNYKTTPPVLDSDGSFFLVSKLTV DKSRWQQGNVFSCSVMHEALHNRFTQKSLSLSPGK | 76 |
| | LC1 | DIQMTQSPSSLSASVGDRVTITCQASQDISNYLNWYQQKPGKAPKL LIYYTSRLHTGVPSRFSGSGSGTDFTFTISSLEPEDIATYYCQQGNTLP WTFGQGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC | 57 |
| Ly2952 | HC1 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPELLGPSVFLFPPKPKDTLMISRTPEVTCVVVDVSHEDPE VKFNWYVDGVEVHNAKTKPREEQYNSTYRVVSVLTVLHQDWLNG KEYKCKVSNKALGAPIEKTISKAKGQPREPQVYTLPPSREEMTKNQ VSLWCLVKGFYPSDIAVEWESNGQPENNYKTTPPVLDSDGSFFLYS KLTVDKSRWQQGNVFSCSVMHEALHNHYTQKSLSLSPGKGGGGSG | 78 |
| BCMA(a) /CD137 | | | |
| | | GGGSGGGGSGGGGSQVQLVQSGAEVKKPGASVKVSCKASGYTFA GFEMHWVRQAPGQGLEWMGAIDPKTGGTDYNQKFKDRVTMTRD TSISTAYMELSRLRSDDTAVYYCARDLGYFDVWGQGTLVTVSS | |
| | HC2 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPELLGPSVFLFPPKPKDTLMISRTPEVTCVVVDVSHEDPE VKFNWYVDGVEVHNAKTKPREEQYNSTYRVVSVLTVLHQDWLNG KEYKCKVSNKALGAPIEKTISKAKGQPREPQVYTLPPSREEMTKNQ VSLSCAVKGFYPSDIAVEWESNGQPENNYKTTPPVLDSDGSFFLVSK LTVDKSRWQQGNVFSCSVMHEALHNRFTQKSLSLSPGKGGGGSGG GGSGGGGSGGGGSDIQMTQSPSSLSASVGDRVTITCRASQDIRSNLN WYQQKPGGAVKLLIYYTSRLHSGVPSRFSGSGSGTDYTLTISSLQPE DFATYFCQQSEKLPRTFGGGTKVEIR | 79 |
| | LC1 | DIQMTQSPSSLSASVGDRVTITCQASQDISNYLNWYQQKPGKAPKL LIYYTSRLHTGVPSRFSGSGSGTDFTFTISSLEPEDIATYYCQQGNTLP WTFGQGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC | 57 |
| Ly2959 | HC1 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSEVQLVESGGGLVQPGGSLRLSCAASGFTF STYAMNWVRQAPGKGLEWVGRIRSKYNNYATYYADSVKGRFTISR DDSKNSLYLQMNSLKTEDTAVYYCVRHGNFGNSYVSWFAYWGQG TLVTVSSGGGGSDKTHTCPPCPAPELLGPSVFLFPPKPKDTLMISRTP EVTCVVVDVSHEDPEVKFNWYVDGVEVHNAKTKPREEQYNSTYR VVSVLTVLHQDWLNGKEYKCKVSNKALGAPIEKTISKAKGQPREPQ VYTLPPSREEMTKNQVSLWCLVKGFYPSDIAVEWESNGQPENNYK TTPPVLDSDGSFFLYSKLTVDKSRWQQGNVFSCSVMHEALHNHYT QKSLSLSPGKGGGGSGGGGSGGGGSGGGGSQVQLVQSGAEVKKPG ASVKVSCKASGYTFTDFWMSWVRQAPGQGLEWMGQIYPNTGTTH SNEKFKGRVTMTRDTSTSTVYMELSSLRSEDTAVYYCARSYHISTTP NWFAYWGQGTLVTVSS | 81 |
| BCMA(a) /CD3 /PD-L1 | | | |
| | HC2 | QVQLVQSGAEVKKPGASVKVSCKASGYTFTNHIIHWVRQAPGQGL EWMGYINPYPGYHAYNEKFQGRATMTSDTSTSTVYMELSSLRSED TAVYYCARDGYYRDTDVLDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKKVEPKSCGGG GSGGGGSGGGGSGGGGSQAVVTQEPSLTVSPGGTVTLTCRSSTGAV TTSNYANWVQQKPGQAPRGLIGGTNKRAPWTPARFSGSLLGGKAA LTITGAQAEDEADYYCALWYSNLWVFGGGTKLTVLGGGGSDKTHT CPPCPAPELLGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSREEMTKNQVSL SCAVKGFYPSDIAVEWESNGQPENNYKTTPPVLDSDGSFFLVSKLTV DKSRWQQGNVFSCSVMHEALHNRFTQKSLSLSPGKGGGGSGGGGS | 82 |
| | | GGGGSGGGGSDIQMTQSPSSLSASVGDRVTITCKASQNVYKKLEWY QQKPGKVPKVLIYHTNILQTGVPSRFSGSGSGTDYTLTISSLQPEDVA TYYCYQWNSGPTFGGGTKVEIK | |
| | LC1 | DIQMTQSPSSLSASVGDRVTITCQASQDISNYLNWYQQKPGKAPKL LIYYTSRLHTGVPSRFSGSGSGTDFTFTISSLEPEDIATYYCQQGNTLP WTFGQGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC | 57 |
| Ly2600 | HC1 | QVQLVQSGAEVKKPGASVKVSCKASGYTFTSYVMHWVRQKPGQG LEWIGYINPYNEGTESTDKFKGRATMTSDKSTSTVYMELSSLRSEDT AVYYCASIYYGYEGTYFGVWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFAGFEMHWVRQAPGQGLEWMGAIDPKTGGTDYN QKFKDRVTMTRDTSISTAYMELSRLRSDDTAVYYCARDLGYFDVW GQGTLVTVSS | 84 |
| B7H3 /CD3 /CD137 | | | |
| | HC2 | QVQLVQSGAEVKKPGASVKVSCKASGYTFTSYVMHWVRQKPGQG LEWIGYINPYNEGTESTDKFKGRATMTSDKSTSTVYMELSSLRSEDT AVYYCASIYYGYEGTYFGVWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGGS GGGGSGGGGSGGGGSQAVVTQEPSLTVSPGGTVTLTCRSSTGAVTT SNYANWVQQKPGQAPRGLIGGTNKRAPWTPARFSGSLLGGKAALT ITGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNHYTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT YFCQQSEKLPRTFGGGTKVEIR | 85 |
| | LC1 | EIVLTQSPATLSLSPGERATLSCRASSSVSYMHWYQQKPGQAPRPWI YATSNLASGIPARFSGSRSGTDYTLTISSLEPEDFAVYYCQQWSSNTL TFGGGTKVEIKRTVAAPSVFIFPPSDEQLKSGTASVVCLLNNFYPRE AKVQWKVDNALQSGNSQESVTEQDSKDSTYSLSSTLTLSKADYEK HKVYACEVTHQGLSSPVTKSFNRGEC | 86 |
| Ly2601 | HC1 | QVQLQQSGAELVRPGSSVKISCKASGYAFSSYWMNWVKQRPGQGL EWIGQIWPGDGDTNYNGKFKGKATLTADESSSTAYMQLSSLASEDS AVYFCARRETTTVGRYYYAMDYWGQGTTVTVSSASTKGPSVFPLA PSSKSTSGGTAALGCLVKDYFPEPVTVSWNSGALTSGVHTFPAVLQ SSGLYSLSSVVTVPSSSLGTQTYICNVNHKPSNTKVDKKVEPKSCGG GGSGGGGSGGGGSGGGGSEVQLVESGGGLVQPGGSLRLSCAASGF TFSTYAMNWVRQAPGKGLEWVGRIRSKYNNYATYYADSVKGRFTI SRDDSKNSLYLQMNSLKTEDTAVYYCVRHGNFGNSYVSWFAYWG QGTLVTVSSGGGGSDKTHTCPPCPAPELLGPSVFLFPPKPKDTLMISR TPEVTCVVVDVSHEDPEVKFNWYVDGVEVHNAKTKPREEQYNSTY RVVSVLTVLHQDWLNGKEYKCKVSNKALGAPIEKTISKAKGQPREP QVYTLPPSREEMTKNQVSLWCLVKGFYPSDIAVEWESNGQPENNY KTTPPVLDSDGSFFLYSKLTVDKSRWQQGNVFSCSVMHEALHNHY TQKSLSLSPGKGGGGSGGGGSGGGGSGGGGSQVQLVQSGAEVKKP GASVKVSCKASGYTFAGFEMHWVRQAPGQGLEWMGAIDPKTGGT DYNQKFKDRVTMTRDTSISTAYMELSRLRSDDTAVYYCARDLGYF DVWGQGTLVTVSS | 87 |
| CD19 /CD3 /CD137 | | | |
| | HC2 | QVQLQQSGAELVRPGSSVKISCKASGYAFSSYWMNWVKQRPGQGL EWIGQIWPGDGDTNYNGKFKGKATLTADESSSTAYMQLSSLASEDS AVYFCARRETTTVGRYYYAMDYWGQGTTVTVSSASTKGPSVFPLA PSSKSTSGGTAALGCLVKDYFPEPVTVSWNSGALTSGVHTFPAVLQ SSGLYSLSSVVTVPSSSLGTQTYICNVNHKPSNTKVDKKVEPKSCGG GGSGGGGSGGGGSGGGGSQAVVTQEPSLTVSPGGTVTLTCRSSTGA VTTSNYANWVQQKPGQAPRGLIGGTNKRAPWTPARFSGSLLGGKA ALTITGAQAEDEADYYCALWYSNLWVFGGGTKLTVLGGGGSDKT HTCPPCPAPELLGPSVFLFPPKPKDTLMISRTPEVTCVVVDVSHEDPE VKFNWYVDGVEVHNAKTKPREEQYNSTYRVVSVLTVLHQDWLNG KEYKCKVSNKALGAPIEKTISKAKGQPREPQVYTLPPSREEMTKNQ VSLSCAVKGFYPSDIAVEWESNGQPENNYKTTPPVLDSDGSFFLVSK LTVDKSRWQQGNVFSCSVMHEALHNHYTQKSLSLSPGKGGGGSGG GGSGGGGSGGGGSDIQMTQSPSSLSASVGDRVTITCRASQDIRSNLN WYQQKPGGAVKLLIYYTSRLHSGVPSRFSGSGSGTDYTLTISSLQPE DFATYFCQQSEKLPRTFGGGTKVEIR | 88 |
| | LC1 | DIQLTQSPASLAVSLGQRATISCKASQSVDYDGDSYLNWYQQIPGQP PKLLIYDASNLVSGIPPRFSGSGSGTDFTLNIHPVEKVDAATYHCQQS TEDPWTFGGGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC | 89 |
| Ly3232 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTCPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS | 54 |
| HER2 /CD3 /CD137 | | | |
| | | VKVSCKASGYTFAGFEMHWVRQAPGQGLEWMGAIDPKTGGTDYN QKFKDRVTMTRDTSISTAYMELSRLRSDDTAVYYCARDLGYFDVW GQGTLVTVSS | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPAVLQSSGLY SLCSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT YFCQQSEKLPRTFGGGTKVEIR | 63 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSQECVTEQDSKDSTYDLSSTLTLSKADYEK HKVYACEVTHQGLSSPVTKSFNRGEC | 66 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQESVTEQDSKDSTYRLCSTLTLSKADY EKHKVYACEVTHQGLSSPVTKSFNRGEC | 69 |
| Ly3233 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTFPACLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFAGFEMHWVRQAPGQGLEWMGAIDPKTGGTDYN QKFKDRVTMTRDTSISTAYMELSRLRSDDTAVYYCARDLGYFDVW GQGTLVTVSS | 74 |
| HER2 /CD3 /CD137 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTCPAVLQSSGLY SLSSVVTVPSSSLGTQTYICNVNHKPSNTKVDKKVEPKSCGGGGSG GGGSGGGGSGGGGSQAVVTQEPSLTVSPGGTVTLTCRSSTGAVTTS | 77 |
| | | NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT YFCQQSEKLPRTFGGGTKVEIR | |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSCESVTEQDSKDSTYDLSSTLTLSKADYEK HKVYACEVTHQGLSSPVTKSFNRGEC | 80 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQECVTEQDSKDSTYRLSSTLTLSKADY EKHKVYACEVTHQGLSSPVTKSFNRGEC | 83 |
| Ly3234 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTFPACLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFAGFEMHWVRQAPGQGLEWMGAIDPKTGGTDYN QKFKDRVTMTRDTSISTAYMELSRLRSDDTAVYYCARDLGYFDVW GQGTLVTVSS | 74 |
| HER2 /CD3 /CD137 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPAVLQSSGLY SLCSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT | 63 |
| | | YFCQQSEKLPRTFGGGTKVEIR | |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSCESVTEQDSKDSTYDLSSTLTLSKADYEK HKVYACEVTHQGLSSPVTKSFNRGEC | 80 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQESVTEQDSKDSTYRLCSTLTLSKADY EKHKVYACEVTHQGLSSPVTKSFNRGEC | 69 |
| Ly3235 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTCPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFAGFEMHWVRQAPGQGLEWMGAIDPKTGGTDYN QKFKDRVTMTRDTSISTAYMELSRLRSDDTAVYYCARDLGYFDVW GQGTLVTVSS | 54 |
| HER2 /CD3 /CD137 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPACLQSSGLY SLSSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT YFCQQSEKLPRTFGGGTKVEIR | 102 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSQESVTEQDSKDSTYDLCSTLTLSKADYEK | 103 |
| | | HKVYACEVTHQGLSSPVTKSFNRGEC | |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSCESVTEQDSKDSTYRLSSTLTLSKADY EKHKVYACEVTHQGLSSPVTKSFNRGEC | 104 |
| Ly3236 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTCPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFAGFEMHWVRQAPGQGLEWMGAIDPKTGGTDYN QKFKDRVTMTRDTSISTAYMELSRLRSDDTAVYYCARDLGYFDVW GQGTLVTVSS | 54 |
| HER2 /CD3 /CD137 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPAVLQSSGLY SLCSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCRASQDIRSNLNWYQ QKPGGAVKLLIYYTSRLHSGVPSRFSGSGSGTDYTLTISSLQPEDFAT YFCQQSEKLPRTFGGGTKVEIR | 63 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSQESVTEQDSKDSTYDLCSTLTLSKADYEK HKVYACEVTHQGLSSPVTKSFNRGEC | 103 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQESVTEQDSKDSTYRLCSTLTLSKADY EKHKVYACEVTHQGLSSPVTKSFNRGEC | 69 |
| Ly3353 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTCPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFTDFWMSWVRQAPGQGLEWMGQIYPNTGTTHSN EKFKGRVTMTRDTSTSTVYMELSSLRSEDTAVYYCARSYHISTTPN WFAYWGQGTLVTVSS | 105 |
| HER2 /CD3 /PD-L1 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPAVLQSSGLY SLCSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCKASQNVYKKLEWYQ QKPGKVPKVLIYHTNILQTGVPSRFSGSGSGTDYTLTISSLQPEDVAT YYCYQWNSGPTFGGGTKVEIK | 106 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSQECVTEQDSKDSTYDLSSTLTLSKADYEK HKVYACEVTHQGLSSPVTKSFNRGEC | 66 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQESVTEQDSKDSTYRLCSTLTLSKADY EKHKVYACEVTHQGLSSPVTKSFNRGEC | 69 |
| Ly3354 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTFPACLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE | 107 |
| HER2 /CD3 /PD-L1 | | | |
| | | VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFTDFWMSWVRQAPGQGLEWMGQIYPNTGTTHSN EKFKGRVTMTRDTSTSTVYMELSSLRSEDTAVYYCARSYHISTTPN WFAYWGQGTLVTVSS | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTCPAVLQSSGLY SLSSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCKASQNVYKKLEWYQ QKPGKVPKVLIYHTNILQTGVPSRFSGSGSGTDYTLTISSLQPEDVAT YYCYQWNSGPTFGGGTKVEIK | 108 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSCESVTEQDSKDSTYDLSSTLTLSKADYEK HKVYACEVTHQGLSSPVTKSFNRGEC | 80 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQECVTEQDSKDSTYRLSSTLTLSKADY EKHKVYACEVTHQGLSSPVTKSFNRGEC | 83 |
| Ly3355 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTFPACLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFTDFWMSWVRQAPGQGLEWMGQIYPNTGTTHSN EKFKGRVTMTRDTSTSTVYMELSSLRSEDTAVYYCARSYHISTTPN WFAYWGQGTLVTVSS | 107 |
| HER2 /CD3 /PD-L1 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPAVLQSSGLY SLCSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCKASQNVYKKLEWYQ QKPGKVPKVLIYHTNILQTGVPSRFSGSGSGTDYTLTISSLQPEDVAT YYCYQWNSGPTFGGGTKVEIK | 106 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSCESVTEQDSKDSTYDLSSTLTLSKADYEK HKVYACEVTHQGLSSPVTKSFNRGEC | 80 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQESVTEQDSKDSTYRLCSTLTLSKADY EKHKVYACEVTHQGLSSPVTKSFNRGEC | 69 |
| Ly3356 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTCPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFTDFWMSWVRQAPGQGLEWMGQIYPNTGTTHSN EKFKGRVTMTRDTSTSTVYMELSSLRSEDTAVYYCARSYHISTTPN WFAYWGQGTLVTVSS | 105 |
| HER2 /CD3 /PD-L1 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPACLQSSGLY SLSSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN | 109 |
| | | WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCKASQNVYKKLEWYQ QKPGKVPKVLIYHTNILQTGVPSRFSGSGSGTDYTLTISSLQPEDVAT YYCYQWNSGPTFGGGTKVEIK | |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSQESVTEQDSKDSTYDLCSTLTLSKADYEK HKVYACEVTHQGLSSPVTKSFNRGEC | 103 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSCESVTEQDSKDSTYRLSSTLTLSKADY EKHKVYACEVTHQGLSSPVTKSFNRGEC | 104 |
| Ly3357 | HC1 | EVQLVESGGGLVQPGGSLRLSCAASGFNIKDTYIHWVRQAPGKDLE WVARIYPTNGYTRYADSVKGRFTISADTSKNTAYLQMNSLRAEDT AVYYCSRWGGDGFYAMDYWGRGTLVTVSSASTKGPSVFPLAPSSK STSGGTAALGCLVRDYFPEPVTVSWNSGALTSGVRTCPAVLQSSGL YSLSSVVTVPSSSLGTQTYICNVNHKPSNTKVDKKVEPKSCGGGGS GGGGSGGGGSGGGGSEVQLVESGGGLVQPGGSLRLSCAASGFTFST YAMNWVRQAPGKGLEWVGRIRSKYNNYATYYADSVKGRFTISRD DSKNSLYLQMNSLKTEDTAVYYCVRHGNFGNSYVSWFAYWGQGT LVTVSSGGGGSDKTHTCPPCPAPELLGPSVFLFPPKPKDTLMISRTPE VTCVVVDVSHEDPEVKFNWYVDGVEVHNAKTKPREEQYNSTYRV VSVLTVLHQDWLNGKEYKCKVSNKALGAPIEKTISKAKGQPREPQV YTLPPSREEMTKNQVSLWCLVKGFYPSDIAVEWESNGQPENNYKTT PPVLDSDGSFFLYSKLTVDKSRWQQGNVFSCSVMHEALHNHYTQK SLSLSPGKGGGGSGGGGSGGGGSGGGGSQVQLVQSGAEVKKPGAS VKVSCKASGYTFTDFWMSWVRQAPGQGLEWMGQIYPNTGTTHSN EKFKGRVTMTRDTSTSTVYMELSSLRSEDTAVYYCARSYHISTTPN WFAYWGQGTLVTVSS | 105 |
| HER2 /CD3 /PD-L1 | | | |
| | HC2 | EVQLVESGGGLVQPGGSLRLSCAASGFTFTDYTMDWVRQAPGKRL EWVADVNPNSGGSIYNQRFKGRFTLSVDRSKNTLYLQMNSLRAED TAVYYCARNLGPSFYFDYWGDGTLVTVSSASTKGPSVFPLAPSSKS TSGGTAALGCLVDDYFPEPVTVSWNSGALTSGVDTFPAVLQSSGLY SLCSVVTVPSSSLGTQTYICNVNHKPSNTKVDKKVEPKSCGGGGSG GGGSGGGGSGGGGSQAVVTQEPSLTVSPGGTVTLTCRSSTGAVTTS NYANWVQQKPGQAPRGLIGGTNKRAPWTPARFSGSLLGGKAALTI TGAQAEDEADYYCALWYSNLWVFGGGTKLTVLGGGGSDKTHTCP PCPAPELLGPSVFLFPPKPKDTLMISRTPEVTCVVVDVSHEDPEVKFN WYVDGVEVHNAKTKPREEQYNSTYRVVSVLTVLHQDWLNGKEYK CKVSNKALGAPIEKTISKAKGQPREPQVYTLPPSREEMTKNQVSLSC AVKGFYPSDIAVEWESNGQPENNYKTTPPVLDSDGSFFLVSKLTVD KSRWQQGNVFSCSVMHEALHNRFTQKSLSLSPGKGGGGSGGGGSG GGGSGGGGSDIQMTQSPSSLSASVGDRVTITCKASQNVYKKLEWYQ QKPGKVPKVLIYHTNILQTGVPSRFSGSGSGTDYTLTISSLQPEDVAT YYCYQWNSGPTFGGGTKVEIK | 106 |
| | LC1 | DIQMTQSPSSLSASVGDRVTITCRASQDVNTAVAWYQQKPGKDPKL LIYSASFLYSGVPSRFSGSRSGTDFTLTISSLQPEDFATYYCQQHYTTP PTFGRGTKVEIKRTVAAPSVFIFPPSDEQLKSGTADVVCLLNNFYPRE AKVQWKVDNALQSGNSQESVTEQDSKDSTYDLCSTLTLSKADYEK HKVYACEVTHQGLSSPVTKSFNRGEC | 103 |
| | LC2 | DIQMTQSPSSLSASVGDRVTITCKASQDVSIGVAWYQQKPGKRPKL LIYSASYRYTGVPSRFSGSGSGTDFTLTISSLQPEDFATYYCQQYYIY PYTFGDGTKVEIKRTVAAPSVFIFPPSDEQLKSGTARVVCLLNNFYP REAKVQWKVDNALQSGNSQESVTEQDSKDSTYRLCSTLTLSKADY EKHKVYACEVTHQGLSSPVTKSFNRGEC | 69 |

The following items are herein described.
1. A multi-specific antibody, comprising:
   (i) a first antigen binding moiety that binds CD3, wherein the first antigen binding moiety is a first Fv fragment comprising a first heavy chain variable region (VH) and a first light chain variable region (VL);
   (ii) a second antigen binding moiety that binds CD137 or PD-L1, wherein the second antigen binding moiety is a second Fv fragment comprising a second VH and a second VL;
   (iii) an Fc fragment connecting the first antigen binding moiety and the second antigen binding moiety, wherein the Fc fragment comprises a hinge domain and a CH2 domain, and wherein the Fc fragment comprises a deletion at position 237 and an amino acid substitution at position P329, optionally P329G, following the EU numbering system; and
   (iv) a third antigen binding moiety that binds a first tumor associated antigen; wherein the third antigen binding moiety is connected to the first antigen binding moiety, optionally via a first peptide linker.
2. The multi-specific antibody of item 1, which further comprise (v) a fourth antigen binding moiety that binds a second tumor associated antigen; wherein the fourth antigen binding moiety is connected to the first antigen binding moiety, optionally via a second peptide linker.
3. The multi-specific antibody of item 1 or item 2, wherein the Fc fragment of (iii) is an IgG1 Fc fragment, which optionally comprises the amino acid sequence of SEQ ID NO: 91.
4. The multi-specific antibody of any one of items 1-3, wherein the Fc fragment further comprises a CH3 domain.
5. The multi-specific antibody of item 4, wherein the CH3 domain comprises one or more mutations that enhance heterodimerization over homodimerization of the Fc fragments comprising such as relative to the wild-type counterpart and/or reduce protein A binding.
6. The multi-specific antibody of item 5, wherein the CH3 domain comprises the amino acid sequence of SEQ ID NO: 93, 94, or 95.
7. The multi-specific antibody of any one of items 1-6, wherein the first VH comprises the same heavy chain complementarity determining regions (CDRs) as those in SEQ ID NO: 7 and the first VL comprises the same light chain CDRs as those in SEQ ID NO: 8.
8. The multi-specific antibody of item 7, wherein the first VH comprises the amino acid sequence of SEQ ID NO: 7 and the first VL comprises the amino acid sequence of SEQ ID NO: 8.
9. The multi-specific antibody of any one of items 1-8, wherein the second antigen binding moiety binds CD137.
10. The multi-specific antibody of item 9, wherein the second VH comprises the same heavy chain complementarity determining regions (CDRs) as those in SEQ ID NO: 9 and the second VL comprises the same light chain CDRs as those in SEQ ID NO: 10.
11. The multi-specific antibody of item 10, wherein the second VH comprises the amino acid sequence of SEQ ID NO: 9 and the second VL comprises the amino acid sequence of SEQ ID NO: 10.
12. The multi-specific antibody of item 9, which comprises a first polypeptide comprising the amino acid sequence of SEQ ID NO: 3 and a second polypeptide comprising the amino acid sequence of SEQ ID NO: 4.
13. The multi-specific antibody of any one of items 1-8, wherein the second antigen binding moiety binds PD-L1.
14. The multi-specific antibody of item 13, wherein the second VH comprises the same heavy chain complementarity determining regions (CDRs) as those in SEQ ID NO: 11 and the second VL comprises the same light chain CDRs as those in SEQ ID NO: 12.
15. The multi-specific antibody of item 14, wherein the second VH comprises the amino acid sequence of SEQ ID NO: 11 and the second VL comprises the amino acid sequence of SEQ ID NO: 12.
16. The multi-specific antibody of item 9, which comprises a first polypeptide comprising the amino acid sequence of SEQ ID NO: 5 and a second polypeptide comprising the amino acid sequence of SEQ ID NO: 6.
17. The multi-specific antibody of any one of items 1-16, wherein the first tumor associated antigen is selected from the group consisting of B7H3, CD19, CD20, PSMA, HER2, CEA, BCMA, P53mut, DLL3, MET, and EGFR.
18. The multi-specific antibody of any one of items 2-17, wherein the second tumor associated antigen is selected from the group consisting of B7H3, CD19, CD20, PSMA, HER2, CEA, BCMA, P53mut, DLL3, MET, and EGFR; optionally wherein the second tumor associated antigen is identical to the first tumor associated antigen.
19. The multi-specific antibody of item 17 or item 18, wherein the first tumor associated antigen and/or the second tumor associated antigen is HER2, CEA, BCMA, B7H3, or CD19; optionally wherein the first tumor associated antigen and/or the second tumor associated antigen is HER2, CEA, or BCMA.
20. The multi-specific antibody of any one of items 2-19, wherein the third antigen binding moiety and the fourth antigen binding moiety bind to different epitopes of the tumor associated antigen.
21. The multi-specific antibody of any one of items 1-20, wherein the third antigen binding moiety and optionally the fourth antigen binding moiety are Fab fragments.
22. The multi-specific antibody of any one of items 1-21, wherein the multi-specific antibody binds:
   (a) HER2, CD3, and CD137;
   (b) HER2, CD3, and PD-L1;
   (c) CEA, CD3, and CD137;
   (d) CEA, CD3, and PD-L1;
   (e) BCMA, CD3, and CD137; and
   (f) BCMA, CD3, and PD-L1.
23. The multi-specific antibody of item 9, which comprises:
   (i) a first polypeptide comprising the amino acid sequence of SEQ ID NO: 23, a second polypeptide comprising the amino acid sequence of SEQ ID NO: 24, a third polypeptide comprising the amino acid sequence of SEQ ID NO: 25, and a fourth polypeptide comprising the amino acid sequence of SEQ ID NO: 26;
   (ii) a first polypeptide comprising the amino acid sequence of SEQ ID NO: 39, a second polypeptide comprising the amino acid sequence of SEQ ID NO: 40, and a third polypeptide comprising the amino acid sequence of SEQ ID NO: 41;
   (iii) a first polypeptide comprising the amino acid sequence of SEQ ID NO: 55, a second polypeptide comprising the amino acid sequence of SEQ ID NO: 56, and a third polypeptide comprising the amino acid sequence of SEQ ID NO: 57;
   (iv) a first polypeptide comprising the amino acid sequence of SEQ ID NO: 58, a second polypeptide comprising the amino acid sequence of SEQ ID NO: 59, and a third polypeptide comprising the amino acid sequence of SEQ ID NO: 60;
   (v) a first polypeptide comprising the amino acid sequence of SEQ ID NO: 54, a second polypeptide comprising the amino acid sequence of SEQ ID NO: 63, a third polypeptide comprising the amino acid sequence of SEQ ID NO: 66, and a fourth polypeptide comprising the amino acid sequence of SEQ ID NO: 69;
   (vi) a first polypeptide comprising the amino acid sequence of SEQ ID NO: 74, a second polypeptide comprising the amino acid sequence of SEQ ID NO: 77, a third polypeptide comprising the amino acid sequence of SEQ ID NO: 80, and a fourth polypeptide comprising the amino acid sequence of SEQ ID NO: 83;
   (vii) a first polypeptide comprising the amino acid sequence of SEQ ID NO: 74, a second polypeptide comprising the amino acid sequence of SEQ ID NO: 63, a third polypeptide comprising the amino acid sequence of SEQ ID NO: 80, and a fourth polypeptide comprising the amino acid sequence of SEQ ID NO: 69;
   (viii) a first polypeptide comprising the amino acid sequence of SEQ ID NO: 54, a second polypeptide comprising the amino acid sequence of SEQ ID NO: 102, a third polypeptide comprising the amino acid sequence of SEQ ID NO: 103, and a fourth polypeptide comprising the amino acid sequence of SEQ ID NO: 104; or
   (ix) a first polypeptide comprising the amino acid sequence of SEQ ID NO: 54, a second polypeptide comprising the amino acid sequence of SEQ ID NO: 63, a third polypeptide comprising the amino acid sequence of SEQ ID NO: 103, and a fourth polypeptide comprising the amino acid sequence of SEQ ID NO: 69.
24. The multi-specific antibody of item 13, which comprises:
   (i) a first polypeptide comprising the amino acid sequence of SEQ ID NO: 27, a second polypeptide comprising the amino acid sequence of SEQ ID NO: 28, a third polypeptide comprising the amino acid sequence of SEQ ID NO: 25, and a fourth polypeptide comprising the amino acid sequence of SEQ ID NO: 26;
   (ii) a first polypeptide comprising the amino acid sequence of SEQ ID NO: 46, a second polypeptide comprising the amino acid sequence of SEQ ID NO: 47, and a third polypeptide comprising the amino acid sequence of SEQ ID NO: 41;
   (iii) a first polypeptide comprising the amino acid sequence of SEQ ID NO: 67, a second polypeptide comprising the amino acid sequence of SEQ ID NO: 68, and a third polypeptide comprising the amino acid sequence of SEQ ID NO: 60;
   (iv) a first polypeptide comprising the amino acid sequence of SEQ ID NO: 81, a second polypeptide comprising the amino acid sequence of SEQ ID NO: 82, and a third polypeptide comprising the amino acid sequence of SEQ ID NO: 57;
   (v) a first polypeptide comprising the amino acid sequence of SEQ ID NO: 105, a second polypeptide comprising the amino acid sequence of SEQ ID NO: 106, a third polypeptide comprising the amino acid sequence of SEQ ID NO: 66, and a fourth polypeptide comprising the amino acid sequence of SEQ ID NO: 69;
   (vi) a first polypeptide comprising the amino acid sequence of SEQ ID NO: 107, a second polypeptide comprising the amino acid sequence of SEQ ID NO: 108, a third polypeptide comprising the amino acid sequence of SEQ ID NO: 80, and a fourth polypeptide comprising the amino acid sequence of SEQ ID NO: 83;
   (vii) a first polypeptide comprising the amino acid sequence of SEQ ID NO: 107, a second polypeptide comprising the amino acid sequence of SEQ ID NO: 106, a third polypeptide comprising the amino acid sequence of SEQ ID NO: 80, and a fourth polypeptide comprising the amino acid sequence of SEQ ID NO: 69;
   (viii) a first polypeptide comprising the amino acid sequence of SEQ ID NO: 105, a second polypeptide comprising the amino acid sequence of SEQ ID NO: 109, a third polypeptide comprising the amino acid sequence of SEQ ID NO: 103, and a fourth polypeptide comprising the amino acid sequence of SEQ ID NO: 104; or
   (ix) a first polypeptide comprising the amino acid sequence of SEQ ID NO: 105, a second polypeptide comprising the amino acid sequence of SEQ ID NO: 106, a third polypeptide comprising the amino acid sequence of SEQ ID NO: 103, and a fourth polypeptide comprising the amino acid sequence of SEQ ID NO: 69.
25. A nucleic acid or a nucleic acid set, which collectively encodes the multi-specific antibody setting forth in any one of items 1-24.
26. The nucleic acid or the nucleic acid set of item 25, which is an expression vector or an expression vector set.
27. A host cell, comprising the nucleic acid or the nucleic acid set of item 25 or item 26.
28. The host cell of item 27, which is a mammalian host cell.
29. A method for producing a multi-specific antibody, comprising:
   (i) culturing the host cell of item 27 or item 28 under conditions allowing for expression of the antibody; and
   (ii) harvesting the antibody thus produced.
30. A pharmaceutical composition comprising a multi-specific antibody set forth in any one of items 1-24 or a nucleic acid or nucleic acid set encoding such, and a pharmaceutically acceptable carrier.
31. A method for modulating immune responses in a subject, the method comprising administering to a subject in need thereof an effective amount of the multi-specific antibody of any one of items 1-24, a nucleic acid(s) encoding such, or a pharmaceutical composition comprising the antibody or the encoding nucleic acid(s).
32. The method of item 31, wherein the subject is a human patient having or suspected of having cancer.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

## Claims

1. A multi-specific antibody, comprising:
(i) a first antigen binding moiety that binds CD3, wherein the first antigen binding moiety is a first Fv fragment comprising a first heavy chain variable region (V_{H}) and a first light chain variable region (V_{L});
(ii) a second antigen binding moiety that binds CD137, wherein the second antigen binding moiety is a second Fv fragment comprising a second V_{H} and a second V_{L};
(iii) an Fc fragment connecting the first antigen binding moiety and the second antigen binding moiety, wherein the Fc fragment comprises a hinge domain and a CH2 domain, and wherein the Fc fragment comprises a deletion at position 237 and an amino acid substitution at position P329, optionally P329G, following the EU numbering system; and
(iv) a third antigen binding moiety that binds a first tumor associated antigen; wherein the third antigen binding moiety is connected to the first antigen binding moiety, optionally via a first peptide linker; and optionally
(v) a fourth antigen binding moiety that binds a second tumor associated antigen; wherein the fourth antigen binding moiety is connected to the first antigen binding moiety, optionally via a second peptide linker;
wherein the first tumor associated antigen and the optional second tumor association antigen are both CEA; optionally wherein the third antigen binding moiety and the optional fourth antigen binding moiety bind to different epitopes of CEA.

2. The multi-specific antibody of claim 1, wherein the Fc fragment of (iii) is an IgG1 Fc fragment, which optionally comprises the amino acid sequence of SEQ ID NO: 91; optionally wherein the Fc fragment further comprises a CH3 domain, which optionally comprises one or more mutations that enhance heterodimerization over homodimerization of the Fc fragments comprising such as relative to the wild-type counterpart and/or reduce protein A binding, preferably wherein the CH3 domain comprises the amino acid sequence of SEQ ID NO: 93, 94, or 95.

3. The multi-specific antibody of claim 1 or claim 2, wherein the first V_{H} comprises the same heavy chain complementarity determining regions (CDRs) as those in SEQ ID NO: 7 and the first V_{L} comprises the same light chain CDRs as those in SEQ ID NO: 8; and/or
wherein the second V_{H} comprises the same heavy chain complementarity determining regions (CDRs) as those in SEQ ID NO: 9 and the second V_{L} comprises the same light chain CDRs as those in SEQ ID NO: 10. .

4. The multi-specific antibody of claim 3, wherein the first V_{H} comprises the amino acid sequence of SEQ ID NO: 7 and the first V_{L} comprises the amino acid sequence of SEQ ID NO: 8; and/or
wherein the second V_{H} comprises the amino acid sequence of SEQ ID NO: 9 and the second V_{L} comprises the amino acid sequence of SEQ ID NO: 10. .

5. The multi-specific antibody of claim 4, which comprises a first polypeptide comprising the amino acid sequence of SEQ ID NO: 3 and a second polypeptide comprising the amino acid sequence of SEQ ID NO: 4.

6. The multi-specific antibody of any one of claims 1-5, wherein the third antigen binding moiety and optionally the fourth antigen binding moiety are Fab fragments.

7. The multi-specific antibody of any one of claims 1-6, wherein the third antigen binding moiety and/or the optional fourth antigen binding moiety comprises the same heavy chain CDRs as those in SEQ ID NO: 17 and the same light chain CDRs as those in SEQ ID NO: 18; optionally wherein the third antigen binding moiety and/or the optional fourth antigen binding moiety comprises the amino acid sequence of SEQ ID NO: 17 and the amino acid sequence of SEQ ID NO: 18.

8. The multi-specific antibody of claim 1, which comprises:
(i) a first polypeptide comprising, from N-terminus to C-terminus, a VH-CH1 chain of the Fab fragments, a VH of the first antigen binding moiety, an Fc fragment, and a VH of the second antigen binding moiety;
(ii) a second polypeptide comprising, from N-terminus to C-terminus, the VH-CH1 chain of the Fab fragments, a VL of the first antigen binding moiety, an Fc fragment, and a VL of the second antigen binding moiety; and
(iii) a third polypeptide comprising a VL-CL of the Fab fragments.

9. The multi-specific antibody of claim 1, wherein the third antigen binding moiety is connected to the first antigen binding moiety via a first peptide linker and/or wherein the optional fourth antigen binding moiety is connected to the first antigen binding moiety via a second peptide linker; and
wherein the first peptide linker, the second peptide linker, or both the peptide linker comprises the amino acid sequence of SEQ ID NO: 96, 97, 98, 99, 100 or 101.

10. The multi-specific antibody of claim 9, which comprises:
a first polypeptide comprising the amino acid sequence of SEQ ID NO: 39, a second polypeptide comprising the amino acid sequence of SEQ ID NO: 40, and a third polypeptide comprising the amino acid sequence of SEQ ID NO: 41.

11. A nucleic acid or a nucleic acid set, which collectively encodes the multi-specific antibody setting forth in any one of claims 1-10; optionally wherein the nucleic acid or the nucleic acid set is an expression vector or an expression vector set.

12. A host cell, comprising the nucleic acid or the nucleic acid set of claim 11; optionally wherein the host cell is a mammalian host cell.

13. A method for producing a multi-specific antibody, comprising:
(i) culturing the host cell of claim 12 under conditions allowing for expression of the antibody; and
(ii) harvesting the antibody thus produced.

14. A pharmaceutical composition comprising a multi-specific antibody set forth in any one of claims 1-10 or a nucleic acid or nucleic acid set encoding such, and a pharmaceutically acceptable carrier.

15. The multi-specific antibody of any one of claims 1-10, a nucleic acid(s) encoding such, or a pharmaceutical composition comprising the antibody or the encoding nucleic acid(s) for use in modulating immune responses or treating cancer in a subject; optionally wherein the subject is a human patient having or suspected of having cancer.
